# EUROPEAN PATENT SPECIFICATION

(11) **EP 2 773 276 B1**
(45) Date of publication and mention of the grant of the patent: **04.04.2018**
(21) Application number: 12788024.3
(22) Date of filing: 02.11.2012
(51) Int. Cl.: A61B 18/14, A61B 18/02, A61B 18/00, A61B 90/00

(54) **SYSTEMS FOR CRYNOGENIC RENAL NEUROMODULATION**
SYSTEME ZUR KRYNOGENEN NIERENNERVENMODULATION
SYSTÈMES POUR UNE NEUROMODULATION RÉNALE CRYOGÉNIQUE

(30) Priority: 05.11.2011 US 201161556211 P; 07.11.2011 US 201161556737 P; 23.12.2011 US 201161580127 P; 27.04.2012 US 201261639852 P
(43) Date of publication of application: 10.09.2014
(73) Proprietor: Medtronic Ardian Luxembourg S.à.r.l., 2124 Luxembourg (LU)
(72) Inventor: BRENNAN, Gabriel, Santa Rosa, California 95403 (US); BERTRAND, Anthony, Santa Rosa, California 95403 (US); CHANG, Stacey, Santa Rosa, California 95403 (US); DOERKSEN, Kyle, Santa Rosa, California 95403 (US); DONLON, Brian, Santa Rosa, California 95403 (US); DUFFY, Angela, Santa Rosa, California 95403 (US); HARRINGTON, Kara, Santa Rosa, California 95403 (US); HOFF, Mark, Santa Rosa, California 95403 (US); HUYNH, Tim, Santa Rosa, California 95403 (US); KELLY, Brian, Santa Rosa, California 95403 (US); KELLY, Grace, Santa Rosa, California 95403 (US); MORIARTY, Micheal, Santa Rosa, California 95403 (US); NASH, Stephen, Santa Rosa, California 95403 (US); OBERWELZ, Elger, Santa Rosa, California 95403 (US); SAATCHI, Sanaz, Santa Rosa, California 95403 (US); SWEENEY, Fiachra, Santa Rosa, California 95403 (US); TRUDEL, Julie, Santa Rosa, California 95403 (US); TUROVSKIY, Roman, Santa Rosa, California 95403 (US); BIRDSALL, Matthew, Santa Rosa, California 95403 (US)
(74) Representative: Zimmermann & Partner Patentanwälte mbB
(86) International application number: PCT/US2012/063411
(87) International publication number: WO 2013/067421

(56) References cited:
- WO-A2-2006/127467
- US-A1- 2002 045 893
- US-A1- 2009 281 533
- US-A1- 2010 198 203
- US-A1- 2011 054 453
- US-A1- 2011 152 855
- US-B1- 6 537 271

## Description

### CROSS-REFERENCE TO RELATED APPLICATION(S)

This application claims the benefit of the following pending applications:
(a) U.S. Provisional Application No. 61/556,211, filed November 5, 2011
(b) U.S. Provisional Application No. 61/556,737, filed November 7, 2011
(c) U.S. Provisional Application No. 61/580,127, filed December 23, 2011
(d) U.S. Provisional Application No. 61/639,852, filed April 27, 2011

All of the foregoing applications are incorporated herein by reference in their entireties. Further, components and features of embodiments disclosed in the applications incorporated by reference may be combined with various components and features disclosed and claimed in the present application.

### TECHNICAL FIELD

The present technology relates generally to cryotherapeutic systems (e.g., cryotherapeutic systems configured for renal neuromodulation). In particular, several embodiments are directed to cryotherapeutic-system components configured to be outside the vasculature during a treatment procedure and to support (e.g., control and/or supply) cryotherapeutic-system components configured to be inside the vasculature during a treatment procedure. Related systems, devices, and methods are also disclosed.

### BACKGROUND

The sympathetic nervous system (SNS) is a primarily involuntary bodily control system typically associated with stress responses. Fibers of the SNS innervate tissue in almost every organ system of the human body and can affect characteristics such as pupil diameter, gut motility, and urinary output. Such regulation can have adaptive utility in maintaining homeostasis or in preparing the body for rapid response to environmental factors. Chronic activation of the SNS, however, is a common maladaptive response that can drive the progression of many disease states. Excessive activation of the renal SNS in particular has been identified experimentally and in humans as a likely contributor to the complex pathophysiology of hypertension, states of volume overload (such as heart failure), and progressive renal disease. For example, radiotracer dilution has demonstrated increased renal norepinephrine (NE) spillover rates in patients with essential hypertension.

Cardio-renal sympathetic nerve hyperactivity can be particularly pronounced in patients with heart failure. For example, an exaggerated NE overflow from the heart and kidneys to plasma is often found in these patients. Heightened SNS activation commonly characterizes both chronic and end-stage renal disease. In patients with end-stage renal disease, NE plasma levels above the median have been demonstrated to be predictive for cardiovascular diseases and several causes of death. This is also true for patients suffering from diabetic or contrast nephropathy. Evidence suggests that afferent signals originating from diseased kidneys are major contributors to initiating and sustaining elevated central sympathetic outflow.

Sympathetic nerves to the kidneys terminate in the blood vessels, the juxtaglomerular apparatus, and the renal tubules. Stimulation of the renal sympathetic nerves can cause increased renin release, increased sodium (Na⁻) reabsorption, and a reduction of renal blood flow. These neural regulation components of renal function are considerably stimulated in disease states characterized by heightened sympathetic tone and likely contribute to increased blood pressure in hypertensive patients. The reduction of renal blood flow and glomerular filtration rate as a result of renal sympathetic efferent stimulation is likely a cornerstone of the loss of renal function in cardio-renal syndrome (i.e., renal dysfunction as a progressive complication of chronic heart failure). Pharmacologic strategies to thwart the consequences of renal efferent sympathetic stimulation include centrally acting sympatholytic drugs, beta blockers (intended to reduce renin release), angiotensin converting enzyme inhibitors and receptor blockers (intended to block the action of angiotensin **II** and aldosterone activation consequent to renin release), and diuretics (intended to counter the renal sympathetic mediated sodium and water retention). These pharmacologic strategies, however, have significant limitations including limited efficacy, compliance issues, side effects, and others. Accordingly, there is a strong public-health need for alternative treatment strategies.

US 2011/0152855 relates to systems, delivery devices and methods to treat tissue, wherein the delivery devices have coolable energy emitting assemblies.

### BRIEF DESCRIPTION OF THE DRAWINGS

Many aspects of the present disclosure can be better understood with reference to the following drawings. The components in the drawings are not necessarily to scale. Instead, emphasis is placed on illustrating clearly the principles of the present technology. Furthermore, components can be shown as transparent in certain views for clarity of illustration only and not to indicate that the illustrated component is necessarily transparent.
FIG. 1A is a partially schematic diagram illustrating a cryotherapeutic system configured in accordance with an embodiment of the present technology and including a console and a cryotherapeutic device.
FIG. 1B is a cross-sectional view illustrating a portion of the cryotherapeutic device of FIG. 1A including a distal portion of a shaft and a cooling assembly in a delivery state.
FIG. 1C is a cross-sectional view illustrating a portion of the cryotherapeutic device of FIG. 1A including a distal portion of a shaft and a cooling assembly in a deployed state.
FIG. 2A is a partially cross-sectional view illustrating portions of a cryotherapeutic device configured in accordance with an embodiment of the present technology with an over-the-wire configuration.
FIG. 2B is a cross-sectional view illustrating the cryotherapeutic device of FIG. 2A taken along the line 2B-2B.
FIG. 3 is a cross-sectional view illustrating a portion of a cryotherapeutic device configured in accordance with an embodiment of the present technology with a rapid-exchange configuration.
FIG. 4 is a cross-sectional view illustrating a portion of a cryotherapeutic device configured in accordance with an embodiment of the present technology and including a cooling assembly having a pressure-monitoring lumen.
FIG. 5 is a partially schematic diagram illustrating cryogenically modulating renal nerves in accordance with an embodiment of the present technology.
FIG. 6 is a block diagram illustrating a method of cryogenically modulating renal nerves in accordance with an embodiment of the present technology.
FIG. 7A is a partially schematic diagram illustrating a cryotherapeutic system configured in accordance with an embodiment of the present technology and including a console having a cartridge housing.
FIG. 7B is a partially schematic diagram illustrating a cryotherapeutic system configured in accordance with an embodiment of the present technology and including a console having a pack housing.
FIG. 7C is a partially schematic diagram illustrating a cryotherapeutic system configured in accordance with an embodiment of the present technology and including a console having an exhaust passage with first and second heat-exchange portions.
FIG. 7D is a partially schematic diagram illustrating a cryotherapeutic system configured in accordance with an embodiment of the present technology and including a cryotherapeutic device having a handle with selected valves and sensors.
FIG. 8A is a partially schematic diagram illustrating a cryotherapeutic system configured in accordance with an embodiment of the present technology and including a console and a cryotherapeutic device having a satellite.
FIG. 8B is a partially schematic diagram illustrating selected fluidic elements of the console of FIG. 8A.
FIG. 8C is a partially schematic diagram illustrating selected fluidic elements of the satellite of FIG. 8A.
FIG. 8D is a partially schematic diagram illustrating selected electrical elements of the console of FIG. 8A.
FIG. 8E is a partially schematic diagram illustrating selected electrical elements of the cryotherapeutic device of FIG. 8A.
FIG. 9A is a partially schematic diagram illustrating a cryotherapeutic system configured in accordance with an embodiment of the present technology and including a handle having a cartridge housing.
FIG. 9B is a partially schematic diagram illustrating a cryotherapeutic system configured in accordance with an embodiment of the present technology and including a vortex tube.
FIG. 9C is a partially schematic diagram illustrating a cryotherapeutic system configured in accordance with an embodiment of the present technology and including a gas thermometer and a timer having a button.
FIG. 9D is a partially schematic diagram illustrating a cryotherapeutic system configured in accordance with an embodiment of the present technology and including a thermocouple and a coupling member having a dynamo.
FIGS. 10A-10B are perspective views illustrating a console assembly configured in accordance with an embodiment of the present technology and including a console having a cartridge housing.
FIG. 10C is a partially exploded profile view illustrating the console of FIGS. 10A-10B.
FIG. 11 is a profile view illustrating a cartridge housing configured in accordance with an embodiment of the present technology and including a latch clamp.
FIG. 12 is a perspective view illustrating a cartridge housing configured in accordance with an embodiment of the present technology and including a coupling assembly.
FIG. 13A is a partially cross-sectional view illustrating a cartridge housing, a cartridge connector, and a cartridge configured in accordance with an embodiment of the present technology with the cartridge spaced apart from the cartridge connector.
FIG. 13B is a partially cross-sectional view illustrating the cartridge housing, the cartridge connector, and the cartridge of FIG. 13A with the cartridge coupled to the cartridge connector.
FIG. 14A is a perspective view illustrating a coupling member configured in accordance with an embodiment of the present technology and including a tip having a generally flat rim.
FIG. 14B is a perspective view illustrating a coupling member configured in accordance with an embodiment of the present technology and including a tip having a rim with a first angle.
FIG. 14C is a perspective view illustrating a coupling member configured in accordance with an embodiment of the present technology and including a tip having a rim with a second angle.
FIG. 15 is a partially exploded profile view illustrating a console and a cartridge configured in accordance with an embodiment of the present technology with the cartridge having a locking member.
FIG. 16 is a partially exploded perspective view illustrating a bag configured in accordance with an embodiment of the present technology.
FIG. 17 is a perspective view illustrating a console assembly configured in accordance with an embodiment of the present technology and including the console of FIGS. 10A-10C, the bag of FIG. 16, and related cryotherapeutic-system components during a treatment procedure.
FIG. 18 is an exploded perspective view illustrating a shell and the console of FIGS. 10A-10C configured in accordance with an embodiment of the present technology.
FIG. 19 is a perspective view illustrating the shell of FIG. 18, the console of FIGS. 10A-10C, and related cryotherapeutic-system components during a treatment procedure.
FIG. 20A is a perspective view illustrating a bag configured in accordance with an embodiment of the present technology.
FIG. 20B is a perspective view illustrating a console assembly configured in accordance with an embodiment of the present technology and including the bag of FIG. 20A.
FIG. 20C is a perspective view illustrating the console assembly of FIG. 20B during a cartridge-loading procedure.
FIG. 21A is a perspective view illustrating a bag configured in accordance with an embodiment of the present technology.
FIG. 21B is a perspective view illustrating a console assembly configured in accordance with an embodiment of the present technology and including the bag of FIG. 21A.
FIG. 22 is a perspective view illustrating a cartridge, a cartridge housing, and a cap configured in accordance with an embodiment of the present technology.
FIG. 23 is a perspective view illustrating a cartridge and a cartridge housing configured in accordance with an embodiment of the present technology with the cartridge including a cap having a first threaded portion and the cartridge housing including a second threaded portion.
FIG. 24 is a perspective view illustrating a cartridge and a cartridge housing configured in accordance with an embodiment of the present technology with the cartridge including a tip portion having a locking member.
FIG. 25 is a perspective view illustrating a cartridge and a cartridge housing configured in accordance with an embodiment of the present technology with the cartridge including a gripping portion.
FIG. 26A is a perspective view illustrating a console assembly configured in accordance with an embodiment of the present technology and including a drape and a console having a user interface during a treatment procedure.
FIG. 26B is a partially schematic diagram illustrating the console assembly of FIG. 26A during use of the user interface.
FIG. 26C is a partially schematic diagram illustrating the console of FIG. 26A during loading of a cartridge.
FIG. 27 is a perspective view illustrating a console assembly configured in accordance with an embodiment of the present technology and including a hub and a primary housing during a treatment procedure.
FIG. 28 is a partially schematic diagram illustrating a cryotherapeutic system configured in accordance with an embodiment of the present technology and including an electrical adapter.
FIGS. 29A-29D are perspective views illustrating intervening cryotherapeutic-system components configured in accordance with embodiments of the present technology and positioned between a hub and a primary housing of a console assembly.
FIGS. 30A-30B are perspective views illustrating a kit configured in accordance with an embodiment of the present technology and including selected cryotherapeutic-system components.
FIG. 31 is a perspective view illustrating a cryotherapeutic system configured in accordance with an embodiment of the present technology and including a ribbon having cartridges.
FIG. 32 is a perspective view illustrating a cryotherapeutic system configured in accordance with an embodiment of the present technology and including a console having a storage space.
FIG. 33 is a perspective view illustrating a console configured in accordance with another embodiment of the present technology and including a body and a neck.
FIG. 34 is a perspective view illustrating a console configured in accordance with another embodiment of the present technology and including a plurality of cartridges.
FIG. 35 is a perspective view illustrating a shell configured in accordance with an embodiment of the present technology and including first and second wings and first and second living hinges.
FIG. 36 is a perspective view illustrating a shell configured in accordance with an embodiment of the present technology and including a sealing member.
FIG. 37 is a perspective view illustrating a cryotherapeutic system configured in accordance with an embodiment of the present technology and including a console having a tower with an angled upper portion.
FIG. 38 is a perspective view illustrating a cryotherapeutic system configured in accordance with an embodiment of the present technology and including a console having a pole.
FIG. 39A is a perspective view illustrating a console assembly configured in accordance with an embodiment of the present technology and including a console having a body with canister housings behind sliding doors.
FIG. 39B is a rear profile view illustrating the cryotherapeutic system of FIG. 39A.
FIG. 39C is a perspective view illustrating the cryotherapeutic system of FIG. 39A with one of the canister housings exposed.
FIG. 40A is a perspective view illustrating a console assembly configured in accordance with an embodiment of the present technology and including a console having an elongated body.
FIG. 40B is a rear profile view illustrating the cryotherapeutic system of FIG. 40A.
FIG. 41A is a perspective view illustrating a console assembly configured in accordance with an embodiment of the present technology and including a console and a table.
FIG. 41B is a rear profile view illustrating the cryotherapeutic system of FIG. 41A.
FIG. 42A is an exploded perspective view illustrating a cryotherapeutic system configured in accordance with an embodiment of the present technology and including a handle assembly having a cartridge housing.
FIG. 42B is a perspective view illustrating the cryotherapeutic system of FIG. 42A.
FIG. 43 is a perspective view illustrating a selection of user-interface devices configured in accordance with embodiments of the present technology.
FIG. 44 is a perspective view illustrating a user-interface device configured in accordance with an embodiment of the present technology and including a recess and a cover.
FIG. 45A is a plan view illustrating a pre-cooling assembly configured in accordance with an embodiment of the present technology.
FIG. 45B is a cross-sectional view illustrating the pre-cooling assembly of FIG. 45A.
FIG. 46 is a cross-sectional view illustrating a pre-cooling assembly configured in accordance with an embodiment of the present technology and including a tubular member having a valve.
FIG. 47A is a cross-sectional view illustrating a pre-cooler configured in accordance with an embodiment of the present technology and including a flow separator.
FIG. 47B is a cross-sectional view illustrating the pre-cooler of FIG. 47A taken along the line 47B-47B.
FIG. 48A is a cross-sectional view illustrating a pre-cooling assembly configured in accordance with an embodiment of the present technology and including a flow separator.
FIG. 48B is a cross-sectional view illustrating the pre-cooling assembly of FIG. 48A taken along the line 48B-48B.
FIG. 49 is a partially cross-sectional view illustrating a pre-cooling assembly configured in accordance with an embodiment of the present technology and including a tubular member coiled around an exhaust portal within a handle.
FIG. 50 is a partially cross-sectional view illustrating a pre-cooling assembly configured in accordance with an embodiment of the present technology and including a tubular member coiled near an exhaust portal within a handle.
FIG. 51 is a plan view illustrating a machine display configured in accordance with an embodiment of the present technology.
FIG. 52 is a profile view illustrating a display configured in accordance with an embodiment of the present technology and including a primary-stage list and an anatomical image.
FIG. 53 is a profile view illustrating a display configured in accordance with an embodiment of the present technology and including a plot of temperature versus time for a cryotherapeutic-cycling stage of a treatment procedure.
FIG. 54 is a profile view illustrating a display configured in accordance with an embodiment of the present technology and including a plot of temperature versus time for a cryotherapeutic-cycling stage of a treatment procedure.
FIG. 55 is a profile view illustrating a display configured in accordance with an embodiment of the present technology and including a circular area having a plurality of segments corresponding to portions of a cryotherapeutic-cycling stage of a treatment procedure.
FIG. 56 is a profile view illustrating a display configured in accordance with an embodiment of the present technology and including a procedure list and a procedure report.
FIG. 57 is a conceptual diagram illustrating the sympathetic nervous system and how the brain communicates with the body via the sympathetic nervous system.
FIG. 58 is an enlarged anatomical view illustrating nerves innervating a left kidney to form a renal plexus surrounding a left renal artery.
FIGS. 59A-59B are anatomical and conceptual views, respectively, illustrating a human body including a brain and kidneys and neural efferent and afferent communication between the brain and kidneys.
FIGS. 60A-60B are anatomic views illustrating, respectively, an arterial vasculature and a venous vasculature of a human.
FIGS. 61-66 are illustrations of additional cryotherapeutic devices configured in accordance with embodiments of the present technology.
FIG. 67 is a partially schematic view illustrating a pre-cooling assembly configured in accordance with an embodiment of the present technology.

### DETAILED DESCRIPTION

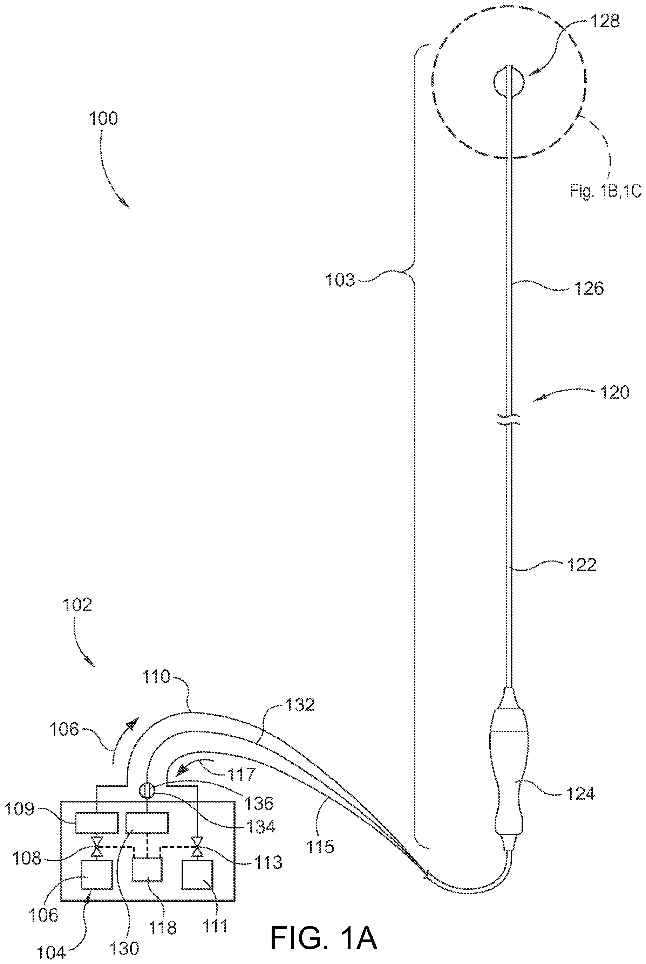

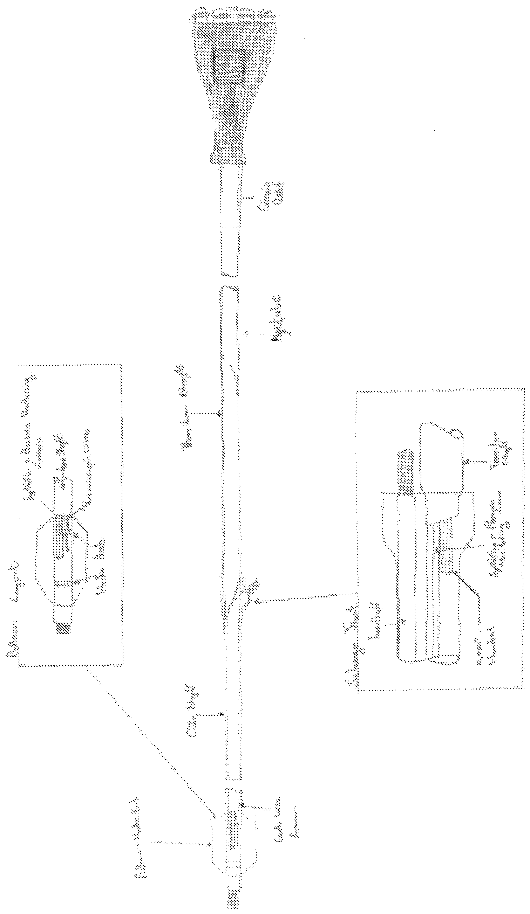

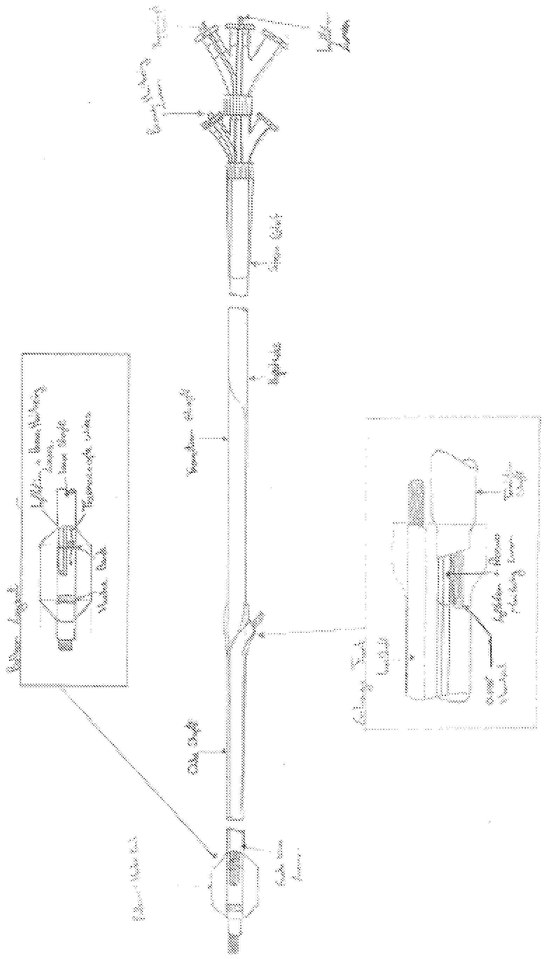

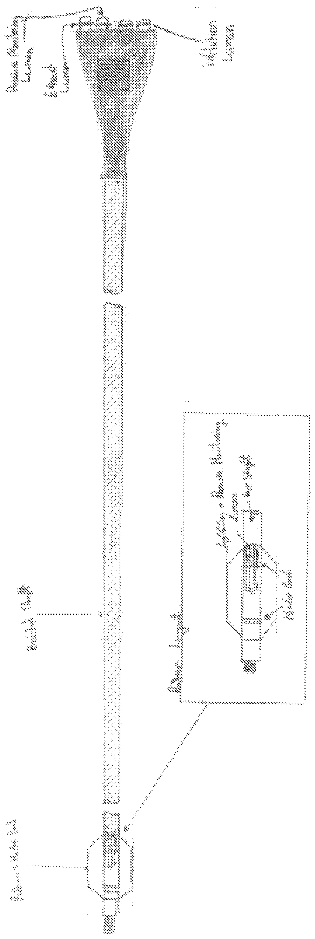

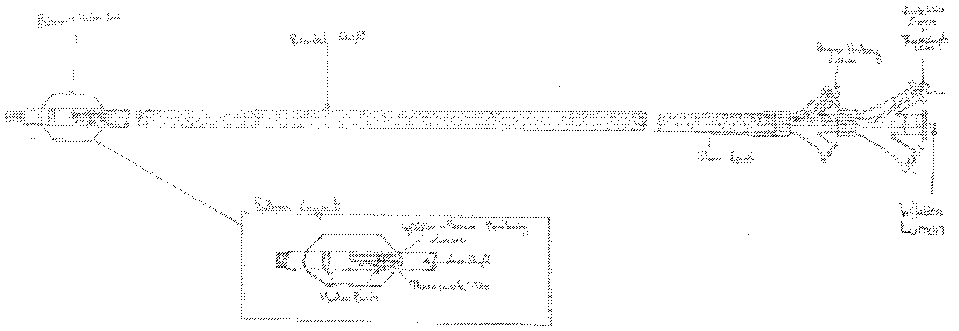

Specific details of several embodiments of the present technology are described herein with reference to FIGS. 1A-67. Although many of the embodiments are described herein with respect to devices, systems, and methods for modulation of renal nerves using cryotherapeutic approaches, other applications and other embodiments in addition to those described herein are within the scope of the present technology. Additionally, several other embodiments of the present technology can have different configurations, components, or procedures than those described herein. A person of ordinary skill in the art, therefore, will understand that the present technology can have other embodiments with additional elements and features, or the present technology can have other embodiments without several of the elements and features described herein with reference to FIGS. 1A-67.

Generally, unless the context indicates otherwise, the terms "distal" and "proximal" within this description reference a position relative to a refrigerant source. For example, "proximal" can refer to a position closer to a refrigerant source, and "distal" can refer to a position that is more distant from a refrigerant source. With respect to some cryotherapeutic-system components described herein, however, the terms "distal" and "proximal" can reference a position relative to an operator and/or a location in the vasculature (e.g., an incision along the vasculature). For ease of reference, throughout this disclosure identical reference numbers are used to identify similar or analogous components or features, but the use of the same reference number does not imply that the parts should be construed to be identical. Indeed, in many examples described herein, the identically numbered parts are distinct in structure and/or function. The headings provided herein are for convenience only.

### A. Cryotherapy and Renal Neuromodulation

Cryotherapeutic systems and components of cryotherapeutic systems configured in accordance with embodiments of the present technology can be configured for renal neuromodulation, i.e., the partial or complete incapacitation or other effective disruption of nerves innervating the kidneys. In particular, renal neuromodulation can include inhibiting, reducing, and/or blocking neural communication along neural fibers (i.e., efferent and/or afferent nerve fibers) innervating the kidneys. Such incapacitation can be long-term (e.g., permanent or for periods of months, years, or decades) or short-term (e.g., for periods of minutes, hours, days, or weeks). Renal neuromodulation can contribute to the systemic reduction of sympathetic tone or drive. Accordingly, renal neuromodulation is expected to be useful in treating clinical conditions associated with systemic sympathetic overactivity or hyperactivity, particularly conditions associated with central sympathetic overstimulation. Renal neuromodulation is expected to efficaciously treat hypertension, heart failure, acute myocardial infarction, metabolic syndrome, insulin resistance, diabetes, left ventricular hypertrophy, chronic and end-stage renal disease, inappropriate fluid retention in heart failure, cardio-renal syndrome, polycystic kidney disease, polycystic ovary syndrome, osteoporosis, and sudden death, among others. Furthermore, renal neuromodulation can potentially benefit a variety of organs and bodily structures innervated by sympathetic nerves. A more detailed description of pertinent patient anatomy and physiology is provided below.

Various techniques can be used to partially or completely incapacitate neural pathways, such as those innervating the kidneys. Cryotherapy, for example, includes cooling tissue at a target site in a manner that modulates neural function. The mechanisms of cryotherapeutic tissue damage include, for example, direct cell injury (e.g., necrosis), vascular injury (e.g., starving the cell from nutrients by damaging supplying blood vessels), and sublethal hypothermia with subsequent apoptosis. Exposure to cryotherapeutic cooling can cause acute cell death (e.g., immediately after exposure) and/or delayed cell death (e.g., during tissue thawing and subsequent hyperperfusion). Several embodiments of the present technology include cooling a structure at or near an inner surface of a renal artery wall such that proximate (e.g., adjacent) tissue is effectively cooled to a depth where sympathetic renal nerves reside. For example, the cooling structure can be cooled to the extent that it causes therapeutically effective cryogenic renal neuromodulation. Sufficiently cooling at least a portion of a sympathetic renal nerve is expected to slow or potentially block conduction of neural signals to produce a prolonged or permanent reduction in renal sympathetic activity.

Cryotherapy has certain characteristics that can be beneficial for renal neuromodulation. For example, rapidly cooling tissue can provide an analgesic effect such that cryotherapies may be less painful than ablating tissue at high temperatures. Cryotherapies may thus require less analgesic medication to maintain patient comfort during a procedure compared to heat-ablation procedures. Additionally, reducing pain can reduce patient movement and thereby increase operator success or reduce procedural complications. Cryotherapy also typically does not cause significant collagen tightening, and therefore is not typically associated with vessel stenosis. Cryotherapies generally include cooling at temperatures that cause cryotherapeutic applicators to adhere to moist tissue. This can be beneficial because it can promote stable, consistent, and continued contact during treatment. The typical conditions of treatment can make this an attractive feature because, for example, a patient can move during treatment, a catheter associated with an applicator can move, and/or respiration can cause the kidneys to rise and fall and thereby move the renal arteries. In addition, blood flow is pulsatile and causes the renal arteries to pulse. Adhesion associated with cryotherapeutic cooling also can be advantageous when treating short renal arteries in which stable intravascular positioning can be more difficult to achieve.

### B. Introductory Examples

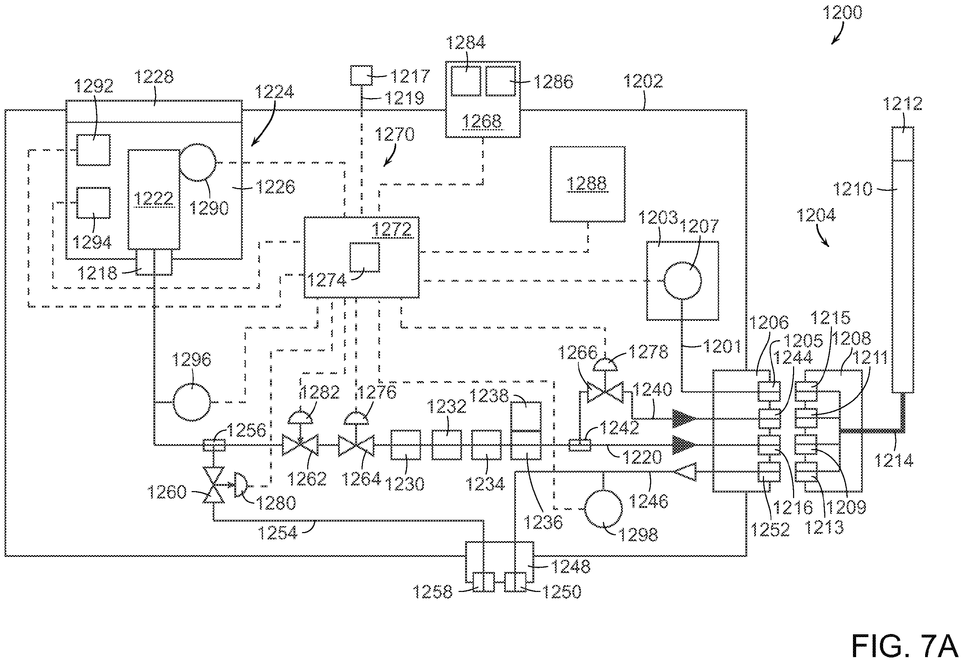

Introductory examples of cryotherapeutic systems, cryotherapeutic methods, and cryotherapeutic-system components configured in accordance with embodiments of the present technology are described in this section with reference to FIGS. 1A-6. Although this disclosure is primarily directed to cryotherapeutic-system components configured to be outside the vasculature, for purposes of introduction, FIGS. 1A-6 are described in this section with emphasis on both cryotherapeutic-system components configured to be outside the vasculature and cryotherapeutic-system components configured to be inside the vasculature. It will be appreciated that specific elements, substructures, advantages, uses, and/or other features of the embodiments described with reference to FIGS. 1A-6 can be suitably interchanged, substituted, or otherwise configured with one another and/or with the embodiments described with reference to FIGS. 7A-67 in accordance with additional embodiments of the present technology. Furthermore, suitable elements of the embodiments described with reference to FIGS. 1A-6 can be used as stand-alone and/or self-contained devices.

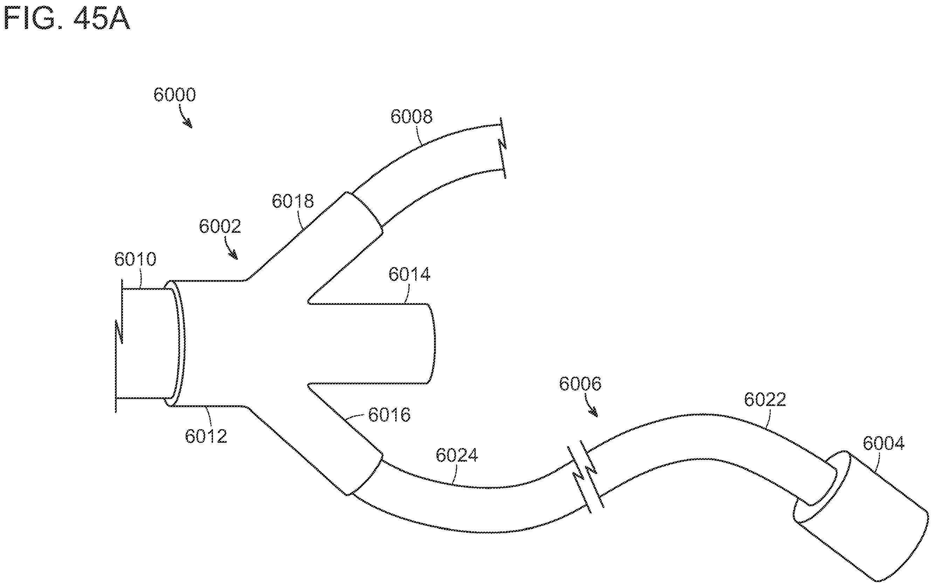

FIG. 1A is a partially schematic diagram illustrating a cryotherapeutic system 100 that can include a console 102 and a cryotherapeutic device 103. The console 102 can include a supply container 104, a refrigerant 106 within the supply container 104, and a supply control valve 108 in fluid communication with the supply container 104. The supply container 104 can be, for example, a cartridge (e.g., a single-use cartridge) or a canister (e.g., a tank, a cylinder, or another suitable container that is not a cartridge). In some embodiments, the supply container 104 can be configured to contain a sufficient quantity of refrigerant 106 to perform multiple treatment procedures. For example, the supply container 104 can be a refillable cylinder. The supply container 104 can be configured to contain the refrigerant 106 at a desired pressure. For example, the supply container 104 can be configured to contain N₂O at a pressure of about 750 psi or greater, which can allow the N₂O to be in at least substantially liquid phase at about ambient temperature. In other embodiments, the refrigerant 106 can include CO₂, a hydrofluorocarbon (e.g., Freon®, R-410A, etc.), and/or another suitable material that can be contained within the supply container 104 at a sufficiently high pressure to be in at least substantially liquid phase at about ambient temperature. For example, R-410A can be in at least substantially liquid phase at about ambient temperature when contained at a pressure of about 210 psi. In some embodiments, the cryotherapeutic system 100 can be configured to pre-cool the refrigerant 106, which can increase the cooling potential of the refrigerant 106. The console 102, for example, can include a pre-cooler 109. In other embodiments, the pre-cooler 109 can have a different position within the cryotherapeutic system 100. Pre-cooling is described, for example, below with reference to FIGS. 45A-50.

The console 102 can include a supply line 110 configured to transport the refrigerant 106 to the cryotherapeutic device 103. The supply control valve 108 can be operably coupled to the supply line 110, and can be configured to manually or automatically control the flow of refrigerant 106 along the supply line 110. The console 102 can further include a pump 111 (e.g., a vacuum pump), a back-pressure control valve 113, and an exhaust line 115. The exhaust line 115 can be configured to receive exhausted refrigerant 117 from the cryotherapeutic device 103, and the back-pressure control valve 113 can be operably coupled to the exhaust line 115. In some embodiments, the pump 111 can be a DC-powered pump. The pump 111 can be configured to reduce the back pressure of exhausted refrigerant 117 to below ambient pressure. Reducing the back pressure of exhausted refrigerant 117 to below ambient pressure using the pump 111 (e.g., in conjunction with increasing the flow rate of refrigerant 106 using the supply control valve 108) can increase the refrigeration potential of the refrigerant 106. In other embodiments, the exhausted refrigerant 117 can exhaust to about ambient pressure. The console 102 can include a controller 118 configured to operate the supply control valve 108 and/or the back-pressure control valve 113. The controller 118, for example, can include a processor (not shown) or dedicated circuitry (not shown) configured to implement a computerized algorithm for executing a treatment procedure or a portion of a treatment procedure automatically.

As shown in FIG. 1A, the cryotherapeutic device 103 can include a shaft 120 having a proximal portion 122, a handle 124 at a proximal region of the proximal portion 122, and a distal portion 126 extending distally relative to the proximal portion 122. The cryotherapeutic device 103 can further include a cooling assembly 128 at the distal portion 126 of the shaft 120. The shaft 120 can be configured to locate the distal portion 126 and/or the cooling assembly 128 intravascularly at a treatment site proximate (e.g., in or near) a renal artery or renal ostium, and the cooling assembly 128 can be configured to provide therapeutically effective cryogenic renal neuromodulation at the treatment site.

The console 102 can include a pressure sensor 130 (e.g., a PX209-100G5V pressure transducer made by OMEGA Engineering Inc. of Stamford, CT) and a pressure line 132. The pressure sensor 130 can be configured to monitor a pressure within a portion of the cryotherapeutic device 103 during a treatment procedure. The pressure sensor 130 can be operably coupled to the controller 118 and can be part of a feedback loop configured to control the supply control valve 108 and/or the back-pressure control valve 113. Flow of the refrigerant 106 to and/or from the cryotherapeutic device 103 can be regulated in response to a sensed pressure. For example, the pressure sensor 130 can be configured to indicate a pressure above a predetermined threshold value or range (e.g., a value or range at or below a burst pressure of a balloon (not shown) of the cooling assembly 128). In response, the controller 118 can be configured to decrease or terminate flow of the refrigerant 106 to the cooling assembly 128 by at least partially closing the supply control valve 108. Similarly, the controller 118 can be configured to increase flow of the refrigerant 106 from the cooling assembly 128 by reducing the back pressure of the exhausted refrigerant 117 (e.g., by using the pump 111). In other embodiments, the pressure sensor 130 can be coupled directly to the supply control valve 108 and/or the back-pressure control valve 113 to automatically regulate the supply control valve 108 and/or the back-pressure control valve 113 in response to a sensed pressure. The cryotherapeutic system 100 can be configured to verify that the pressure sensor 130 is calibrated properly before initiating a treatment procedure or a portion thereof. For example, the cryotherapeutic system 100 can be configured to automatically check the functionality of the pressure sensor 130 as the cryotherapeutic system 100 powers on by comparing a pressure reading from the pressure sensor 130 with the ambient pressure.

In some embodiments, the pressure line 132 can include an adapter 134 (e.g., a quick-connect adapter). The adapter 134 can include an internal channel 136 configured to fluidly connect the pressure line 132 to the pressure sensor 130. In other embodiments, the channel 136 can be a reservoir. The channel 136 can have a substantially small volume so as not to disrupt a pressure differential between the pressure line 132 and the pressure sensor 130. For example, the channel 136 can have an internal volume less than about 0.1 cc. In other embodiments, the channel 136 can have a larger internal volume. The adapter 134 can have a variety of suitable positions within the cryotherapeutic system 100. For example, the adapter 134 can be along the pressure line 132 proximate the pressure sensor 130. In other embodiments, the adapter 134 can be a portion of the cryotherapeutic device 103. For example, the adapter 134 can be configured to couple a pressure-monitoring lumen (not shown) extending through the shaft 120 to the pressure line 132 at the handle 124 or at another position proximate the proximal portion 122 of the shaft 120. In these and other embodiments, the adapter 134 can be configured to allow the pressure-monitoring lumen and/or the pressure line 132 to be detached from the pressure sensor 130 after a treatment procedure. This can allow the pressure-monitoring lumen and/or the pressure line 132 to be discarded and the pressure sensor 130 to be reused (e.g., along with the handle 124 and/or the console 102) for subsequent treatment procedures without disrupting the accuracy of the pressure reading at the pressure sensor 130.

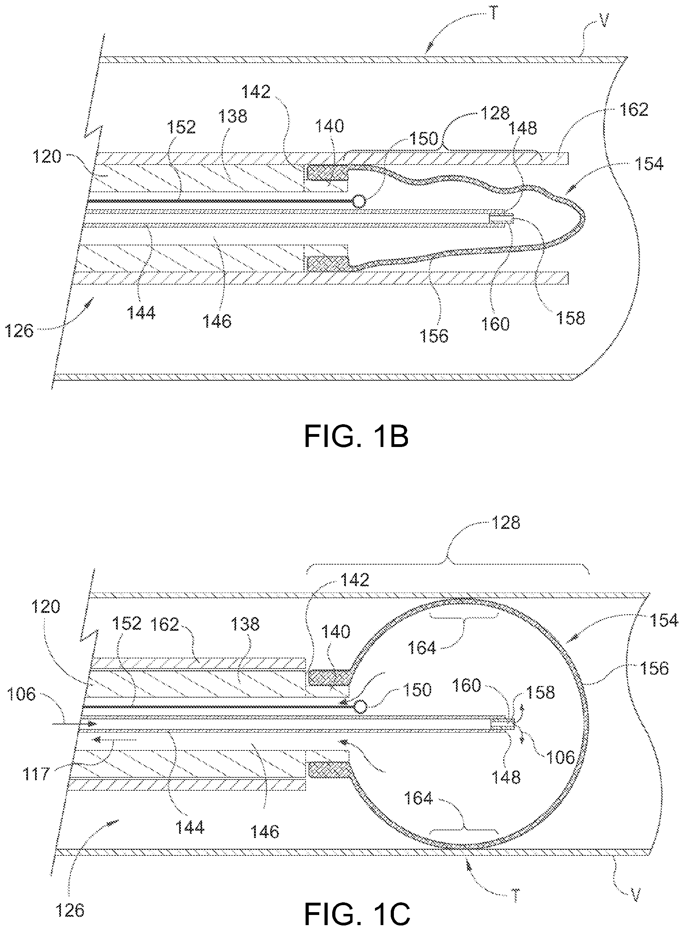

FIGS. 1B-1C are cross-sectional views illustrating the cooling assembly 128 of the cryotherapeutic device 103 of FIG. 1A in a delivery state (e.g., FIG. 1B shows a low-profile or collapsed configuration) and a deployed state (e.g., FIG. 1C shows an expanded configuration). As shown in FIGS. 1B-1C, the distal portion 126 can include a first zone 138 and a second zone 140 (shown separated by broken lines) recessed inwardly relative to the first zone 138. The distal portion 126 can further include a step 142 demarcating the first zone 138 from the second zone 140. In some embodiments, the step 142 can be a rabbet (e.g., an annular or other circumferential groove configured to be fitted with another member). The first zone 138 can have a first outer dimension or first cross-sectional dimension (e.g., area or diameter), and the second zone 140 can have a second outer dimension or second cross-sectional dimension that is smaller than the first dimension.

The cryotherapeutic device 103 (FIG. 1A) can include a supply lumen 144 and an exhaust lumen 146 along at least a portion of the shaft 120. The supply lumen 144 can be a relatively small tube configured to retain the refrigerant 106 in at least substantially liquid phase at relatively high pressure. The inner diameter of the supply lumen 144 can be selected such that at least a portion of the refrigerant 106 reaching the cooling assembly 128 is in liquid phase at a distal end 148 of the supply lumen 144. The exhaust lumen 146 can be within an outer tube, and the supply lumen 144 can extend within the exhaust lumen 146 (e.g., at least along the distal portion 126 of the shaft 120). The cryotherapeutic device 103 can include a sensor 150 (e.g., a temperature sensor or a pressure sensor) and a lead 152 extending between the sensor 150 and the controller 118 (FIG. 1A). In some embodiments, the cryotherapeutic device 103 can include more than one sensor 150 and/or more than one lead 152. The cryotherapeutic system 100 (FIG. 1A) can be configured to verify the proper calibration of the sensor 150 before a treatment procedure. For example, when the sensor 150 is a temperature sensor, the cryotherapeutic system 100 can be configured to automatically compare a measured temperature from the sensor 150 with room temperature as the cryotherapeutic system 100 initiates a power-up cycle.

As shown in FIGS. 1B-1C, the cooling assembly 128 can include an applicator 154 and a balloon 156 or another type of expandable member. In other embodiments, the balloon 156 can be replaced with a non-expandable member that includes an internal expansion chamber. The balloon 156 can be configured to fully or partially occlude a renal artery or renal ostium. The cooling assembly 128 can further include an orifice 158 in fluid communication with the balloon 156. In some embodiments, the cooling assembly 128 can include a capillary tube 160 inserted into the distal end 148 of the supply lumen 144, and the orifice 158 can be at a distal end of the capillary tube 160. In other embodiments, the distal end 148 of the supply lumen 144 can include the orifice 158. The diameter of the capillary tube 160 and/or the orifice 158 can be less than that of the supply lumen 144, which can cause the orifice 158 to impede flow of the refrigerant 106 in or near the balloon 156. Accordingly, the refrigerant 106 can experience a pressure drop as it enters the balloon 156, which can cause evaporation of the refrigerant 106 within the cooling assembly 128. This can concentrate cooling at the cooling assembly 128. In other embodiments, the supply lumen 144 can have a substantially constant inner diameter such that the orifice 158 has a diameter at least equal to that of the supply lumen 144. In these and other embodiments, the cryotherapeutic device 103 (FIG. 1A) can include hardware (e.g., valves, flow and pressure gauges, etc.) and/or software in the handle 124 (FIG. 1A), in the console 102 (FIG. 1A), and/or in another suitable cryotherapeutic-system component to control the flow of refrigerant 106 through the supply lumen 144. Such hardware and software can be useful to concentrate cooling toward the distal portion 126 of the shaft 120. The orifice 158 can be sized relative to the area and/or length of the exhaust lumen 146 at the distal portion 126 to provide a sufficient flow rate of the refrigerant 106, to produce a sufficient pressure drop in the cooling assembly 128, and/or to allow for sufficient venting of the exhausted refrigerant 117 through the exhaust lumen 146.

The cryotherapeutic device 103 can include a sheath 162 (e.g., a guide sheath). In some embodiments, the sheath 162 can be 8 Fr or smaller. For example, the sheath 162 can be a 6 Fr guide sheath configured to accommodate renal arteries. During a treatment procedure, the cooling assembly 128 can be passed intravascularly to a target site (T) within a vessel (V) while in the delivery state (FIG. 1B). With reference to FIG. 1C, the cooling assembly 128 and the sheath 162 can then be moved relative to each other such that the cooling assembly 128 extends distally beyond the sheath 162. For example, the sheath 162 can be pulled proximally and/or the cooling assembly 128 can be pushed distally. The refrigerant 106 can pass through the supply lumen 144, through the orifice 158, and into the balloon 156. As the refrigerant 106 passes through the orifice 158, a portion of the refrigerant 106 can expand into gaseous phase inflating the balloon 156 and/or causing a significant temperature drop in the balloon 156. The portion of the applicator 154 contacting the vessel at the target site can be a heat-transfer region 164 that, together with the refrigerant 106 in the balloon 156, can cause therapeutically effective cryogenic renal neuromodulation. The exhausted refrigerant 117 can pass in a proximal direction through the exhaust lumen 146. Although the balloon 156 is shown in FIG. 1C with a generally spherical shape, the balloon 156 can have other suitable shapes in other embodiments. For example, the balloon 156 can be elongated with tapered proximal and distal ends.

The cooling assembly 128 can be configured to fully or partially occlude the vessel and/or to cause fully circumferential or partially circumferential ablation at the target site. Fully occluding the vessel can increase the cooling power at the heat-transfer region 164 by reducing convective heat exchange with blood flow. Although occlusion of a renal artery for an excessive period of time can potentially cause ischemia of a kidney, it has been found that a renal artery can be fully occluded for a period of time (e.g., 2-5 minutes) sufficient to complete therapeutically effective cryogenic renal neuromodulation at a target site without causing ischemia of a kidney. In some embodiments, the controller 118 (FIG. 1A) can be configured to limit the duration of flow of the refrigerant 106 (e.g., to 2-5 minutes). For example, the controller 118 can include a timer (not shown) configured to control the supply control valve 108 (FIG. 1A). In other embodiments, a timer can be incorporated into the handle 124 (FIG. 1A) or another portion of the cryotherapeutic device 103. If present, the sensor 150 can provide feedback to the controller 118 to regulate or control the cryotherapeutic system 100 (FIG. 1A). In some embodiments, the controller 118 can be configured to execute a fully automated control algorithm. In other embodiments, the controller 118 can be configured to execute a control algorithm that utilizes user input. Furthermore, in some embodiments, the duration of flow of the refrigerant 106 can be limited by the volume of the refrigerant 106 in the supply container 104 (FIG. 1A).

The sensor 150 can have positions other than the position shown in FIGS. 1B-1C. For example, the sensor 150 can be a thermocouple positioned on an outer surface of the balloon 156 and can be configured to provide a reading (e.g., a real-time reading) of the external temperature of the balloon 156. In these and other embodiments, the cryotherapeutic system 100 (FIG. 1A) can be regulated via the controller 118 (FIG. 1A) (e.g., using a software control loop) such that the cooling-power output increases or decreases based on differences between the real-time reading of the external temperature of the balloon 156 and a predetermined treatment temperature (e.g., -40°C, -60°C, etc.). For example, the cooling-power output can be regulated by switching valves (e.g., the supply control valve 108 (FIG. 1A) and/or the back-pressure control valve 113 (FIG. 1A)) on and off at various stages of a treatment procedure in response to measured temperatures. In other embodiments, the cooling-power output can be modulated using proportional control. For example, the delivery pressure of the refrigerant 106 and/or the exhaust pressure of the exhausted refrigerant 117 can be varied in response to the external temperature of the balloon 156. Accordingly, the sensor 150 can facilitate adjustment of the cryotherapeutic system 100 to compensate for variables that can affect cooling at the target site (e.g., variations in renal-artery diameter, blood flow through the renal artery, blood flow through other vessels in the vicinity of the renal artery, and/or other such variables).

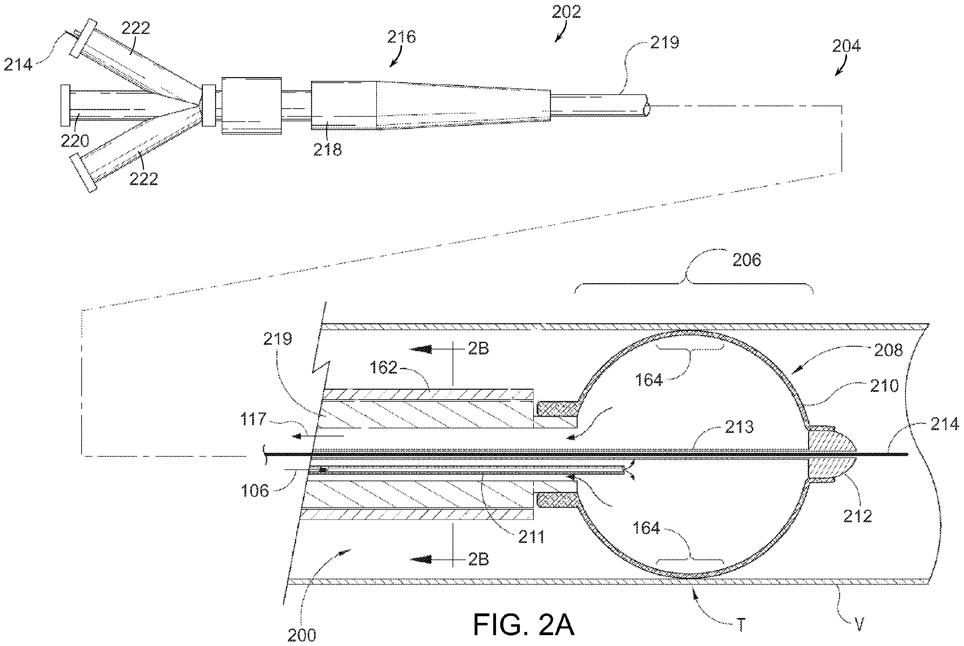

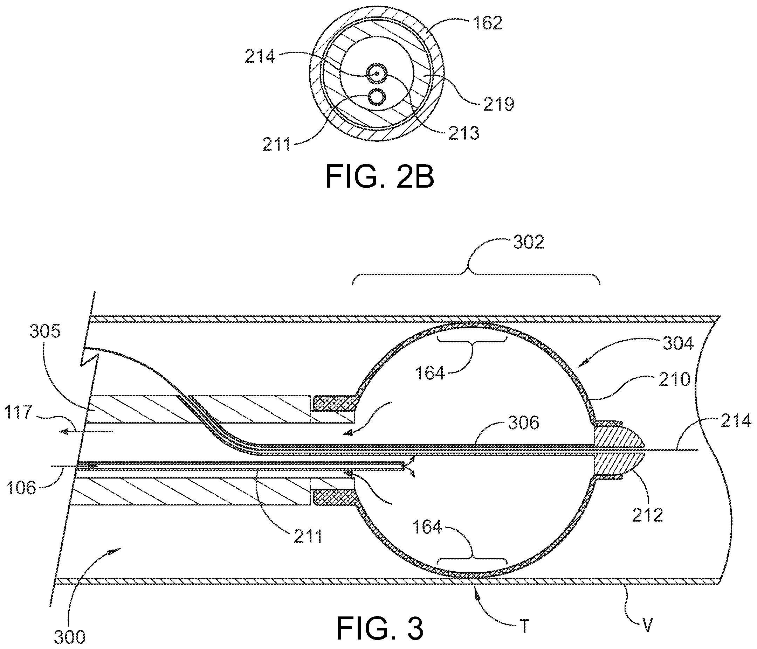

Cryotherapeutic devices configured in accordance with embodiments of the present technology can include a variety of suitable guidance configurations. FIG. 2A is a partially cross-sectional view illustrating a distal portion 200 and a proximal portion 202 of a cryotherapeutic device 204 having an over-the-wire configuration. FIG. 2B is a cross-sectional view illustrating the distal portion 200 taken along the line 2B-2B. The cryotherapeutic device 204 can include a cooling assembly 206 having an applicator 208 and a balloon 210. The cryotherapeutic device 204 can further include a supply lumen 211 configured to carry the refrigerant 106, a distal connector 212 at a distal portion of the balloon 210, and a guidewire lumen 213 extending to the distal connector 212. The guidewire lumen 213 can be configured to receive a guidewire 214 of the cryotherapeutic device 204. In some embodiments, the guidewire lumen 213 and the guidewire 214 can be used to guide the distal portion 200 through the vasculature. At the proximal portion 202, the cryotherapeutic device 204 can include an adapter 216 having a coupling member 218. The cryotherapeutic device 204 can further include a shaft 219, and the coupling member 218 can be connected to a proximal portion of the shaft 219. The adapter 216 can include a main passage 220 and a plurality of branch passages 222. In some embodiments, the adapter 216, the main passage 220, and/or the branch passages 222 can be portions of a handle (not shown). As shown in FIG. 2A, the guidewire 214 can extend through the shaft 219 from a proximal opening of one of the branch passages 222 to beyond a distal opening of the distal connector 212 in an over-the-wire configuration.

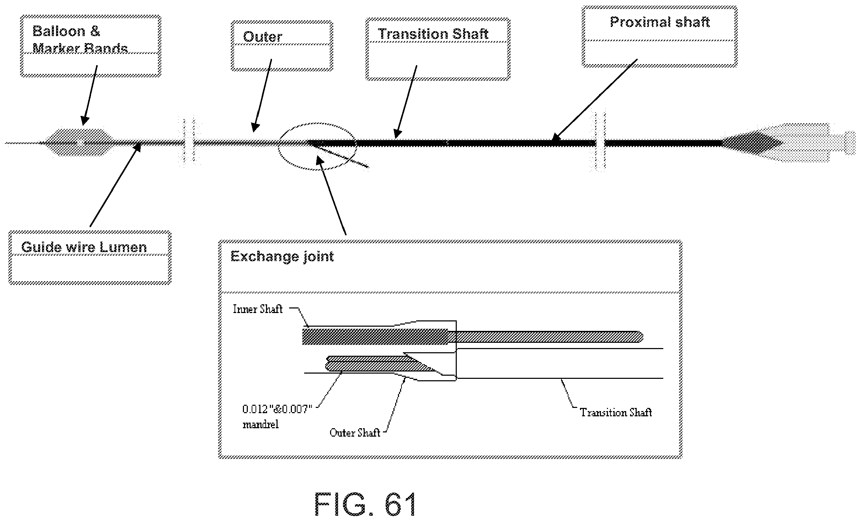

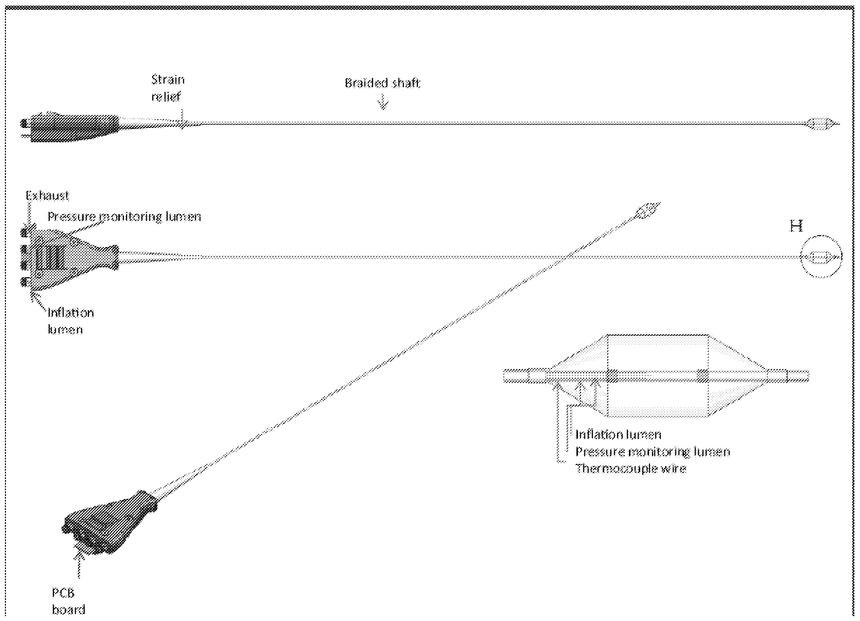

FIG. 3 is a cross-sectional view illustrating a distal portion 300 of a cryotherapeutic device (not separately identified) having a rapid-exchange configuration. The cryotherapeutic device can include a cooling assembly 302 having an applicator 304. The cryotherapeutic device can further include a shaft 305 and a guidewire lumen 306 extending between a sidewall of the shaft 305 and the distal connector 212. A proximal end of the guidewire lumen 306 can be accessible at any suitable portion of the sidewall between the proximal and distal ends of the shaft 305. Other guidance configurations (e.g., other over-the-wire and rapid-exchange configurations) are also possible. For example, FIG. 61 is an illustration of a cryotherapeutic device configured in accordance with an embodiment of the present technology and having a rapid-exchange configuration in which a proximal end of a guidewire lumen is accessible at an exchange joint between a transition shaft and an outer shaft. FIGS. 62-66 are illustrations of cryotherapeutic devices configured in accordance with embodiments of the present technology and having additional and/or alternative features, including other guidance configurations.

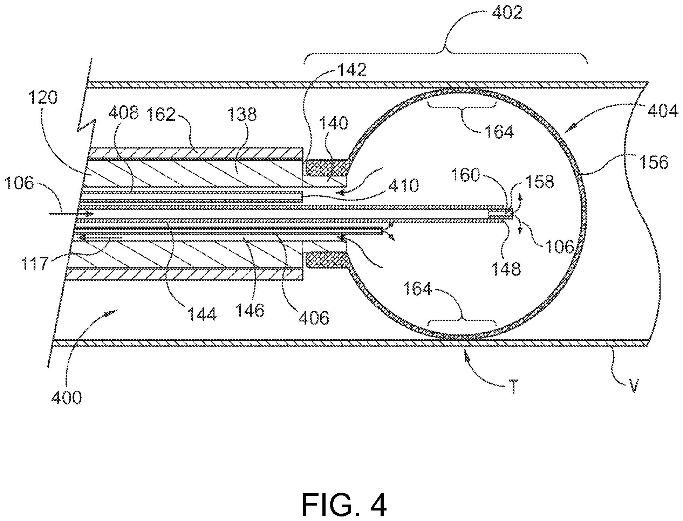

FIG. 4 is a cross-sectional view illustrating a distal portion 400 of a cryotherapeutic device (not separately identified) that can include a cooling assembly 402 having an applicator 404. The cryotherapeutic device can further include a secondary supply lumen 406 and a pressure-monitoring lumen 408. In some embodiments, the cryotherapeutic device can be used in the cryotherapeutic system 100 of FIG. 1A. For example, the secondary supply lumen 406 can be coupled to the supply container 104 (FIG. 1A) or another suitable supply reservoir. The secondary supply lumen 406 can be configured to deliver additional refrigerant or another suitable material to the balloon 156. In some embodiments, the secondary supply lumen 406 can be configured to deliver a pressurized or non-pressurized gas (e.g., air) to the balloon 156 before or during delivery of the refrigerant 106 to the balloon 156 via the orifice 158 to increase the pressure within the balloon 156. Additional gas in the balloon 156 can decrease the pressure drop from the supply line 110 (FIG. 1A) into the balloon 156, and thereby reduce refrigerant phase change and increase the operating temperature of the cooling assembly 402. Accordingly, the secondary supply lumen 406 can be used to initiate, restrict, and/or suspend the inflow of additional gas to the balloon 156 (e.g., using a valve (not shown)) and thereby regulate the temperature of the balloon 156. In some embodiments, control of the secondary supply lumen 406 can be independent of the console 102 (FIG. 1A). For example, the secondary supply lumen 406 can be attached to a syringe (not shown). When the secondary supply lumen 406 is coupled to a suitable supply reservoir other than the supply container 104, the secondary supply lumen 406 can be used, for example, to deliver gas into the balloon 156 (e.g., to inflate the balloon 156 and/or to position the balloon 156) before delivering the refrigerant 106 into the balloon 156.

The pressure-monitoring lumen 408 can extend through the shaft 120 and can include a distal opening 410 in fluid communication with the balloon 156. The dimensions (e.g., cross-sectional area, inner diameter, and/or outer diameter) of the pressure-monitoring lumen 408 can be large enough to sense a pressure reading within the balloon 156 with substantial accuracy, but small enough to reduce or prevent interference with outflow of the refrigerant exhaust 117 through the exhaust lumen 146. With reference to FIGS. 1A and 4 together, the pressure-monitoring lumen 408 can be coupled to the pressure line 132. During a treatment procedure, the pressure-monitoring lumen 408, the pressure line 132, and the pressure sensor 130 can be configured to provide a signal indicating a change in pressure within the balloon 156. For example, the pressure sensor 130 can be configured to monitor a threshold pressure below the burst pressure of the balloon 156. The threshold pressure can be selected to provide an adequate response time to react to a change in pressure before the balloon 156 ruptures. In other embodiments, the pressure sensor 130 can be configured to indicate when the balloon 156 operates outside its desired operating pressure range (e.g., 20-60 psi).

The time delay between the pressure at the distal opening 410 of the pressure-monitoring lumen 408 and the pressure reading at the pressure sensor 130 can depend on the volume of the pressure-monitoring lumen 408. The pressure-monitoring lumen 408 can have a volume that corresponds to a response time sufficient to adequately respond to a change in pressure in the balloon 156 (e.g., before rupture of the balloon 156). In some embodiments, the pressure sensor 130 can have a response time of less than about 1.5 seconds (e.g., a response time of less than about 1 second, less than about 0.2 second, less than about 0.1 second, or less than about 15 milliseconds). To enhance the accuracy of the pressure reading and decrease the response time of the pressure sensor 130, the length of the pressure-monitoring lumen 408 can be shortened. In some embodiments, the pressure-monitoring lumen 408 can be coupled to the pressure line 132 at the proximal portion 122 of the shaft 120 (e.g., at the handle 124), and the pressure line 132 can have a cross-sectional area similar to that of the pressure-monitoring lumen 408. In other embodiments, the pressure-monitoring lumen 408 can be coupled to the pressure sensor 130 at the handle 124 (e.g., omitting the pressure line 132) to shorten the total length between the distal opening 410 of the pressure-monitoring lumen 408 and the pressure sensor 130. In these and other embodiments, electrical wires (not shown) can be coupled to the pressure sensor 130 within the handle 124 to carry a signal to the console 102.

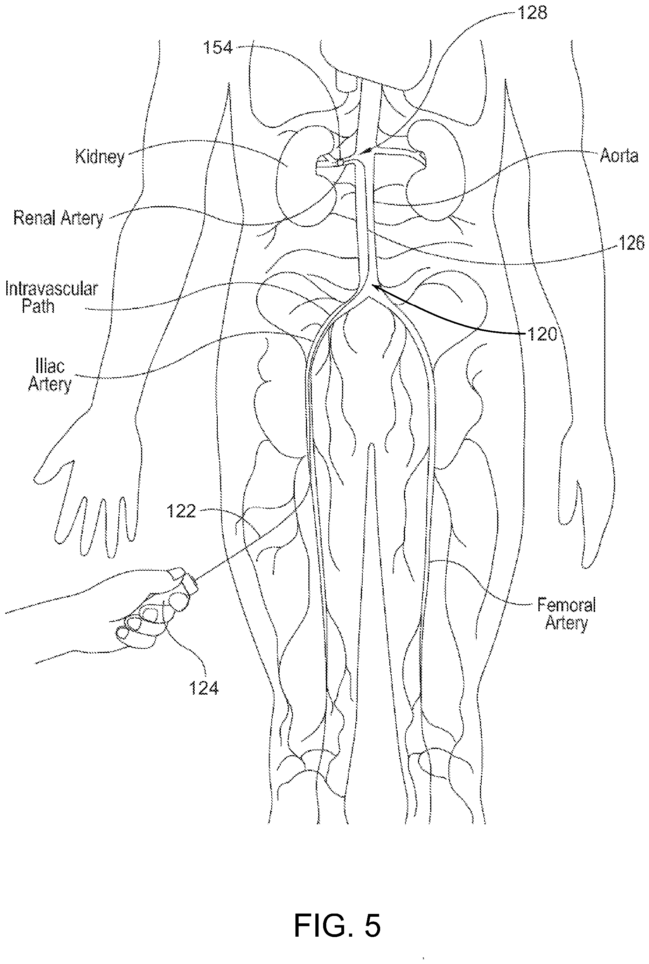

FIG. 5 is a partially schematic diagram illustrating cryogenically modulating renal nerves using the cryotherapeutic device 103 of FIG. 1A. The cryotherapeutic device 103 can be configured to provide access to the renal plexus through an intravascular path that leads to a renal artery. As shown in FIG. 5, a section of the proximal portion 122 of the cryotherapeutic device 103 can be exposed externally of the patient. By manipulating the proximal portion 122 from outside the intravascular path, the operator can advance the shaft 120 through tortuous portions of the intravascular path (e.g., via the femoral artery or a radial artery) and remotely manipulate the distal portion 126 of the cryotherapeutic device 103 (e.g., via an actuator (not shown) in the handle 124). For example, the shaft 120 can further include one or more pull wires (not shown) or other guidance devices configured to direct the distal portion 126 through the vasculature. Image guidance (e.g., CT, radiographic, IVUS, OCT, another suitable guidance modality, or combinations thereof) can be used to aid the operator's manipulation. After the applicator 154 of the cooling assembly 128 is adequately positioned in the renal artery or renal ostium, it can be expanded or otherwise deployed using the console 102 (FIG. 1A), the handle 124, and/or in another suitable manner until the applicator 154 contacts the inner wall of the renal artery or renal ostium. Cooling power from the applicator 154 can then be purposefully applied to tissue to induce one or more desired neuromodulating effects on localized regions of the renal artery or renal ostium and adjacent regions of the renal plexus, which lay intimately within, adjacent to, or in close proximity to the adventitia of the renal artery. The purposeful application of the cooling power, for example, can achieve neuromodulation along all or a portion of the renal plexus.

The neuromodulating effects can be generally a function of, at least in part, the temperature of the applicator 154, contact between the applicator 154 and the vessel wall, the dwell time of the applicator 154 while cooling, the number of cooling cycles (e.g., one or more cooling cycles separated by a warming period), and blood flow through the vessel. Desired cooling effects can include cooling the applicator 154 such that the temperatures of target neural fibers are below a desired threshold value or range to achieve therapeutically effective cryogenic renal neuromodulation. For example, the applicator 154 can be cooled to a temperature between about -88°C and about -60°C (e.g., between about -80°C and about - 40°C). Therapeutically effective cryogenic renal neuromodulation can occur within about 100 seconds (e.g., within about 90 seconds, within about 75 seconds, within about 60 seconds, or within about 30 seconds) of the applicator 154 reaching a cryogenic temperature when adjacent to the renal artery or renal ostium or of the applicator 154 being applied to the renal artery or renal ostium when the applicator 154 is already cryogenically cooled. In some embodiments, a treatment procedure can include two cooling cycles separated by a warming period. In other embodiments, a treatment procedure can include more than two cooling cycles separated by warming periods. The cooling cycles can have the same duration or different durations, such as between about 10 seconds and about 90 seconds each. The duration(s) of the warming periods can be sufficient to partially or completely thaw frozen matter at an interface between the applicator 154 and the inner wall of the renal artery or renal ostium. In some embodiments, the duration(s) of the warming periods can be between about 5 seconds and about 90 seconds. Individual warming periods between cooling cycles can last for the same amount of time or for different amounts of time.

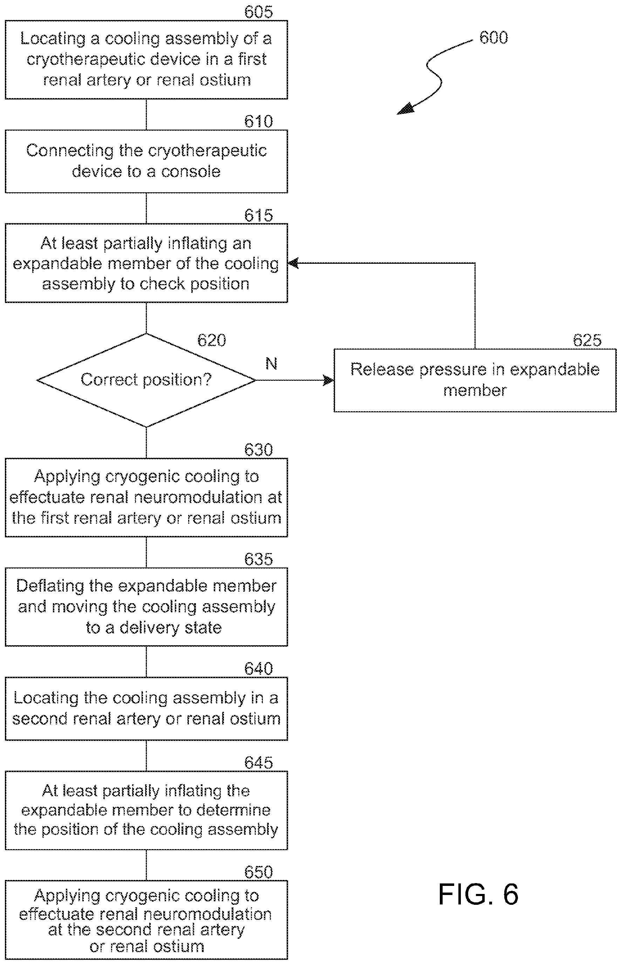

FIG. 6 is a block diagram illustrating a method 600 of cryogenically modulating renal nerves using the cryotherapeutic system 100 of FIG. 1A-1C. With reference to FIGS. 1A-1C and 6 together, the method 600 can include intravascularly locating the cooling assembly 128 in a delivery state to a first target site in or near a first renal artery or renal ostium (block 605). The cryotherapeutic device 103 and/or portions thereof (e.g., the cooling assembly 128) can be inserted into a guide catheter (not shown) to facilitate intravascular delivery of the cooling assembly 128. In some embodiments, the cryotherapeutic device 103 can be configured to fit within an 8 Fr guide catheter or a smaller guide catheter (e.g., 7 Fr, 6 Fr, etc.) to access small peripheral vessels. A guidewire (not shown) can be used to manipulate and enhance control of the shaft 120 and the cooling assembly 128 (e.g., in an over-the-wire or a rapid-exchange configuration). Radiopaque markers and/or markings (not shown) on the cryotherapeutic device 103 and/or the guidewire can facilitate placement of the cooling assembly 128 at the first target site. In some embodiments, a contrast material can be delivered distally beyond the cooling assembly 128, and fluoroscopy and/or other suitable imaging techniques can be used to aid in placement of the cooling assembly 128 at the first target site.

The method 600 can further include connecting the cryotherapeutic device 103 to the console 102 (block 610), and partially or fully inflating the balloon 156 of the cooling assembly 128 to determine whether the cooling assembly 128 is in the correct position at the first target site (blocks 615 and 620). The balloon 156 can be inflated with refrigerant 106 from the supply container 104 of the console 102 and/or with another suitable fluid (e.g., air) from a secondary fluid-supply reservoir (not shown) in fluid communication with the balloon 156. If the cooling assembly 128 is not in the correct position, at least some of the pressure in the balloon can be released (block 625). In some embodiments, the balloon 156 can be fully deflated by disconnecting the cryotherapeutic device 103 from the console 102 and using a syringe (not shown) to manually deflate the balloon via a proximal end portion of the shaft 120. In other embodiments, the cryotherapeutic device 103 can remain attached to the console 102 and a syringe (e.g., a stopcock syringe) (not shown) can be connected along the length of the shaft 120 to deflate the balloon 156. In other embodiments, the controller 118 can include one or more algorithms for partially or fully deflating the balloon 156.

Once the cooling assembly 128 is properly located within the first renal artery or renal ostium, the console 102 can be manipulated to initiate cryogenic cooling of the cooling assembly 128 and modulation of renal nerves at the first target site to cause partial or full denervation of the kidney associated with the first target site (block 630). Cryogenic cooling can be applied for one or more cycles (e.g., for 30-second increments, 60-second increments, 90-second increments, etc.) in one or more locations along the circumference and/or length of the first renal artery or renal ostium. For example, two 90-second cryogenic cooling cycles can be used with a partial or complete thaw between the cryogenic cooling cycles. In some embodiments, the balloon 156 can remain fully or partially inflated to maintain the position of the cooling assembly 128 at the first target site between the cooling cycles. The cooling cycles can be, for example, fixed periods or can be fully or partially dependent on detected temperatures (e.g., temperatures detected by a thermocouple (not shown) of the cooling assembly 128). In some embodiments, a first stage can include cooling tissue until a first target temperature is reached. A second stage can include maintaining cooling for a set period, such as 15-180 seconds (e.g., 90 seconds). A third stage can include terminating or decreasing cooling to allow the tissue to warm to a second target temperature higher than the first target temperature. A fourth stage can include continuing to allow the tissue to warm for a set period, such as 10-120 seconds (e.g., 60 seconds). A fifth stage can include cooling the tissue until the first target temperature (or a different target temperature) is reached. A sixth stage can include maintaining cooling for a set period, such as 15-180 seconds (e.g., 90 seconds). Finally, a seventh stage can include allowing the tissue to warm completely (e.g., to reach a body temperature).

After renal neuromodulation at the first renal artery or renal ostium, the method 600 can further include deflating the balloon 156 and retracting the cooling assembly 128 into a delivery state (block 635). The balloon 156 can be deflated by one of the methods described, for example, above with reference to block 625. In some embodiments, the cooling assembly 128 can be withdrawn back into the guide catheter after the balloon 156 is deflated. Furthermore, the cooling assembly 128 can be removed from the guide catheter during repositioning and temporarily stored in a sterile location (e.g., in a sterile saline solution). After removal from the first target site, the cooling assembly 128 can be located at a second target site in a second renal artery or renal ostium (block 640), and the balloon 156 can be expanded to confirm the position of the cooling assembly 128 (block 645). In some embodiments, a contrast material can be delivered distally beyond the cooling assembly 128, and fluoroscopy and/or another suitable imaging technique can be used to aid in navigating the cooling assembly 128 from the first target site to the second target site. If the supply container 104 of the console 102 is depleted, it can be refilled or removed and replaced with a new supply container (e.g., a disposable cartridge) to provide sufficient refrigerant for treatment at the second target site. If the console 102 was detached from the cryotherapeutic device 103 during repositioning of the cooling assembly 128, the console 102 can be reconnected to the cryotherapeutic device 103. The method 600 can further include modulation of renal nerves at the second target site to cause partial or full denervation of the kidney associated with the second target site (block 650).

### C. Selected Examples of Cryotherapeutic-System Configurations

Selected examples of cryotherapeutic-system configurations in accordance with embodiments of the present technology are described in this section with reference to FIGS. 7A-9D. It will be appreciated that specific elements, substructures, advantages, uses, and/or other features of the embodiments described with reference to FIGS. 7A-9D can be suitably interchanged, substituted, or otherwise configured with one another and/or with the embodiments described with reference to FIGS. 1A-6 and 10A-67 in accordance with additional embodiments of the present technology. Furthermore, suitable elements of the embodiments described with reference to FIGS. 7A-9D can be used as stand-alone and/or self-contained devices.

FIG. 7A is a partially schematic diagram illustrating a cryotherapeutic system 1200 that can include a console 1202 and a cryotherapeutic device 1204 operatively connectable such that the console 1202 can control, monitor, supply, or otherwise support operation of the cryotherapeutic device 1204. For example, the console 1202 can include a first umbilical connector 1206, the cryotherapeutic device 1204 can include a second umbilical connector 1208, and the first and second umbilical connectors 1206, 1208 can be removably connectable. In other embodiments, the console 1202 and the cryotherapeutic device 1204 can be permanently connected or removably connectable by a different type of connector. When the first and second umbilical connectors 1206, 1208 are connected, the cryotherapeutic system 1200 can be configured for therapeutically effective cryogenic renal neuromodulation. For example, the cryotherapeutic device 1204 can include an elongated shaft 1210 and a cooling assembly 1212 at a distal end of the shaft 1210. The shaft 1210 can be configured to locate the cooling assembly 1212 intravascularly at a treatment site in or otherwise proximate a renal artery or renal ostium. The cryotherapeutic device 1204 can further include a handle (not shown) at a proximal portion of the shaft 1210 and an umbilical cord 1214 extending between the second umbilical connector 1208 and the handle.

As shown in FIG. 7A, the first umbilical connector 1206 can include a first outlet adapter 1216. The console 1202 can include a cartridge connector 1218 and a supply passage 1220 extending between the first outlet adapter 1216 and the cartridge connector 1218. In some embodiments, the supply passage 1220 can be configured to carry refrigerant in liquid or substantially liquid phase toward the cryotherapeutic device 1204. When the first and second umbilical connectors 1206, 1208 are connected, the supply passage 1220 can be fluidly connected to a supply line or lumen of the cryotherapeutic device 1204. Supply lines and lumens are described, for example, above with reference to FIGS. 1A-1C. For example, the supply passage 1220 can be fluidly connected to the supply line 110 shown in FIG. 1A. Refrigerant in liquid or substantially liquid phase can travel through the supply line or lumen to the cooling assembly 1212 where it can expand to cause cryogenic cooling during a treatment procedure.

The cryotherapeutic system 1200 can include a cartridge 1222 configured to contain pressurized refrigerant suitable for therapeutically effective cryogenic renal neuromodulation (e.g., refrigerant that can reach cryogenic temperatures at or near its normal boiling point). Examples of suitable refrigerants are discussed, for example, with reference to FIG. 1A above. In some embodiments, the refrigerant can be gaseous at standard temperature and pressure and stored in at least a substantially liquid phase within the cartridge 1222. As shown in FIG. 7A, the cartridge 1222 can be connected to the cartridge connector 1218. The supply passage 1220 can be configured to carry refrigerant from the cartridge 1222. Accordingly, the supply passage 1220 can have a relatively high pressure rating (e.g., a burst pressure greater than about 750 psi) along all or a portion of its length.

The cartridge 1222 can be relatively compact. Furthermore, the cartridge 1222 can be portable and/or disposable (e.g., disposable after treatment of a single patient). In some embodiments, however, the cartridge 1222 can be non-disposable and/or refillable. Unlike higher-capacity refrigerant sources, the cartridge 1222 can be small enough to be transportable by common carrier (e.g., via air mail). In some embodiments, the cartridge 1222 can have a capacity less than about 1000% (e.g., less than about 800% or less than about 600%) of an average quantity of refrigerant expended to perform therapeutically effective cryogenic renal neuromodulation on a single patient without complications. Furthermore, the cartridge 1222 can have a capacity such that it generally does not need to be changed during a treatment procedure. The capacity of the cartridge 1222, for example, can be greater than about 200% (e.g., greater than about 300% or greater than about 400%) of the average quantity of refrigerant expended to perform therapeutically effective cryogenic renal neuromodulation on a single patient without complications. The cartridge 1222 can have an internal volume, for example, less than about 1L (e.g., less than about 500 mL or less than about 300 mL). In some embodiments, the cartridge 1222 can have an internal volume between about 5 cc and about 1 L (e.g., between about 5 cc and about 500 cc or between about 10 cc and about 300 cc). The cartridge 1222 can be elongated and can have a length less than about 400 mm (e.g., less than about 300 mm or less than about 200 mm). In other embodiments, the cartridge 1222 can have other suitable capacities and/or dimensions.

The cartridge 1222 can have a variety of suitable shapes. For example, the cartridge 1222 can be elongated or non-elongated and can be generally shaped as a cylinder, a triangular solid, a cuboid, another suitable polygonal solid, or another suitable shape. In some embodiments, the cartridge 1222 can be shaped to rest flat on a surface without rolling when not in use. Furthermore, the cartridge 1222 can be sized or shaped to facilitate dispensing or handling. For example, multiple cartridges 1222 can be included in a container (not shown) (e.g., a disposable or reusable box) including an opening through which the cartridges 1222 can be withdrawn. The container can be configured for placement with the opening at a lower portion of the container such that gravity causes a new cartridge 1222 to be automatically staged near the opening after each cartridge 1222 is removed from the container until the container is empty. When the cartridges 1222 are elongated, the opening can be elongated and the container can be configured for placement with a long axis of the opening and long axes of the cartridges 1222 generally parallel to a support surface on which the container rests. In some embodiments, the cartridges 1222 can include a textured and/or compressible skin (not shown) that can facilitate handling, durability, and/or performance. For example, the skin can facilitate gripping, absorb impact (e.g., if the cartridge 1222 is dropped), and/or thermally insulate the contents of the cartridge 1222. Thermally insulating the contents of the cartridge 1222 with the skin, double-wall construction, or another suitable form of insulation can be useful, for example, to increase the time during which the contents of the cartridge 1222 will remain at an elevated temperature in embodiments in which the cartridge 1222 can be heated prior to or during use.

The cartridge 1222 can have advantages relative to at least some alternative refrigerant sources (e.g., higher-capacity refrigerant sources). For example, supplying refrigerant from the cartridge 1222 can mitigate at least some cost and/or inconvenience associated with certain alternative refrigerant sources. Furthermore, supplying refrigerant from the cartridge 1222 can enable the console 1202 to be relatively portable and compact with few, if any, connections to stationary or cumbersome objects in an operating room. In some embodiments, the cryotherapeutic system 1200 can be generally self-contained for therapeutically effective cryogenic renal neuromodulation when the cartridge 1222 is loaded in the console 1202. In these and other embodiments, it can be possible to move the console 1202 during a treatment procedure without the need to reposition one or more supply lines.

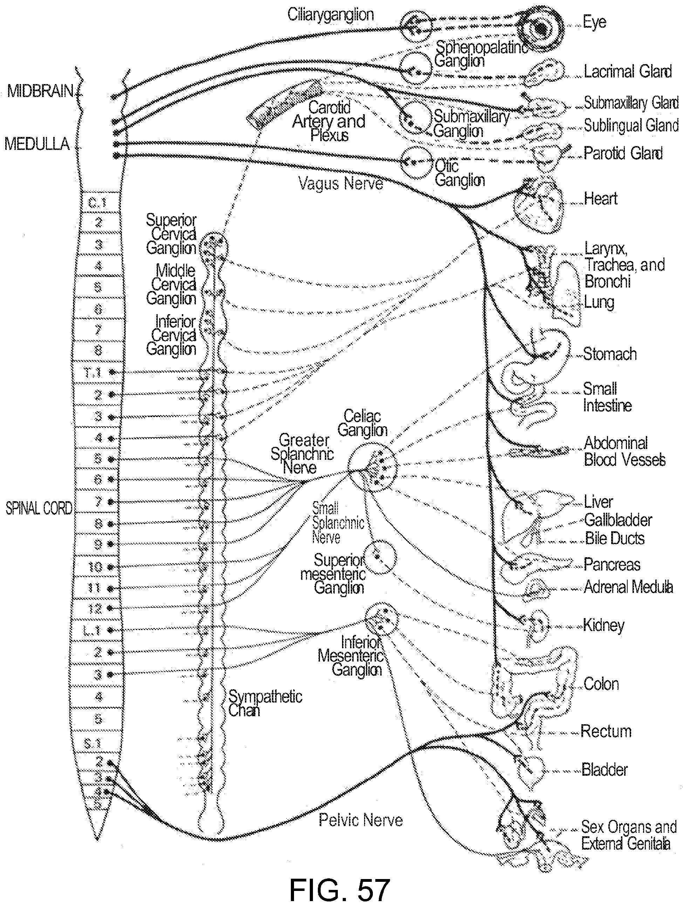

The cartridge 1222 can be produced or maintained to have specifications not readily achievable with at least some alternative refrigerant sources. For example, the cartridge 1222 can be fully or partially sterile (e.g., included in a sterile package prior to being connected to the cartridge connector 1218). Furthermore, refrigerant within the cartridge 1222 and/or the cartridge 1222 itself can have properties or undergo processing specifically related to enhanced suitability for therapeutically effective cryogenic renal neuromodulation. For example, refrigerant within the cartridge 1222 can have a moisture concentration while in the cartridge 1222 of less than about 10 ppm (e.g., less than about 8 ppm or less than about 6 ppm). At the low temperatures typically used in therapeutically effective cryogenic renal neuromodulation (e.g., -60°C and lower), excessive levels of moisture in refrigerant can freeze and obstruct supply or exhaust lines and potentially cause system failures. Moreover, the supply and exhaust lines used in therapeutically effective cryogenic renal neuromodulation can be smaller and more susceptible to obstruction than those used in other types of treatments performed from within the vasculature. Characteristics of the renal vasculature are described, for example, below with reference to FIGS. 57-60B.

Although the cartridge 1222 can have many advantages, in some embodiments, refrigerant can be supplied from a refrigerant source having a greater capacity than the cartridge 1222. For example, refrigerant can be supplied from a canister (e.g., a tank capable of containing more than about 1 L of liquid-phase refrigerant). In these embodiments, the cartridge connector 1218 can be replaced with a connector configured for connection to a canister internal or external to the console 1202. Use of a canister can be advantageous, for example, when the canister is readily available (e.g., in an operating room equipped for certain cardiac cryotherapeutic procedures). In some embodiments, refrigerant within a canister can be more easily maintained with low levels of contamination (e.g., moisture contamination) in comparison to refrigerant within the cartridge 1222. Furthermore, in comparison to lower-capacity refrigerant sources, higher-capacity refrigerant sources can provide more consistent output pressure and/or can reduce the frequency of servicing (e.g., refilling). When the cartridge 1222 is disposable, use of a canister can generate less waste.

As shown in FIG. 7A, the console 1202 can include a cartridge housing 1224 adjacent to the cartridge connector 1218 that can fully contain the cartridge 1222. In other embodiments, the cartridge housing 1224 can partially contain the cartridge 1222 or the cartridge housing 1224 can be eliminated and the cartridge connector 1218 can be at or near an external portion of the console 1202. With reference to FIG. 7A, the cartridge housing 1224 can include a main portion 1226 and a lid 1228. The lid 1228 can be removably connectable to the main portion 1226 and accessible from outside the console 1202 to allow the cartridge 1222 to be replaced as needed. In some embodiments, the console 1202 can include one or more additional cartridge connectors at or near the cartridge housing 1224 or one or more additional cartridge housings. For example, the console 1202 can be modified to include a backup supply system including a backup cartridge, a backup cartridge connector, a backup supply passage extending between the backup cartridge connector and the supply passage 1220, and a backup valve along the backup supply passage. The backup cartridge can be connected to the backup cartridge connector, and opening the backup valve can release refrigerant from the backup cartridge into the supply passage 1220. In this way, the supply of refrigerant can be continued generally without interruption as the cartridge 1222 nears and reaches a depleted state. In other embodiments, the backup supply system can include a connection to a canister. In these and other embodiments, the backup supply system can act as an alternative supply system, and the console 1202 can include a selector switch allowing an operator to select between refrigerant supply from the cartridge 1222 and refrigerant supply from the canister.

A variety of suitable conditioning structures can be included along the supply passage 1220. For example, the console 1202 can include a first particulate filter 1230, a molecular sieve 1232, a second particulate filter 1234, and a sterilizer 1236 fluidly connected to the supply passage 1220. These and other suitable conditioning structures can be useful to condition refrigerant that has not been pre-conditioned for therapeutically effective cryogenic renal neuromodulation and/or to provide redundant processing of refrigerant that has been pre-conditioned for therapeutically effective cryogenic renal neuromodulation. The first and second particulate filters 1230, 1234 can be configured to remove particulates that might obstruct or damage (e.g., corrode) delicate components of the cryotherapeutic device 1204. In some embodiments, the first and second particulate filters 1230, 1234 can include first and second media, respectively, that can be the same or different. For example, the second media can be configured to remove finer particulates than the first media. The second particulate filter 1234 can be positioned to remove material shed from the molecular sieve 1232. Suitable media for the first and second particulate filters 1230, 1234 include, but are not limited to, porous metal media having a media grade from about 0.1 to about 100. The primary composition of the media can be, for example, stainless steel (e.g., 316L, 304L, 310, 347, or 430), HASTELLOY® metal alloy (e.g., C-276, C-222, X, N, B, or B2), INCONEL® alloy (e.g., 600, 625, or 690), nickel (e.g., 200), MONEL® nickel alloy (e.g., 400), titanium, alloy 20, combinations thereof, or other suitable materials. Some examples of suitable media are available from Mott Corporation (Farmington, CT).

The molecular sieve 1232 can be configured to remove refrigerant impurities at the molecular level. For example, as discussed above with reference to the cartridge 1222, excessive levels of moisture in refrigerant can freeze and obstruct small supply and/or exhaust lines, which can cause system failures. The molecular sieve 1232 can be a desiccating filter and can be configured to reduce a moisture concentration in refrigerant within the supply passage 1220 (e.g., to a level less than about 10 ppm, less than about 8 ppm, or less than about 6 ppm). In some embodiments, the molecular sieve 1232 can include one or more aluminosilicate minerals, clay, porous glass, microporous charcoal, zeolite, active carbon, or combinations thereof. In addition to or instead of removing water, the molecular sieve 1232 can be configured to remove other undesirable molecules, such as hydrocarbons having higher molecular weights than the refrigerant. Some examples of suitable molecular sieves are available from Sigma-Aldrich (Saint Louis, MO).

Refrigerant typically does not directly contact the blood stream during therapeutically effective cryogenic renal neuromodulation. It can still be useful, however, for the refrigerant to be sterile. For example, refrigerant can be exhausted into the atmosphere of an operating room after expansion. Microbial contamination of this exhaust stream can be undesirable. The sterilizer 1236 can be configured to partially or fully sterilize refrigerant within the supply passage 1220. As shown in FIG. 7A, the sterilizer 1236 can include a radiation source 1238. The radiation source 1238 can be configured to provide ultraviolet light or another suitable form of sterilizing radiation to refrigerant as it passes through the sterilizer 1236.

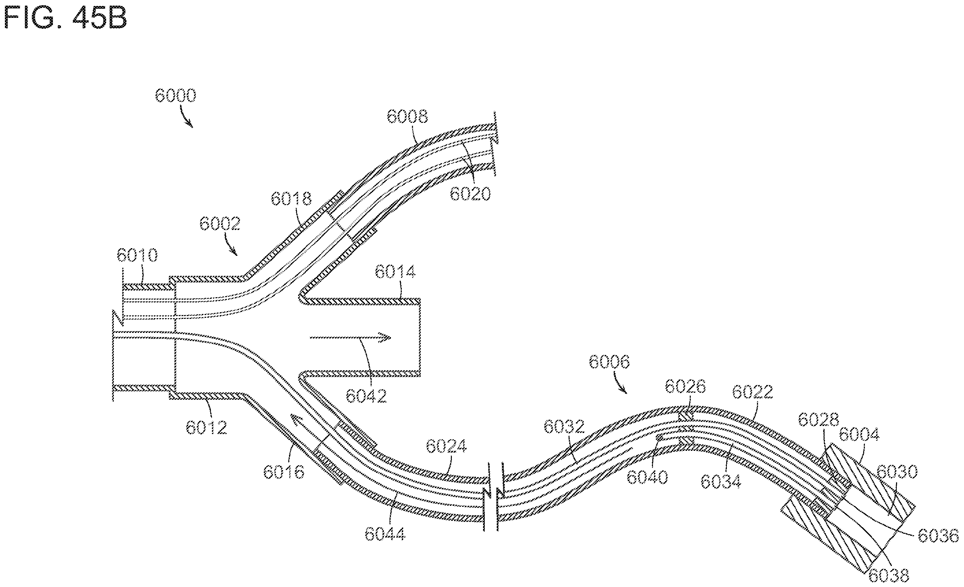

In some embodiments, the console 1202 can be configured to pre-cool refrigerant in the supply passage 1220 and/or to supply refrigerant to one or more pre-coolers in other cryotherapeutic-system components. Pre-cooling can be useful to increase the cooling capacity of refrigerant, as described, for example, below with reference to FIGS. 45A-50. With reference to FIG. 7A, in addition to the supply passage 1220, the console 1202 can include a pre-cooling passage 1240. The console 1202 can further include a first branch connection 1242 fluidly connected to the supply passage 1220. The first umbilical connector 1206 can include a second outlet adapter 1244. The pre-cooling passage 1240 can extend from the first branch connection 1242 to the second outlet adapter 1244. When the first and second umbilical connectors 1206, 1208 are connected, the pre-cooling passage 1240 can be fluidly connected to a pre-cooling supply lumen (not shown) of the cryotherapeutic device 1204. Pre-cooling supply lumens are described, for example, below with reference to FIGS. 45A-50. For example, the pre-cooling passage 1240 can be fluidly connected to the second supply tube 6034 shown in FIG. 45B. Refrigerant in liquid or substantially liquid phase can travel through a pre-cooling supply lumen to a pre-cooling expansion chamber (not shown), where it can expand to cool refrigerant within a primary-supply lumen (not shown).

The console 1202 can include an exhaust passage 1246 and an exhaust assembly 1248 having an exhaust portal 1250. In some embodiments, the exhaust assembly 1248 can be at or near an external portion of the console 1202. The first umbilical connector 1206 can include a first inlet adapter 1252 and the exhaust passage 1246 can extend between the first inlet adapter 1252 and the exhaust portal 1250. As shown in FIG. 7A, the exhaust portal 1250 can be open to the atmosphere and vent near the console 1202. In other embodiments, the exhaust portal 1250 can include a permanent exhaust tube or an exhaust adapter (e.g., a luer) configured to connect to an exhaust tube, to an exhaust containment vessel, or to an inflation/deflation tool (e.g. a syringe). When the first and second umbilical connectors 1206, 1208 are connected, the exhaust passage 1246 can be fluidly connected to an exhaust line or lumen (not shown) of the cryotherapeutic device 1204. Examples of exhaust lines and lumens are described, for example, above with reference to FIGS. 1A-1C. For example, the exhaust passage 1246 can be fluidly connected to the exhaust lines 115 shown in FIG. 1A. Refrigerant in gaseous phase or substantially gaseous phase can travel along the exhaust line or lumen after expansion in the cooling assembly 1212.

The console 1202 can include a pressure-relief passage 1254 and a second branch connection 1256 fluidly connected to the supply passage 1220. The pressure-relief passage 1254 can extend between the second branch connection 1256 and a pressure-relief portal 1258 of the exhaust assembly 1248. Similar to the exhaust portal 1250, the pressure-relief portal 1258 can be open to the atmosphere and vent near the console 1202. In other embodiments, the pressure-relief portal 1258 can include a permanent pressure-relief tube or a pressure-relief adapter configured to connect to a pressure-relief tube or to a pressure-relief containment vessel. A pressure-relief adapter, a pressure-relief tube, and/or a pressure-relief containment vessel can be combined with an exhaust adapter, an exhaust tube, or an exhaust containment vessel, as described, for example, above with reference to the exhaust passage 1246. Furthermore, in some embodiments, the pressure-relief passage 1254 and the exhaust passage 1246 can join within the console 1202.

The console 1202 can include a variety of suitable valves on the supply passage 1220, the pre-cooling passage 1240, and/or the exhaust passage 1246. Some of these valves can be configured to prevent refrigerant from traveling to the cryotherapeutic device 1204 in the event of an error state (e.g., a loss of power). For example, the console 1202 can include a pressure-relief valve 1260 along the pressure-relief passage 1254 and an isolation valve 1262 along the supply passage 1220. When an error state occurs, the pressure-relief valve 1260 can default (e.g., spring) to an open position and the isolation valve 1262 can default (e.g., spring) to a closed position. The closed isolation valve 1262 can prevent additional refrigerant from traveling through the supply passage 1220, and the open pressure-relief valve 1260 can exhaust any refrigerant remaining in the cartridge 1222 and the supply passage 1220 between the cartridge connector 1218 and the isolation valve 1262. The console 1202 can include a first supply valve 1264 and a second supply valve 1266 along the supply passage 1220 and the pre-cooling passage 1240, respectively. The first supply valve 1264 and the second supply valve 1266 can be configured to control (e.g., regulate) refrigerant flow through the supply passage 1220 and the pre-cooling passage 1240, respectively, during normal operation. In some embodiments, the isolation valve 1262 and the first supply valve 1264 can be combined, and the first supply valve 1264 can default (e.g., spring) to a closed position.

The console 1202 can include a user interface 1268 and a control assembly 1270 having a controller 1272, a processor 1274, and a network of electrical lines (shown dashed) configured for communication and/or power supply. The control assembly 1270 can be configured to control operation of various components of the console 1202 according to signals from the user interface 1268 as well as from various sensors of the console 1202. Actuators (e.g., solenoid or another suitable type of actuator) can be operably connected to valves within the console 1202. The actuators can be electric, pilot-operated, or have another modality. As shown in FIG. 7A, the control assembly 1270 can include a first actuator 1276 operably connected to the first supply valve 1264, a second actuator 1278 operably connected to the second supply valve 1266, a third actuator 1280 operably connected to the pressure-relief valve 1260, and a fourth actuator 1282 operably connected to the isolation valve 1262. The first actuator 1276 and/or the second actuator 1278 can be configured, respectively, to cause the first supply valve 1264 and the second supply valve 1266 to provide generally continuous flow rates even as refrigerant within the cartridge 1222 is depleted and pressure within the cartridge 1222 diminishes. For example, in some embodiments, the control assembly 1270 can include a pressure-compensated flow regulator operably connected to the first supply valve 1264 and/or the second supply valve 1266. In embodiments in which the console 1202 includes a backup supply system, the control assembly 1270 can include a backup actuator operably connected to a backup valve.

The user interface 1268 can include an initiation switch 1284 and a termination switch 1286, and the control assembly 1270 can be configured to signal the first actuator 1276 to open or close the first supply valve 1264 in response to signals from the initiation switch 1284 and the termination switch 1286, respectively. In this way, the initiation switch 1284 and the termination switch 1286 can be used to initiate and terminate, respectively, refrigerant flow to the cryotherapeutic device 1204. The initiation switch 1284 and the termination switch 1286 can have a variety of suitable configurations (e.g., button, dial, flip-switch, etc.). The user interface 1268 also can include various suitable displays and indicators (not shown). The user interface 1268 can be integral with the other portions of the console 1202 (e.g., as shown in FIG. 7A) or the user interface 1268 can be included partially or fully on a remote unit (e.g., a remote unit having a wired or wireless connection to other portions of the control assembly 1270). With reference again to FIG. 7A, the control assembly 1270 can include a timer 1288 configured to work in conjunction with the controller 1272 and/or the initiation switch 1284. For example, the initiation switch 1284 can trigger the timer 1288. The controller 1272 can be configured to signal the first actuator 1276 to close the first supply valve 1264 in response to a signal from the timer 1288. A cycle time, such as a cycle time suitable for therapeutically effective cryogenic renal neuromodulation, can be a time between triggering the timer 1288 and a signal from the timer 1288 to close the first supply valve 1264.

The console 1202 can include suitable sensors configured to detect conditions within the console 1202 that can be reported via the user interface 1268 and/or used by the control assembly 1270 to control operation of components of the console 1202. For example, the console 1202 can include a cartridge sensor 1290 configured to detect an indication of a temperature of the cartridge 1222, of the cartridge housing 1224, and/or of refrigerant within the cartridge 1222. Operation of the cryotherapeutic system 1200 can be optimized for refrigerant at a particular temperature or range of temperatures. If the cartridge 1222 is too cold or too hot (e.g., if the cartridge 1222 was recently removed from a cold or hot environment), the cartridge sensor 1290 can register a temperature below or above, respectively, a threshold value or range. This can cause the control assembly 1270 to override the initiation switch 1284 until the cartridge 1222 reaches an acceptable temperature. Furthermore, the console 1202 can include a heater 1292 and a chiller 1294 operably connected to the cartridge housing 1224 to change the temperature of the refrigerant For example, the control assembly 1270 can be configured to activate the heater 1292 or the chiller 1294 if the cartridge sensor 1290 registers a temperature below or above, respectively, a threshold value or range. In some embodiments, heating the cartridge 1222 at least partially mitigates pressure loss associated with refrigerant depletion. This can be especially advantageous when the cartridge 1222 is relatively small.

As shown in FIG. 7A, the console 1202 can include a supply sensor 1296 operably connected to the supply passage 1220 and configured to detect a flow rate and/or pressure of refrigerant within the supply passage 1220. This can be used, for example, to indicate whether the cartridge 1222 is full and properly connected to the cartridge connector 1218. If the supply sensor 1296 detects a pressure below a threshold value, the control assembly 1270 can be configured to override the initiation switch 1284. In this way, it can be possible to reduce the likelihood of incomplete treatments due to insufficient refrigerant within the cartridge 1222 and/or improper connection of the cartridge 1222 to the cartridge connector 1218. In other embodiments, the console 1202 can include a weight sensor (e.g., a scale) in place of or in addition to the supply sensor 1296. The weight sensor can be configured, for example, to weigh the cartridge 1222 and to communicate the weight to the control assembly 1270. The control assembly 1270 can be configured to use the weight (e.g., relative to the weight of the cartridge 1222 when empty) to determine whether the cartridge 1222 contains sufficient refrigerant for a treatment procedure.

The console 1202 can include an exhaust sensor 1298 operably connected to the exhaust passage 1246 and configured to detect a flow rate and/or pressure of refrigerant within the exhaust passage 1246. Data from the exhaust sensor 1298 can be used in conjunction with data from the supply sensor 1296. For example, the control assembly 1270 can be configured to signal the first actuator 1276 to close the first supply valve 1264 if a difference between a first phase-independent flow rate or pressure within the supply passage 1220 and a second phase-independent flow rate or pressure within the exhaust passage 1246 is greater than a threshold value or range. Such a difference can indicate a failure (e.g., a leak or rupture) within a portion of the cryotherapeutic device 1204. As shown in FIG. 7A, the supply sensor 1296 can be between the cartridge connector 1218 and the second branch connection 1256. In other embodiments, the supply sensor 1296 can have a different position along the supply passage 1220 (e.g., downstream relative to the sterilizer 1236). Phase correction of material flow rates through the supply passage 1220 and the exhaust passage 1246 can occur, for example, within the controller 1272 or separately within the supply sensor 1296 and the exhaust sensor 1298.

In some embodiments, the console 1202 can include suitable sensors configured to detect conditions within the cryotherapeutic device 1204 that can be reported via the user interface 1268 and/or used by the control assembly 1270 to control operation of components of the console 1202. For example, as shown in FIG. 7A, the console 1202 can include a pressure-monitoring passage 1201 and a pressure-monitoring chamber 1203. The first umbilical connector 1206 can include a first pressure-monitoring adapter 1205. The pressure-monitoring passage 1201 can extend between the pressure-monitoring chamber 1203 and the first pressure-monitoring adapter 1205. The console 1202 can include a pressure-monitoring sensor 1207 operably connected to the pressure-monitoring chamber 1203. When the first and second umbilical connectors 1206, 1208 are connected, the pressure monitoring passage 1201 can be fluidly connected to a pressure-monitoring line or lumen (not shown) of the cryotherapeutic device 1204. Examples of pressure-monitoring lines and lumens are described, for example, above with reference to FIGS. 1A and 4. For example, the pressure-monitoring passage 1201 can be fluidly connected to the pressure line 132 shown in FIG. 1A. The pressure-monitoring line or lumen can be configured to provide a fluidic connection to a balloon (not shown) configured to be within the vasculature so that a pressure within the balloon can be monitored from outside the vasculature. In some embodiments, the control assembly 1270 can be configured to signal the first actuator 1276 to close the first supply valve 1264 if the pressure within the pressure-monitoring chamber 1203 is greater than a threshold value or range. This can serve, for example, to prevent over-inflation of the balloon during a treatment procedure.

As discussed above, the first umbilical connector 1206 can include the first outlet adapter 1216, the second outlet adapter 1244, the first inlet adapter 1252, and the first pressure-monitoring adapter 1205. The second umbilical connector 1208 can include a second inlet adapter 1209, a third inlet adapter 1211, a third outlet adapter 1213, and a second pressure-monitoring adapter 1215 removably connectable to the first outlet adapter 1216, the second outlet adapter 1244, the first inlet adapter 1252, and the first pressure-monitoring adapter 1205, respectively. The adapters of the first and second umbilical connectors 1206, 1208 can have various suitable configurations (e.g., threaded, compression, barbed, or another suitable configuration). In some embodiments, the first and second umbilical connectors 1206, 1208 can include a different number of adapters and/or different types of adapters. For example, the first inlet adapter 1252 and the third outlet adapter 1213 can be eliminated (e.g., in embodiments in which refrigerant exhaust is vented prior to reaching the console 1202). The second umbilical connector 1208 can be configured to merge passages associated with the adapters of the second umbilical connector 1208 into the umbilical cord 1214. The umbilical cord 1214 can include a plurality of separate, parallel passages extending from the adapters of the second umbilical connector 1208. In some embodiments, the second umbilical connector 1208 and/or the umbilical cord 1214 can be replaced, respectively, with a plurality of independent adapters and/or a plurality of individual cords.

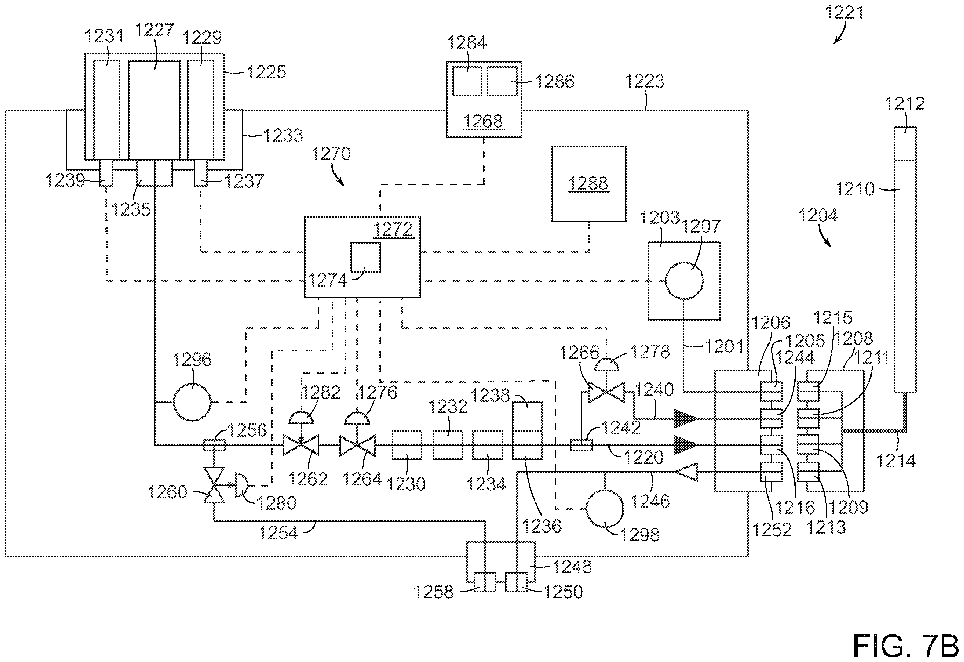

The console 1202 can have a variety of suitable power-supply configurations. As shown in FIG. 7A, the console 1202 can include a power adapter 1217 (e.g., a plug configured to fit into a standard power receptacle or a receptacle of an external power-supply unit) and a power cord 1219 electrically connected to the control assembly 1270. In other embodiments, the console 1202 can be configured to receive power from a battery, such as a rechargeable battery within a pack removably connectable to the console 1202. Such a pack can also include the cartridge 1222 and, in some embodiments, more than one cartridge. For example, FIG. 7B is a partially schematic diagram illustrating a cryotherapeutic system 1221 that can include a console 1223 and a pack 1225 having a cartridge 1227, a battery 1229, and a memory device 1231. The console 1223 can include a pack housing 1233 in which the pack 1225 can be at least partially contained. In other embodiments, the pack 1225 can be fully contained within the pack housing 1233. The console 1223 can include a cartridge connector 1235, a battery connector 1237, and a memory connector 1239 at or near the pack housing 1233 and configured to removably connect to the cartridge 1227, the battery 1229, and the memory device 1231, respectively, when the pack 1225 is within the pack housing 1233.

The pack 1225 can be configured to provide sufficient refrigerant and electrical power to perform a cryogenic renal neuromodulation on a single patient or on multiple patients. After the cartridge 1227 or the battery 1229 is exhausted, the pack 1225 can be recyclable or disposable. For example, the pack 1225 can be provided with instructions and/or packaging that facilitates a return shipment of the pack 1225 to a supplier for recharging and reuse. In some embodiments, the pack 1225 can be included in packaging (e.g., a bag) that preserves the pack 1225 in a sterile condition prior to use and can be used for shipment of the pack 1225 to a supplier or another third party after use. The cartridge 1227 can have any suitable feature described, for example, above with reference to the cartridge 1222 shown in FIG. 7A. The battery 1229 can be, for example, a rechargeable battery having sufficient capacity to power the control assembly 1270 during a treatment procedure. The memory device 1231 can store information to facilitate interaction between the pack 1225 and the control assembly 1270. For example, the memory device 1231 can store a recharge date for the pack 1225 and/or information about whether the pack 1225 has been previously used and/or a level of previous use. The control assembly 1270 can be configured to override the initiation switch 1284 according to information from the memory device 1231 (e.g., if the memory device 1231 indicates that the pack 1225 is expired due to previous use or an elapsed time after the recharge date). The control assembly 1270 also can be configured to assign data to the memory device 1231 (e.g., to cause the memory device 1231 to designate the pack 1225 as used until the pack 1225 is recharged and the memory device 1231 is reset).

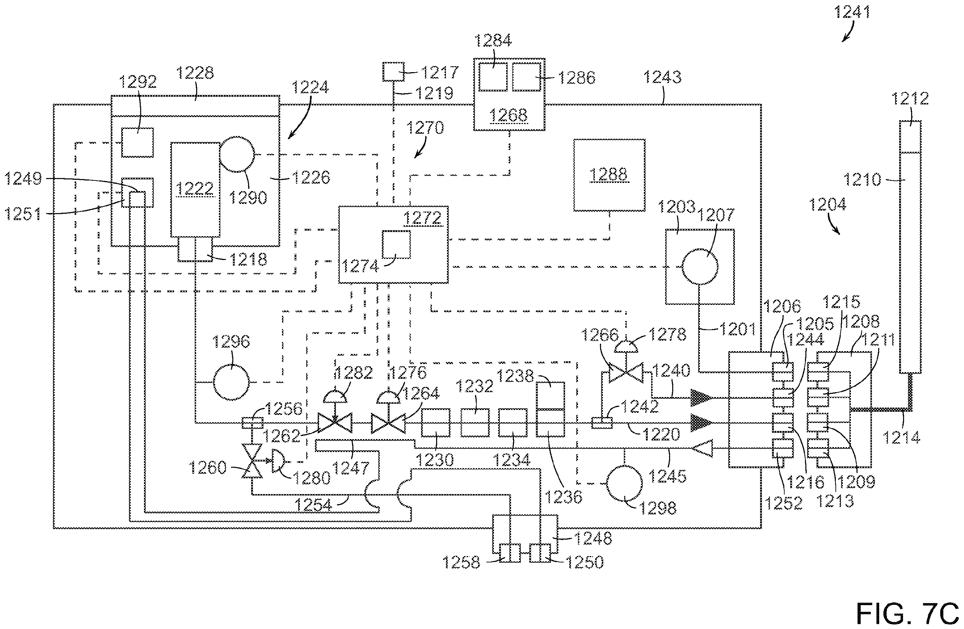

As shown in FIG. 7A, the exhaust passage 1246 can have a relatively simple routing within the console 1202. Alternatively, the exhaust passage 1246 can have a more complex routing such that a portion of the exhaust passage 1246 is proximate components of the console 1202 that can benefit from cooling during operation. Gaseous refrigerant within the exhaust passage 1246 can have some capacity for cooling after exiting the cryotherapeutic device 1204. For example, FIG. 7C is a partially schematic diagram illustrating a cryotherapeutic system 1241 that can include a console 1243 having an exhaust passage 1245 with a first heat-exchange portion 1247 and a second heat-exchange portion 1249. The first heat-exchange portion 1247 can be proximate the isolation valve 1262, the first supply valve 1264, the first actuator 1276, and the fourth actuator 1282. In some embodiments, the first actuator 1276 and the fourth actuator 1282 can release heat during operation. Refrigerant exhaust passing through the first heat-exchange portion 1247 can reduce an operating temperature of the first actuator 1276 and the fourth actuator 1282. The console 1243 can include a chiller 1251 proximate the cartridge 1222 and the second heat-exchange portion 1249 can be within the chiller 1251. Refrigerant exhaust passing through the second heat-exchange portion 1249 can cool the cartridge 1222.

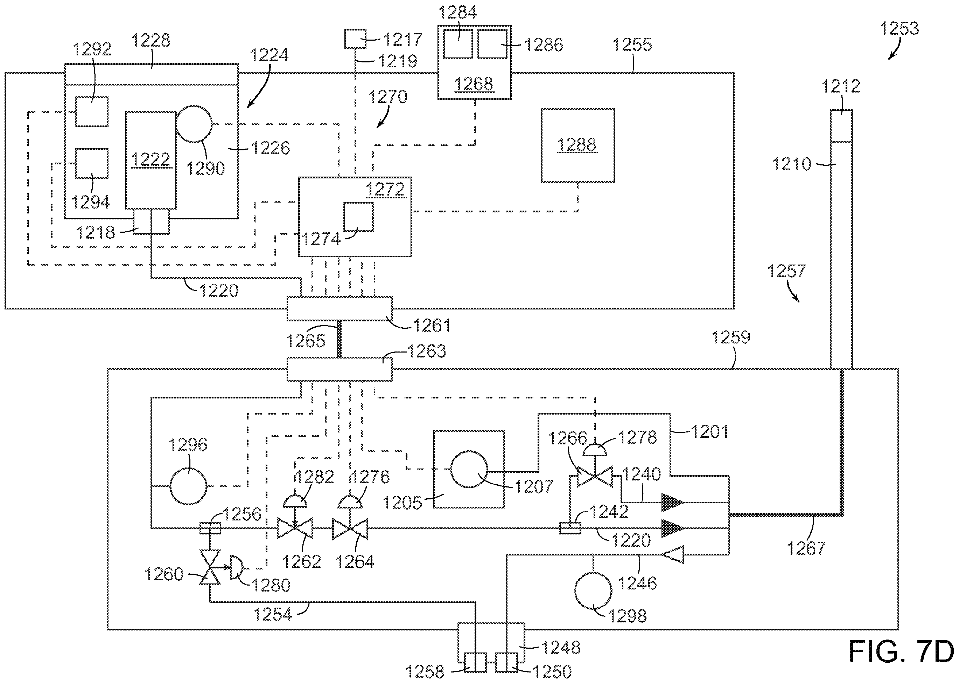

In some embodiment, elements of the consoles 1202, 1223, 1243 shown in FIGS. 7A-7C can be distributed among multiple cryotherapeutic-system components. For example, FIG. 7D is a partially schematic diagram illustrating a cryotherapeutic system 1253 that can include a console 1255 and a cryotherapeutic device 1257 having a handle 1259 at a proximal portion of the shaft 1210. The console 1255 and the handle 1259 can include a first hub 1261 and a second hub 1263, and the cryotherapeutic system 1253 can include a flexible connector 1265 extending between the first hub 1261 and the second hub 1263. The first hub 1261, the second hub 1263, and the connector 1265 can be removable from the console 1255 and/or the handle 1259. For example, the first hub 1261 can be removably connectable to a receptacle (not shown) of the console 1255 and/or the second hub 1263 can be removably connectable to a receptacle (not shown) of the handle 1259. In other embodiments, the first hub 1261, the second hub 1263, and the connector 1265 can be permanently connected between the console 1255 and the handle 1259. The connector 1265 can include a portion of the supply passage 1220 and portions of electrical lines (not separately identified) of the control assembly 1270. Furthermore, the connector 1265 can have a sufficient length to allow the console 1255 to be outside the sterile field during a treatment procedure. In some embodiments, the connector 1265 can have a length from about 0.5 meter to about 5 meters (e.g., from about 1 meter to about 4 meters, or another suitable length).

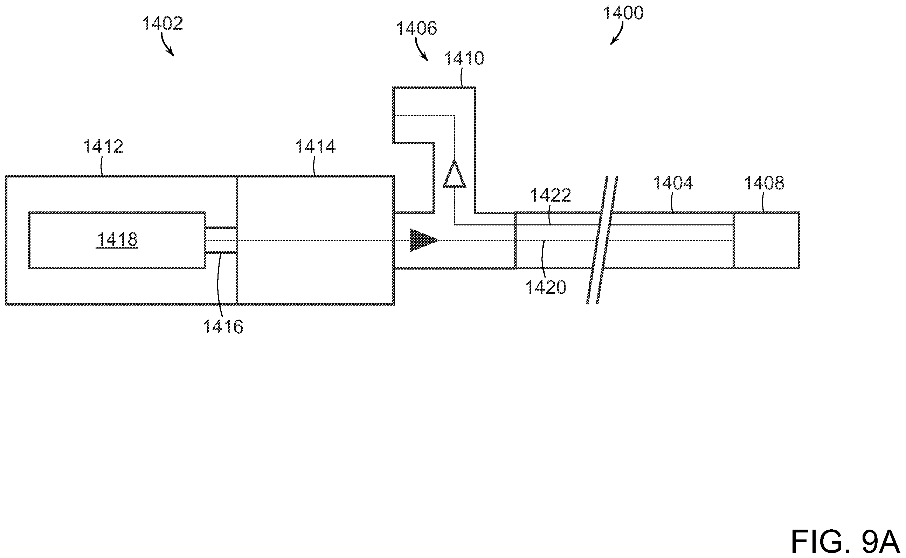

The console 1255 can include the cartridge connector 1218 and a portion of the supply passage 1220 extending between the cartridge connector 1218 and the first hub 1261. In some embodiments, the console 1255 can be configured to be generally stationary during a treatment procedure and the handle 1259 can be configured to move with the proximal portion of the shaft 1210. The cartridge 1222 can have a relatively significant size and/or weight when loaded with refrigerant. Accordingly, locating the cartridge connector 1218 within the console 1255 can be useful to avoid restricting mobility of the handle 1259. Furthermore, particularly when the console 1255 is configured to be outside the sterile field during a treatment procedure, it can be useful to replace the cartridge connector 1218 with a connector configured for connection to a canister that is internal or external to the console 1255. Canisters are described, for example, above with reference to FIG. 7A. In some embodiments, the console 1255 can be a relatively large unit (e.g., a wheeled cart). In these and other embodiments, the cartridge 1222 can be replaced with a canister, and the cartridge housing 1224 can be sized to receive the canister. In still other embodiments, it can be desirable to include the cartridge 1222 and associated components in the handle 1259. This is discussed, for example, below with reference to FIG. 9A.

The handle 1259 can include a variety of suitable valves that fluidly connect to lines extending into the shaft 1210. In some embodiments, the handle 1259 can include generally all valves within the cryotherapeutic system 1253. For example, the handle 1259 can include the pressure-relief valve 1260, the isolation valve 1262, the first supply valve 1264, and the second supply valve 1266. Furthermore, portions of the control assembly 1270 can be within the handle 1259. For example, the first, second, third, and fourth actuators 1276, 1278, 1280, 1282 can be within the handle 1259. In other embodiments, the handle 1259 can include only some of the valves and/or actuators shown in FIG. 7D, and the other valves and actuators can be (a) included in the console 1255, (b) included in another cryotherapeutic-system component, or (c) absent from the cryotherapeutic system 1253. In some embodiments, some or all of the actuators within the handle 1259 can be pneumatic or hydraulic. The actuators can also be electric or have another suitable modality. As shown in FIG. 7D, the handle 1259 can include the exhaust assembly 1248. In other embodiments, the exhaust assembly 1248 can be positioned within the console 1255 such that the exhaust passage 1246 passes through the connector 1265. In these and other embodiments, the second branch connection 1256, the pressure-relief valve 1260, the pressure-relief passage 1254, and the third actuator 1280 of the control assembly 1270 can be within the console 1255. In some embodiments, the exhaust assembly 1248 can be divided with the exhaust portal 1250 being within the handle 1259 and the pressure-relief portal 1258 being within the console 1255.

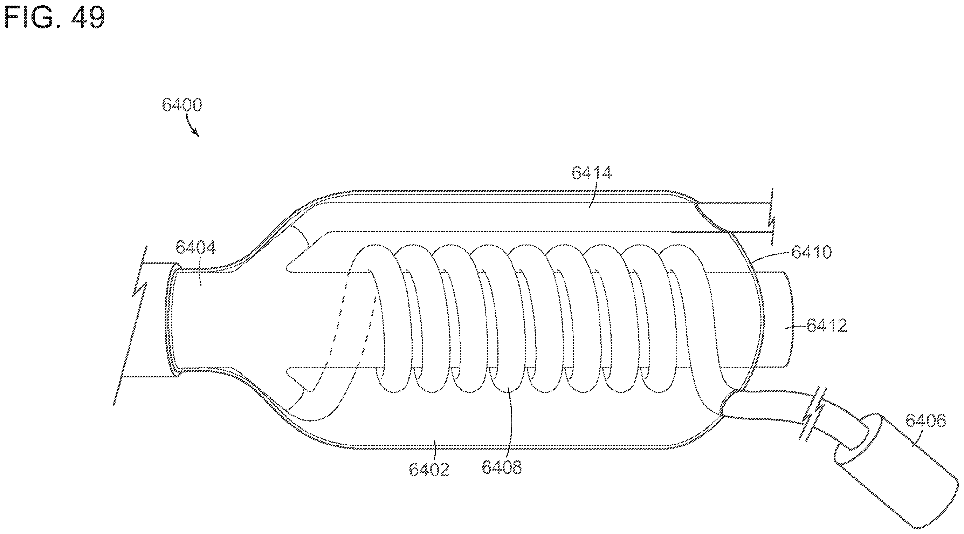

As shown in FIG. 7D, the first branch connection 1242, the pre-cooling passage 1240, the second supply valve 1266, and the second actuator 1278 of the control assembly 1270 can be within the handle 1259. This can reduce the distance that pre-cooled refrigerant travels before reaching the cooling assembly 1212 and, correspondingly, reduce potential warming of the pre-cooled refrigerant. Pre-cooling within a cryotherapeutic-system handle is described, for example, below with reference to FIGS. 49-50. Locating the first supply valve 1264 or both the first supply valve 1264 and the isolation valve 1262 within the handle 1259 can also be advantageous (e.g., by reducing the time delay between a termination signal and cessation of refrigerant flow to the cooling assembly 1212). For example, when the first supply valve 1264 is within the handle 1259, the length of the supply passage 1220 between the first supply valve 1264 and the cooling assembly 1212 can be shorter than it would be if the first supply valve 1264 was in the console 1255. This reduces the amount of refrigerant remaining upstream of the first supply valve 1264 compared to locating the first supply valve 1264 in the console 1255 which, in the event of an error state (e.g., a leak or rupture within a portion of the cryotherapeutic device 1257) during a treatment procedure, can reduce the amount of refrigerant flowing to the cooling assembly 1212 after the error state is detected.

Some or all of the sensors of the control assembly 1270 can be within the handle 1259. For example, the supply sensor 1296, the exhaust sensor 1298, and the pressure-monitoring sensor 1207 can be positioned, respectively, along portions of the supply passage 1220, the exhaust passage 1246, and the pressure-monitoring passage 1201 within the handle 1259. The sensors within the handle 1259 can communicate with other portions of the control assembly 1270 through the network of electrical lines and/or wirelessly. Locating the pressure-monitoring sensor 1207 and the pressure-monitoring passage 1201 within the handle 1259 can reduce the time it takes for a pressure signal traveling along the pressure-monitoring passage 1201 to reach the pressure-monitoring sensor 1207. Pressure signals move more slowly than electrical signals and the length of the pressure-monitoring passage 1201 can be shorter than in embodiments in which the pressure-monitoring sensor 1207 is within the console 1255. In some embodiments, the handle 1259 can include all or a portion of the user interface 1268. For example, the handle 1259 can include the initiation switch 1284 and/or the termination switch 1286 to allow a single operator to control refrigerant flow while also controlling movement of the shaft 1210.

The handle 1259 can be directly connected to the shaft 1210. As shown in FIG. 7D, the pressure-monitoring passage 1201, the pre-cooling passage 1240, the supply passage 1220, and the exhaust passage 1246 can be arranged in an internal conduit assembly 1267 of the handle 1259 that passes directly into the proximal portion of the shaft 1210. In other embodiments, the conduit assembly 1267 can be exposed between the handle 1259 and the shaft 1210. Furthermore, the cryotherapeutic system 1253 can include additional components between the handle 1259 and the shaft 1210 and/or between the console 1255 and the handle 1259. In some embodiments, the handle 1259 can be configured for use with multiple patients and the shaft 1210 can be disposable. In these and other embodiments, the handle 1259 can include a coupling adapter (not shown) at the proximal portion of the shaft 1210. The handle 1259 can have a shape that facilitates gripping. In other embodiments, the handle 1259 can be replaced with a hub or another structure that is not configured for gripping.

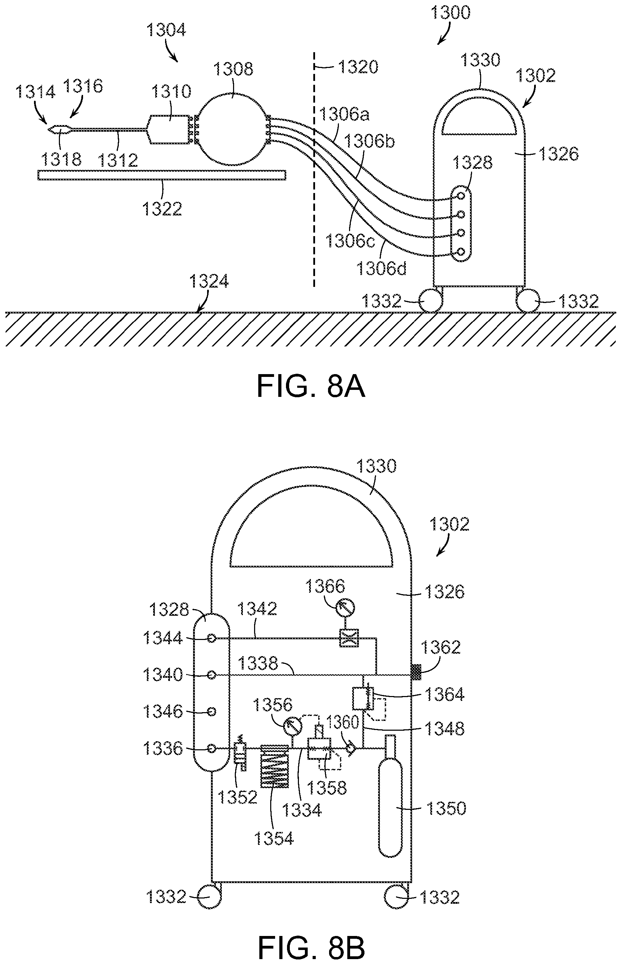

FIGS. 8A-8E are partially schematic diagrams illustrating a cryotherapeutic system 1300. As shown in FIG. 8A, the cryotherapeutic system 1300 can include a console 1302, a cryotherapeutic device 1304, and a plurality of fluidic and/or electrical lines 1306 (individually identified as 1306a-d) extending between the console 1302 and the cryotherapeutic device 1304. The plurality of lines 1306 can include various suitable supply, exhaust, and communication lines (e.g., a refrigerant supply line, a refrigerant exhaust line, an electrical line, and a pressure-monitoring line). The cryotherapeutic device 1304 can include a satellite 1308 connectable to the plurality of lines 1306, a hub 1310 connectable to the satellite 1308, and an elongated shaft 1312 extending from the hub 1310. The cryotherapeutic device 1304 can further include a cooling assembly 1314 at a distal portion 1316 of the shaft 1312. The cooling assembly 1314 can include a balloon 1318. In some embodiments, a length of the shaft can be from about 80 cm to about 85 cm or another suitable length. The hub 1310 can be configured to facilitate manipulation of the shaft 1312 and can be ergonomically shaped for gripping. The satellite 1308 can contain valves, sensors, and other suitable elements of the cryotherapeutic system 1300 that benefit from close proximity to the shaft 1312 (e.g., to reduce response times). Examples of these elements are described, for example, above with reference to FIG. 7D. As shown in FIG. 8A, the hub 1310 and the satellite 1308 can be directly connectable in an interlocking relationship. In other embodiments, a flexible connector can be included between the hub 1310 and the satellite 1308.

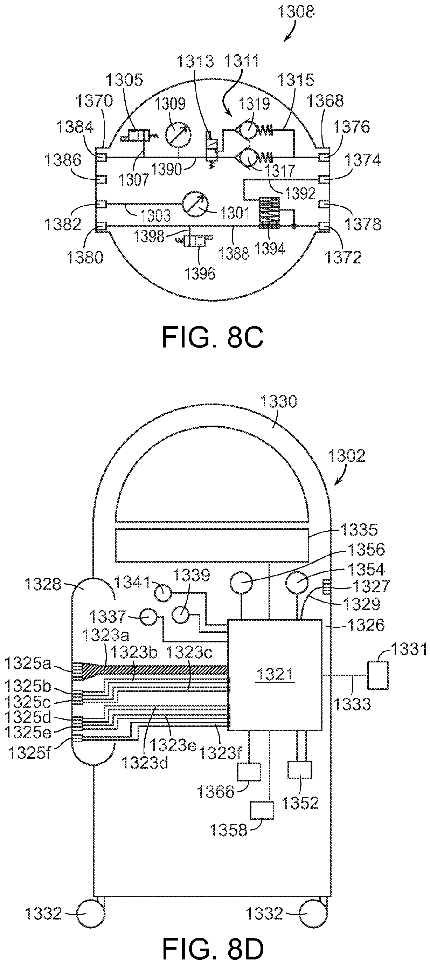

FIG. 8A shows a boundary 1320 between sterile and non-sterile fields during a treatment procedure. The cryotherapeutic device 1304 can rest or otherwise be in the proximity of a bed 1322 and a patient (not shown) within the sterile field, and the console 1302 can be self-supported on the floor 1324 outside the sterile field. The console 1302 can include a main portion 1326 having a connection panel 1328 configured for connection to the plurality of lines 1306. The console 1302 can also include a handle 1330 and a plurality of wheels 1332. The plurality of lines 1306 can have a variety of suitable lengths (e.g., between about 5 feet and about 6 feet). In some embodiments, the plurality of lines 1306 can be collected into an umbilical cord (not shown). Several embodiments of user interface elements of the console 1302 and the satellite 1308 are not shown in FIG. 8A for simplicity of illustration, but are described, for example, below with reference to FIGS. 8D-8E. Furthermore, several embodiments of connections between the console 1302, the satellite 1308, and the hub 1310 are not identified in FIG. 8A, but are described, for example, below with reference to FIGS. 8B-8E.

FIG. 8B is a partially schematic diagram illustrating selected fluidic elements of the console 1302 shown in FIG. 8A. As shown in FIG. 8B, the console 1302 can include a supply passage 1334 extending from a first fluidic adapter 1336 of the connection panel 1328, a first exhaust passage 1338 extending from a second fluidic adapter 1340 of the connection panel 1328, and a second exhaust passage 1342 extending from a third fluidic adapter 1344 of the connection panel 1328. The connection panel 1328 can also include an electrical adapter 1346 described, for example, below with reference to FIG. 8D. As shown in FIG. 8B, the console 1302 can include a pressure-relief passage 1348 extending between the first exhaust passage 1338 and the supply passage 1334. The console 1302 can further include a tank 1350 connectable to the supply passage 1334 and configured to hold sufficient refrigerant to treat multiple patients. In some embodiments, the console 1302 can include a backup supply system (not shown) including a backup tank and a backup valve configured to switch fluidic connection manually or automatically between the tank 1350 and the backup tank as needed. Along the supply passage 1334, between the first fluidic adapter 1336 and the tank 1350, the console 1302 can include an isolation valve 1352, a pre-cooler 1354, a pressure sensor 1356, a supply valve 1358, and a check valve 1360. The isolation valve 1352 can be configured to mechanically default (e.g., spring) to a closed position and electrically actuate to an open position. The pre-cooler 1354 can be configured to perform a closed-loop refrigeration cycle. In some embodiments, the supply valve 1358 can be an electropneumatic regulator and can be operatively connected to the pressure sensor 1356. For example, the supply valve 1358 can be a pressure-compensated flow regulator configured to generally maintain the pressure of refrigerant downstream from the supply valve 1358 within a range of pressures as the pressure of refrigerant in the tank 1350 decreases during use.

The console 1302 can include an exhaust portal 1362, and the first exhaust passage 1338 can extend between the second fluidic adapter 1340 and the exhaust portal 1362. The exhaust portal 1362 can be threaded or otherwise configured for connection to an exhaust conduit (not shown) (e.g., an exhaust conduit leading to a vacuum source (not shown)). As shown in FIG. 8B, the pressure-relief passage 1348 can connect to the supply passage 1334 between the check valve 1360 and the tank 1350. The console 1302 can include a pressure-relief valve 1364 along the pressure-relief passage 1348. In some embodiments, the pressure-relief valve 1364 can be configured for mechanical operation (e.g., without electrical actuation). For example, when pressure within the supply passage 1334 exceeds a threshold value or range, the pressure-relief valve 1364 can be configured to move from a fully closed position to a fully opened position automatically. This can allow excess pressure within the supply passage 1334 to vent through the exhaust portal 1362 via the pressure-relief passage 1348 and the first exhaust passage 1338. When pressure within the supply passage 1334 drops below the threshold value or range, the pressure-relief valve 1364 can be configured to return to the fully closed position automatically. The second exhaust passage 1342 can also be fluidly connected to the first exhaust passage 1338. In some embodiments, the console 1302 can include an exhaust sensor 1366 (e.g., a flow meter) along the second exhaust passage 1342.

FIG. 8C is a partially schematic diagram illustrating selected fluidic elements of the satellite 1308 shown in FIG. 8A. As shown in FIG. 8C, the satellite 1308 can include a first connector portion 1368 and a second connector portion 1370. The first connector portion 1368 can include a first fluidic adapter 1372, a second fluidic adapter 1374, a third fluidic adapter 1376, and a first electrical adapter 1378. With reference to FIG. 8A, the lines 1306 can be configured to connect, respectively, the first, second, and third fluidic adapters 1372, 1374, 1376 and the first electrical adapter 1378 of the first connector portion 1368 of the satellite 1308 to the first, second, and third fluidic adapters 1336, 1340, 1344 and the electrical adapter 1346 of the connection panel 1328 of the console 1302. With reference again to FIG. 8C, the second connector portion 1370 can include a fourth fluidic adapter 1380, a fifth fluidic adapter 1382, a sixth fluidic adapter 1384, and a second electrical adapter 1386. As shown in FIG. 8C, the first and second connector portions 1368, 1370 can be at opposite ends of the satellite 1308. In other embodiments, the first connector portion 1368 can be at one end of the satellite 1308 and the second connector portion 1370 can be radially spaced apart from the second connector portion 1370 by an angle between about 60° and about 160°, such as between about 80° and about 140° or between about 90° and about 120°. The angle between the first and second connector portions 1368, 1370 can facilitate manipulation of the hub 1310 (FIG. 8A) or another portion of the cryotherapeutic device 1304 (FIG. 8A) with reduced interference from the lines 1306 (FIG. 8A).

The satellite 1308 can include a supply passage 1388 extending between the first fluidic adapter 1372 and the fourth fluidic adapter 1380, an exhaust passage 1390 extending between the third fluidic adapter 1376 and the sixth fluidic adapter 1384, and a pre-cooling passage 1392 extending between the second fluidic adapter 1374 and the supply passage 1388. The fourth, fifth, and sixth fluidic adapters 1380, 1382, 1384 and the second electrical adapter 1386 can be configured for connection to corresponding adapters (not shown) of the hub 1310 (FIG. 8A). In some embodiments, the satellite 1308 can include a pre-cooler 1394 along the supply passage 1388. The pre-cooler 1394 can be configured to perform an open-loop refrigeration cycle including expansion of refrigerant in the pre-cooling passage 1392. Accordingly, the pre-cooler 1394 of the satellite 1308 can be more compact than the pre-cooler 1354 of the console 1302 (FIG. 8B). The pre-cooler 1394 can be configured to provide supplemental pre-cooling, such as to partially or fully compensate for refrigerant warming that can occur as the refrigerant travels from the console 1302 to the satellite 1308. The satellite 1308 can further include a first pressure-relief valve 1396 and a pressure-relief passage 1398 extending between the supply passage 1388 and the first pressure-relief valve 1396. In some embodiments, the first pressure-relief valve 1396 can be configured to mechanically default (e.g., spring) to an open position and to electrically actuate to a closed position. The satellite 1308 can include an exhaust port (not shown) connected to the first pressure-relief valve 1396.

As shown in FIG. 8C, the satellite 1308 can include a first pressure-monitoring sensor 1301 and a pressure-monitoring passage 1303 extending between the fifth fluidic adapter 1382 and the first pressure-monitoring sensor 1301. The supply passage 1388, the pressure-monitoring passage 1303, and the exhaust passage 1390 can be configured for connection to a supply lumen (not shown), a pressure-monitoring lumen (not shown), and an exhaust lumen (not shown) of the shaft 1312 (FIG. 8A) via the hub 1310 (FIG. 8A). The satellite 1308 can further include an exhaust valve 1305 and an exhaust branch 1307 extending between the exhaust passage 1390 and the exhaust valve 1305. In some embodiments, the exhaust valve 1305 can be configured to mechanically default (e.g., spring) to an open position and to electrically actuate to a closed position. The satellite 1308 can include an exhaust port (not shown) connected to the exhaust valve 1305, which can be the same as or different than an exhaust port connected to the first pressure-relief valve 1396. In some embodiments, the exhaust port can be a syringe port configured for manual inflation and/or deflation of the balloon 1318 (FIG. 8A) via an exhaust lumen (not shown) of the shaft 1312 (FIG. 8A).

Along the exhaust passage 1390, the satellite 1308 can further include a second pressure-monitoring sensor 1309 and a pressure-relief valve assembly 1311. The second pressure-monitoring sensor 1309 can be configured to measure the pressure within the exhaust passage 1390, which can correspond to a back pressure within the cryotherapeutic device 1304 (FIG. 8A). The pressure-relief valve assembly 1311 can include a switch valve 1313, a bypass passage 1315, a second pressure-relief valve 1317, and a third pressure-relief valve 1319. The second pressure-relief valve 1317 can be along the exhaust passage 1390 and the third pressure-relief valve 1319 can be along the bypass passage 1315. The switch valve 1313 can be configured to switch exhaust flow along the exhaust passage 1390 to the bypass passage 1315 when the switch valve 1313 is actuated. Depending on the position of the switch valve 1313, the second and third pressure-relief valves 1317, 1319 can be selectively deployed. The second and third pressure-relief valves 1317, 1319 can have different pressure ratings. Accordingly, by selectively deploying the second and third pressure-relief valves 1317, 1319, the switch valve 1313 can change the distal pressure within the exhaust passage 1390.

FIG. 8D is a partially schematic diagram illustrating selected electrical elements of the console 1302 shown in FIG. 8A. Some elements of the console 1302 are illustrated differently in FIG. 8D than in FIG. 8B (e.g., as blocks rather than as instrumentation symbols). As shown in FIG. 8D, the console 1302 can include a processor 1321 electrically connected to the isolation valve 1352, the pre-cooler 1354, the pressure sensor 1356, the supply valve 1358, and the exhaust sensor 1366. The console 1302 can further include a plurality of electrical lines 1323 (individually identified as 1323a-f) extending between the processor 1321 and a plurality of electrical connectors 1325 (individually identified as 1325a-f) of the connection panel 1328. The electrical line 1323a is illustrated as a bus. In some embodiments, one or more of the electrical lines 1323b-f can similarly include multiple, parallel conductors. The electrical connectors 1325 can collectively correspond to the electrical adapter 1346 shown in FIG. 8B. The console 1302 can further include a data port 1327 (e.g., a universal serial bus port) and an output data line 1329 extending between the data port 1327 and the processor 1321. In some embodiments, the data port 1327 can be used to download historical and/or real-time data from the console 1302 (e.g., for display, recordkeeping, or analysis). The data port 1327 can also be replaced with a wireless transmitter (e.g., a transmitter configured to generate a Wi-Fi signal). As shown in FIG. 8D, the console 1302 can further include a power adapter 1331 and a power cord 1333 extending between the processor 1321 and the power adapter 1331. In some embodiments, a length of the power cord 1333 can be between about 8 feet and about 12 feet or another suitable length.

The console 1302 can further include a user interface (not separately identified) having a display 1335, an initiation button 1337, a termination button 1339, and a test button 1341, each connected to the processor 1321. The console 1302 can be configured such that the initiation button 1337 and the termination button 1339, respectively, start and stop refrigerant flow to the cryotherapeutic device 1304 (FIG. 8A) (e.g., by opening and closing the isolation valve 1352). The console 1302 can be further configured such that the test button 1341 initiates a test sequence including starting refrigerant flow to the cryotherapeutic device 1304 (FIG. 8A), measuring pressure and/or flow rate data, stopping the refrigerant flow, and reporting the status of the cryotherapeutic system 1300 (FIG. 8A) via the display 1335. In some embodiments, the processor 1321, the electrical lines 1323, the electrical connectors 1325, the data port 1327, and the output data line 1329 can be part of a control assembly (not separately identified) of the cryotherapeutic system 1300 (FIG. 8A).

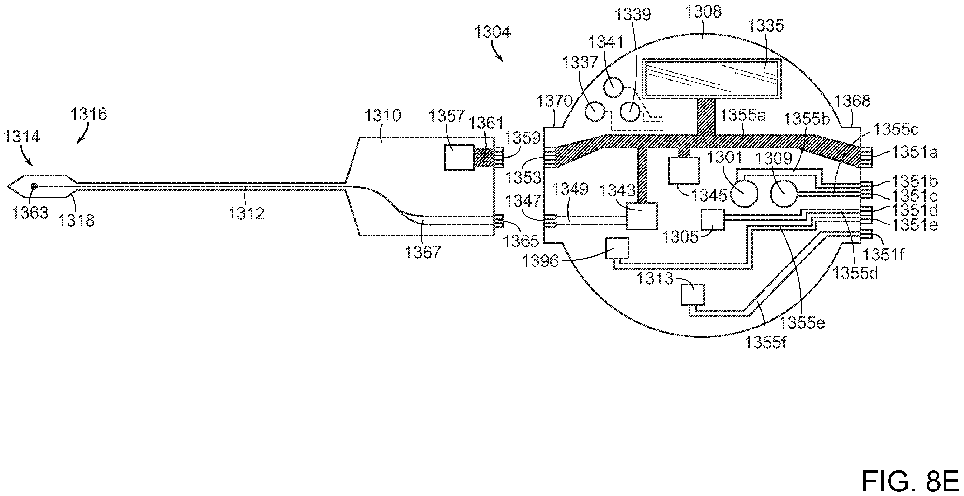

FIG. 8E is a partially schematic diagram illustrating selected electrical elements of the cryotherapeutic device 1304 shown in FIG. 8A. Some elements of the satellite 1308 are illustrated differently in FIG. 8E than in FIG. 8C (e.g., as blocks rather than as instrumentation symbols). Similar to the console 1302 (FIG. 8A), the satellite 1308 can include a user interface (not separately identified) including a display 1335, an initiation button 1337, a termination button 1339, and a test button 1341. The user interface of the satellite 1308 can be redundant to the user interface of the console 1302. Including user interfaces at the satellite 1308 and the console 1302 can facilitate control and/or monitoring of the cryotherapeutic system 1300 (FIG. 8A) from different locations. Other embodiments can include a user interface in the console 1302 only, in the satellite 1308 only, or in neither.

As shown in FIG. 8E, the satellite 1308 can include a first analog-to-digital converter 1343 and a second analog-to-digital converter 1345. One or both of the first and second analog-to-digital converters 1343, 1345 can be configured to process thermocouple data. For example, the second connector portion 1370 can include a first thermocouple connector 1347 and the satellite 1308 can include a first thermocouple lead 1349 extending between the first thermocouple connector 1347 and the first analog-to-digital converter 1343. The first connector portion 1368 of the satellite 1308 can include a plurality of first electrical connectors 1351 (individually identified as 1351a-f) and the second connector portion 1370 can include a second electrical connector 1353. The satellite 1308 can further include a plurality of electrical lines 1355 (individually identified as 1355a-f). The electrical line 1355a is illustrated as a bus. In some embodiments, one or more of the electrical lines 1355b-f can similarly include multiple, parallel conductors. The first electrical connectors 1351 can collectively correspond to the first electrical adapter 1378 shown in FIG. 8C. Similarly, the second electrical connector 1353 and the first thermocouple connector 1347 can collectively correspond to the second electrical adapter 1386 shown in FIG. 8C.

In addition to the satellite 1308, FIG. 8E shows the hub 1310 and the shaft 1312 of the cryotherapeutic device 1304. In some embodiments, the hub 1310 and the shaft 1312 can be disposable and the satellite 1308 can be reusable. The hub 1310 can include a memory device 1357, a third electrical connector 1359, and an electrical line 1361 (shown as a bus) extending between the memory device 1357 and the third electrical connector 1359. The memory device 1357 can be similar to the memory device 1231 of FIG. 7B. For example, the memory device 1231 can be configured to store expiration, usage, and/or other information related to portions of the cryotherapeutic device 1304. In some embodiments, the memory device 1357 can be an FRAM device. As shown in FIG. 8E, the cryotherapeutic device 1304 can include a thermocouple 1363 at the distal portion 1316 if the shaft 1312. The hub 1310 can further include a second thermocouple connector 1365 and the cryotherapeutic device 1304 can include a second thermocouple lead 1367 extending between the thermocouple 1363 and the second thermocouple connector 1365. The third electrical connector 1359 and the second thermocouple connector 1365 of the hub 1310 can be configured, respectively, to connect to the second electrical connector 1353 and the first thermocouple connector 1347 of the second connector portion 1370 of the satellite 1308.

FIGS. 9A-9D are partially schematic diagrams illustrating cryotherapeutic systems that can have relatively compact sizes and/or relatively few (if any) connections to external equipment and/or material sources. FIG. 9A illustrates a cryotherapeutic system 1400 that can include a handle 1402, an elongated shaft 1404, and a coupling member 1406 between the handle 1402 and the shaft 1404. In some embodiments, the shaft 1404 can be permanently attached to the coupling member 1406. In other embodiments, the shaft 1404 and the coupling member 1406 can be separable and can include corresponding coupling adapters. The cryotherapeutic system 1400 can further include a cooling assembly 1408 at a distal end of the shaft 1404. Similar to the shaft 1404, the handle 1402 can be permanently or removably attached to the coupling member 1406. When the handle 1402 and the coupling member 1406 are removably attached, they can include corresponding coupling adapters (not shown). Between connections to the handle 1402 and the shaft 104, the coupling member 1406 can include a branch 1410. In other embodiments, the coupling member 1406 can include additional branches or no branches. Furthermore, in some embodiments, the handle 1402 can be directly attached to the shaft 1404 without the coupling member 1406. For example, the handle 1402 and the shaft 1404 can include corresponding coupling adapters (not shown).

As shown in FIG. 9A, the handle 1402 can include a cartridge housing 1412 and a cap 1414 having a cartridge connector 1416. The cryotherapeutic system 1400 can include a cartridge 1418 within the cartridge housing 1412. The cartridge housing 1412 and the cap 1414 can be separable to expose an interior of the cartridge housing 1412, which can allow replacement of the cartridge 1418. The cryotherapeutic system 1400 can include a supply passage 1420 extending between the cartridge connector 1416 and the cooling assembly 1408. When the cartridge housing 1412 and the cap 1414 are connected, the cartridge connector 1416 can be configured to open a fluidic connection between the cartridge 1418 and the supply passage 1420. The cryotherapeutic system 1400 can further include an exhaust passage 1422 extending between the branch 1410 of the coupling member 1406 and the cooling assembly 1408. The branch 1410 can be configured to vent refrigerant exhaust to the atmosphere proximate the handle 1402. In some embodiments, the branch 1410 can include an adapter (e.g., a luer) configured to facilitate connection of the exhaust passage 1422 to an exhaust tube, to an exhaust containment vessel, or to an inflation/deflation tool (e.g., a syringe).

Including the cartridge 1418 in the handle 1402 can allow the cryotherapeutic system 1400 to have reduced size, greater maneuverability, and/or fewer external connections than other cryotherapeutic systems. In some embodiments, the cryotherapeutic system 1400 can be handheld and/or can have a total weight less than about 2 kg (e.g., less than about 1 kg or less than about 0.5 kg). The cryotherapeutic system 1400 can be fully or partially disposable. Furthermore, the compact size of the cryotherapeutic system 1400 can facilitate shipment to a central location for recycling or refurbishing. In some embodiments, the cryotherapeutic system 1400 can include generally no battery or other electrical components. In these and other embodiments, the cryotherapeutic system 1400 can be functional for therapeutically effective cryogenic renal neuromodulation without connection to external equipment or external material sources (e.g., external sources of power or refrigerant). This can reduce the complexity of setup for a treatment procedure. The cartridge 1418 can be relatively compact and can have one or more of the size and capacity characteristics discussed above with reference to the cartridge 1222 shown in FIG. 7A.

Operation of the cryotherapeutic system 1400 can include separating the cartridge housing 1412 from the cap 1414, introducing the cartridge 1418 into the cartridge housing 1412, and reconnecting the cartridge housing 1412 and the cap 1414. In some embodiments, connecting the cartridge housing 1412 and the cap 1414 can cause the cartridge connector 1416 to puncture a membrane or open a check valve of the cartridge 1418. For example, the cartridge housing 1412 can be configured to hold the cartridge 1418 in a fixed position and connecting the cartridge housing 1412 and the cap 1414 can force a pin (not shown) of the cartridge connector 1416 into a membrane (not shown) or a check valve (not shown) of the cartridge 1418. In some embodiments, the cartridge housing 1412 and the cap 1414 can be threaded, and connecting the cartridge housing 1412 and the cap 1414 can include screwing together the cartridge housing 1412 and the cap 1414. For example, the cartridge housing 1412 can include an opening (not shown) through which the cartridge 1418 can be introduced and a female-threaded portion (not shown) around the opening. The cap 1414 can include a male-threaded portion proximate an end of the cap 1414 adjacent to the cartridge connector 1416. In other embodiments, the cartridge housing 1412 can include a male-threaded portion and the cap 1414 can include a female-threaded portion. The cartridge housing 1412 and the cap 1414 can also have a different connection mechanism.

In some embodiments, fully connecting the cartridge housing 1412 and the cap 1414 can initiate refrigerant flow to the cooling assembly 1408. For example, corresponding threaded portions of the cartridge housing 1412 and the cap 1414 can be partially engaged to keep the cartridge housing 1412 and the cap 1414 together during placement of the cooling assembly 1408 within the vasculature and then fully engaged to begin cryotherapeutic renal-nerve modulation. The thread pattern of the corresponding threaded portions can be selected to facilitate rapid movement of the cartridge connector 1416 from a disengaged position to an engaged position. For example, the thread pitch can be selected to increase the amount of axial movement of the cartridge connector 1416 toward the cartridge 1418 caused by relative rotation of the cartridge housing 1412 and the cap 1414. In some embodiments, the thread pattern can have a pitch greater than about 3 mm (e.g., greater than about 4 mm or greater than about 5 mm). The cartridge housing 1412, the cap 1414, and/or the cartridge 1418 can be configured to reduce or eliminate refrigerant leakage. For example, the cartridge 1418 or the cartridge connector 1416 can include a sealing member (e.g., a gasket) configured to at least partially seal an interface between the cartridge 1418 and the cartridge connector 1416 during and after connecting the cartridge housing 1412 and the cap 1414. Similarly, an interface between the cartridge housing 1412 and the cap 1414 can facilitate sealing. In some embodiments, corresponding threaded portions of the cartridge housing 1412 and the cap 1414 can be coated with a sealing material (e.g., polytetrafluoroethylene or another suitable material).

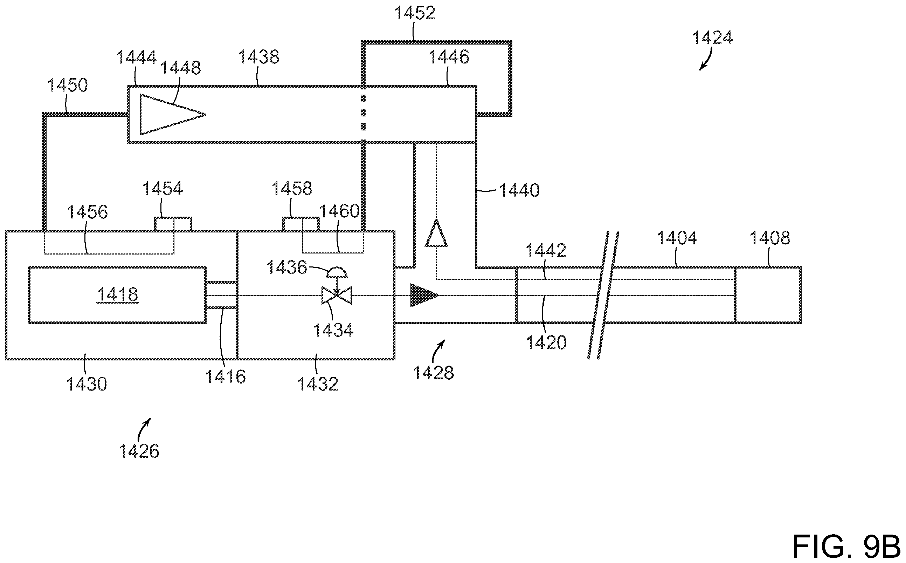

FIG. 9B is a partially schematic diagram illustrating a cryotherapeutic system 1424 similar to the cryotherapeutic system 1400 shown in FIG. 9A. The cryotherapeutic system 1424 can include a handle 1426 and a coupling member 1428 between the handle 1426 and the shaft 1404. The handle 1426 can include a cartridge housing 1430 and a cap 1432. Along the supply passage 1420 and within the cap 1432, the cryotherapeutic system 1424 can include a supply valve 1434. The cryotherapeutic system 1424 can further include a first actuator 1436 operably connected to the supply valve 1434. The supply valve 1434 can be configured to mechanically default (e.g., spring) to a closed position. In some embodiments, the first actuator 1436 can be manual (e.g., a push button) and can require positive force to maintain the supply valve 1434 in an open position. For example, when the first actuator 1436 is a button, the supply valve 1434 can be open when the button is held down by an operator (e.g., by an operator's thumb) and can close rapidly when the button is released. This configuration can increase the likelihood that the supply valve 1434 will be opened deliberately and for a deliberate period of time. When closed, the supply valve 1434 can prevent refrigerant from flowing to the cooling assembly 1408. When open, the supply valve 1434 can permit refrigerant to flow to the cooling assembly 1408 and can thereby initiate and maintain cryogenic cooling proximate the cooling assembly 1408.

As shown in FIG. 9B, the cryotherapeutic system 1424 can include a vortex tube 1438 and the coupling member 1428 can include a branch 1440 connected to the vortex tube 1438. The cryotherapeutic system 1424 can further include a first exhaust passage 1442 extending between the cooling assembly 1408 and the vortex tube 1438. The vortex tube 1438 can be configured to separate all or a portion of the refrigerant exhaust flowing through the first exhaust passage 1442 into separate streams, with one stream being warmer than the other. For example, the vortex tube 1438 can include a first end portion 1444, a second end portion 1446, and an elongated chamber (not separately identified) extending between the first and second end portions 1444, 1446. The first exhaust passage 1442 can connect to the chamber generally tangentially and angled relative to the longitudinal axis of the chamber so as to cause refrigerant exhaust from the first exhaust passage 1442 to swirl within the chamber toward the first end portion 1444. The vortex tube 1438 can further include a tapered element 1448 (e.g., a cone-shaped element) at the first end portion 1444. The tapered element 1448 can be configured to redirect cooler refrigerant exhaust within an inner portion of the chamber toward the second end portion 1446. Warmer refrigerant exhaust can exit the chamber through a passage around the tapered element 1448. Cooler refrigerant exhaust can exit the chamber through a passage at the second end portion 1446. Other configurations of the vortex tube 1438 are also possible.

In some embodiments, warmer and cooler refrigerant streams can be useful for modifying the temperature (e.g., via heat exchange) of other refrigerant within the cryotherapeutic system 1424 prior to and during delivery to the cooling assembly 1408. For example, the cooler refrigerant stream from the vortex tube 1438 can be used to pre-cool refrigerant after it exits the cartridge 1418 (e.g., to increase the cooling capacity of the refrigerant). The warmer refrigerant stream from the vortex tube 1438 can be used, for example, to heat refrigerant within the cartridge 1418 (e.g., to extend the time before pressure within the cartridge 1418 decays to an unacceptably low level). In contrast to other mechanisms for providing warmer and cooler refrigerant streams, the vortex tube 1438 can operate without electricity and can have few (if any) moving parts. Accordingly, the vortex tube 1438 can be particularly useful when the cryotherapeutic system 1424 is self-contained.

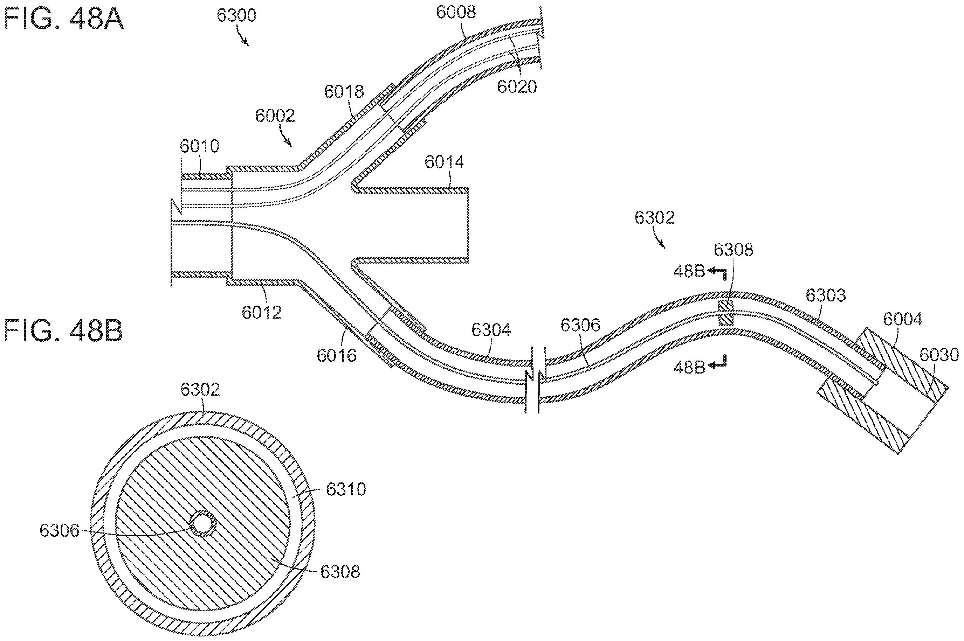

The cryotherapeutic system 1424 can include a first intermediate conduit 1450 extending between the first end portion 1444 and the cartridge housing 1430 and a second intermediate conduit 1452 extending between the second end portion 1446 and the cap 1432. As shown in FIG. 9B, the first and second intermediate conduits 1450, 1452 can be exposed (e.g., spaced apart from the vortex tube 1438). In other embodiments, the first and second intermediate conduits 1450, 1452 can be embedded within a periphery of the vortex tube 1438 and/or within the branch 1440. In still other embodiments, the vortex tube 1438 can be directly adjacent to the handle 1426 and the first and second intermediate conduits 1450, 1452 can be replaced with openings between the vortex tube 1438 and the handle 1426. With reference again to FIG. 9B, the cartridge housing 1430 can include a first exhaust port 1454 and a second exhaust passage 1456 extending between the first intermediate conduit 1450 and the first exhaust port 1454. The second exhaust passage 1456 can be configured to warm refrigerant within the cartridge 1418 (e.g., to a temperature greater than ambient temperature). For example, the second exhaust passage 1456 can include a heat-exchange portion proximate the cartridge 1418. Advantages of warming refrigerant within the cartridge 1418 are described, for example, above with reference to FIG. 7A. The cap 1432 can include a second exhaust port 1458 and a third exhaust passage 1460 extending between the second intermediate conduit 1452 and the second exhaust port 1458. The third exhaust passage 1460 can be configured to cool refrigerant within the supply passage 1420. For example, the third exhaust passage 1460 can include a heat-exchange portion (not shown) proximate the supply passage 1420. Advantages of cooling refrigerant within the supply passage 1420 are described, for example, below with reference to FIGS. 48A-50.

In some embodiments, another heat source in addition to or instead of the second exhaust passage 1456 can warm refrigerant within the cartridge 1418. For example, the cartridge housing 1430 can be configured to receive and/or the cryotherapeutic system 1424 can include a heat pack (not shown) (e.g., a chemical and/or disposable heat pack) proximate the cartridge 1418. Use of the cryotherapeutic system 1424 can include activating the heat pack prior to initiating refrigerant flow to the cooling assembly 1408. Furthermore, the cartridge housing 1430 can be configured to facilitate heat transfer between an operator's hand and the cartridge 1418. For example, the cartridge housing 1430 can be configured such that there is generally no air gap and an average thickness between about 3 mm and about 12 mm (e.g., between about 4 mm and about 10 mm) between refrigerant within the cartridge 1418 loaded in the cartridge housing 1430 and an external surface of the cartridge housing 1430 along at least one side of the cartridge 1418 (e.g., a side configured to be adjacent to an operator's palm).

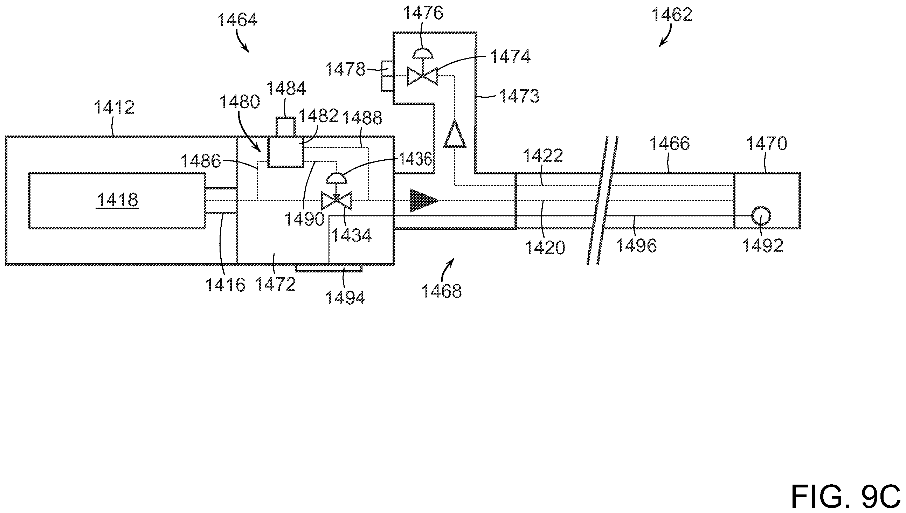

FIG. 9C is a partially schematic diagram illustrating a cryotherapeutic system 1462 that can include a handle 1464, an elongated shaft 1466, and a coupling member 1468 between the handle 1464 and the shaft 1466. The cryotherapeutic system 1462 can further include a cooling assembly 1470 at a distal end of the shaft 1466. The handle 1464 can include a cap 1472 connected to the coupling member 1468. Along the exhaust passage 1422 and within a branch 1473 of the coupling member 1468, the cryotherapeutic system 1462 can include an exhaust valve 1474. The cryotherapeutic system 1462 can further include a second actuator 1476 operatively connected to the exhaust valve 1474. In some embodiments, the second actuator 1476 can be a spring-loaded actuator configured to maintain a pressure within the exhaust passage 1422 distal to the exhaust valve 1474. For example, the second actuator 1476 can be configured to cause the exhaust valve 1474 to operate as a pressure-relief valve.

The exhaust valve 1474 can be configured to maintain a back pressure within the cooling assembly 1470. As discussed, for example, above with reference to FIG. 1A, the back pressure within the cooling assembly 1470 can change the cooling temperature within the cooling assembly 1470. In some embodiments, the second actuator 1476 can be adjustable (e.g., manually adjustable) and the exhaust valve 1474 can change the back pressure within the cooling assembly 1470 to cause different cooling temperatures within the cooling assembly 1470. The exhaust valve 1474 can also maintain and/or change an inflation pressure of a balloon (not shown) within the cooling assembly 1470. The coupling member 1468 can include an adapter 1478 fluidly connected to the exhaust passage 1422 proximate the exhaust valve 1474. The adapter 1478 can be configured to receive an inflation/deflation device (e.g., a syringe). Operating the cryotherapeutic system 1462 can include inflating the balloon via the adapter 1478 after the cooling assembly 1470 is at a desired position within the vasculature and before opening the supply valve 1434. The exhaust valve 1474 can prevent the balloon from deflating via the exhaust passage 1422. In some embodiments, the exhaust valve 1474 can be opened or removed after a treatment procedure is completed so that the balloon can be deflated (e.g., in response to external pressure on the balloon within the vasculature and/or in response to suction applied to the exhaust passage 1422 via the adapter 1478).

As shown in FIG. 9C, the cryotherapeutic system 1462 can include a timer 1480 having a body 1482 and a button 1484. The timer 1480 can be configured to operate without electricity. For example, the timer 1480 can be mechanical and/or pneumatic. The cryotherapeutic system 1462 can include a pneumatic inlet line 1486 and a pneumatic control line 1488 extending, respectively, between the body 1482 and portions of the supply passage 1420 proximal and distal to the supply valve 1434. The cryotherapeutic system 1462 can further include a pneumatic outlet line 1490 extending between the body 1482 and the first actuator 1436. The timer 1480 can be configured to pneumatically open the supply valve 1434 via the first actuator 1436 when the button 1484 is pressed and to maintain the supply valve 1434 in an opened state for a predetermined period (e.g., the duration of a cryogenic cooling cycle). At the end of the period, the timer 1480 can be configured to release the first actuator 1436 and to cause the supply valve 1434 to default (e.g., spring) to a closed position. In some embodiments, the timer 1480 can include a piston (not shown) and a piston chamber (not shown). The piston chamber can be configured to receive refrigerant exhaust from the pneumatic control line 1488. Pushing the button 1484 can unlock and/or move the piston, which can unblock the pneumatic outlet line 1490, actuate the first actuator 1436, and cause the supply valve 1434 to open. The pneumatic control line 1488 and/or an orifice (not shown) along the pneumatic control line 1488 can restrict the flow of refrigerant exhaust into the piston chamber so as to cause a delay before the piston chamber reaches a pressure sufficient to return the piston to its original position. In its original position, the piston can block the pneumatic outlet line 1490, which can remove pneumatic pressure from the first actuator 1436 and cause the supply valve 1434 to default (e.g., spring) to a closed position. Other configurations of the timer 1480 are also possible.

The cryotherapeutic system 1462 can be configured for temperature monitoring (e.g., cryogenic-temperature monitoring) at or near the cooling assembly 1470. In some embodiments, the temperature monitoring can be non-electrical. For example, the cryotherapeutic system 1462 can include a gas thermometer (not separately identified) including a bulb 1492 within the cooling assembly 1470 and a temperature display 1494 at an exterior portion of the cap 1472. The gas thermometer can further include a capillary tube 1496 extending between the bulb 1492 and the temperature display 1494. The temperature display 1494 can include a mechanical gauge (not shown) calibrated to indicate temperature based on changes in pressure within the bulb 1492 and the capillary tube 1496 caused by changes in temperature proximate the bulb 1492. In embodiments having a gas thermometer, the connections between the cap 1472 and the coupling member 1468 and between the coupling member 1468 and the shaft 1466 can be permanent to prevent disruption of the capillary tube 1496. In other embodiments, the temperature display 1494 can be at an external portion of the coupling member 1468 and the connection between the cap 1472 and the coupling member 1468 can be releasable.

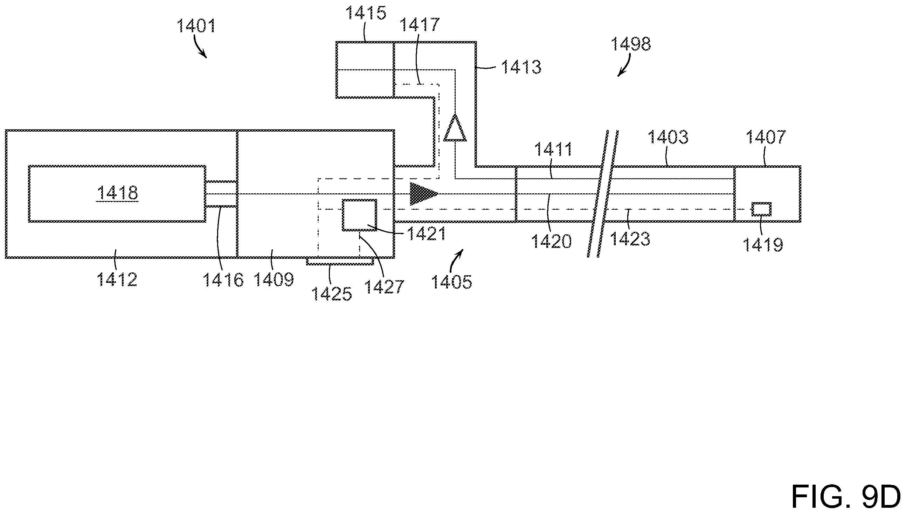

FIG. 9D is a partially schematic diagram illustrating a cryotherapeutic system 1498 similar to the cryotherapeutic system 1400 shown in FIG. 9A. The cryotherapeutic system 1498 can include a handle 1401, an elongated shaft 1403, and a coupling member 1405 between the handle 1401 and the shaft 1403. The cryotherapeutic system 1498 can further include a cooling assembly 1407 at a distal end of the shaft 1403. The handle 1401 can include a cap 1409 connected to the coupling member 1405. The cryotherapeutic system 1498 can include an exhaust passage 1411 and can be configured to generate electricity from refrigerant exhaust moving through the exhaust passage 1411. As shown in FIG. 9D, the coupling member 1405 can include a branch 1413 and a dynamo 1415 at an end portion of the branch 1413. The exhaust passage 1411 can extend through the branch 1413 and the dynamo 1415 to a vent (not shown) of the dynamo 1415. The dynamo 1415 can include a turbine (not shown) configured to turn in response to movement of refrigerant exhaust through the dynamo 1415. The cryotherapeutic system 1498 can include a first electrical line 1417 configured to carry electrical energy generated by the dynamo 1415.

Electrical energy from the dynamo 1415 can be used to power a variety of suitable sensors, displays, actuators, or other elements of the cryotherapeutic system 1498. In some embodiments, electrical energy from the dynamo 1415 can be used to power temperature-monitoring elements of the cryotherapeutic system 1498. For example, as shown in FIG. 9D, the cryotherapeutic system 1498 can include a thermocouple 1419 within the cooling assembly 1407. The thermocouple 1419 can be configured to measure a temperature proximate the cooling assembly 1407. The cryotherapeutic system 1498 can further include an analog-to-digital converter 1421 within the cap 1409 and a second electrical line 1423 extending between the thermocouple 1419 and the analog-to-digital converter 1421. The second electrical line 1423 can be configured to carry an analog signal from the thermocouple 1419 to the analog-to-digital converter 1421, and the analog-to-digital converter 1421 can be configured to convert the analog signal to a digital signal. The cryotherapeutic system 1498 can further include a temperature display 1425 (e.g., a liquid-crystal display) at an exterior portion of the cap 1409 and a third electrical line 1427 extending between the analog-to-digital converter 1421 and the temperature display 1425. The third electrical line 1427 can be configured to carry a digital signal from the analog-to-digital converter 1421 to the temperature display 1425, and the temperature display 1425 can be configured to convert the digital signal into a readable form. As shown in FIG. 9D, the first electrical line 1417 can branch and extend to the analog-to-digital converter 1421 and the temperature display 1425. In some embodiments, the analog-to-digital converter 1421 and/or the temperature display 1425 can have different locations in the cryotherapeutic system 1498 (e.g., within or on the coupling member 1405).

### D. Selected Examples of Cryotherapeutic-System Components

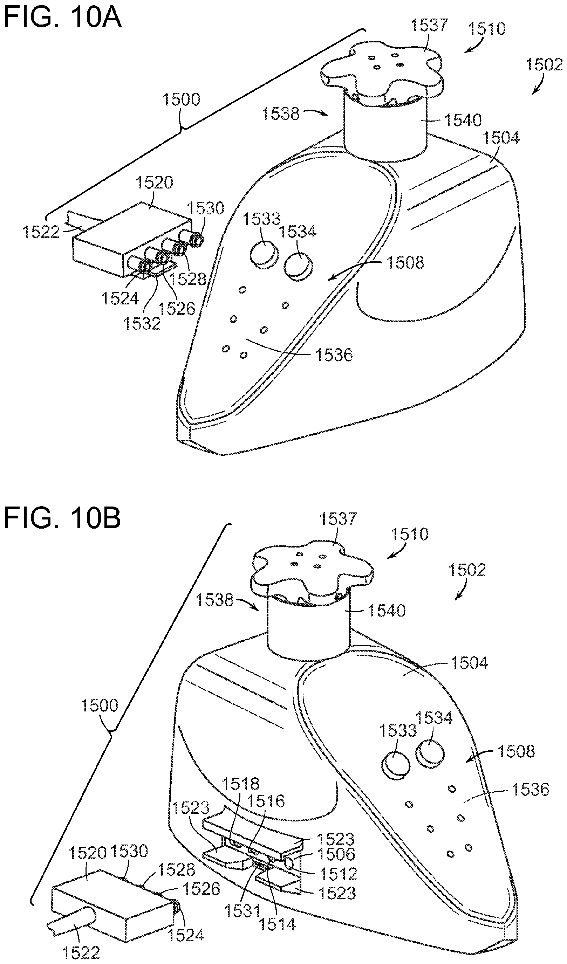

Selected examples of cryotherapeutic-system components configured in accordance with embodiments of the present technology are described in this section with reference to FIGS. 10A-44. It will be appreciated that specific elements, substructures, advantages, uses, and/or other features of the embodiments described with reference to FIGS. 10A-44 can be suitably interchanged, substituted, or otherwise configured with one another and/or with the embodiments described with reference to FIGS. 1A-9D and 45B-67 in accordance with additional embodiments of the present technology. Furthermore, suitable elements of the embodiments described with reference to FIGS. 10A-44 can be used as stand-alone and/or self-contained devices.

FIGS. 10A-10B are perspective views illustrating a console assembly 1500 that can include a console 1502 having a primary housing 1504, a first umbilical connector 1506 (FIG. 10B), a user interface 1508, and a cartridge housing 1510. Similar to the first umbilical connector 1206 of FIGS. 7A-7C, the first umbilical connector 1506 of FIG. 10B can include a first outlet adapter 1512, a second outlet adapter 1514, a first inlet adapter 1516, and a first pressure-monitoring adapter 1518. The console assembly 1500 can further include a second umbilical connector 1520 and an umbilical cord 1522 extending to other portions of a cryotherapeutic device (not shown). In some embodiments, the first umbilical connector 1506 can be integral to the primary housing 1504. As shown in FIG. 10B, the first umbilical connector 1506 can have flanges 1523 extending laterally from upper and lower portions of the first umbilical connector 1506. The flanges 1523 can facilitate placement of the second umbilical connector 1520.

The second umbilical connector 1520 can include a second inlet adapter 1524, a third inlet adapter 1526, a third outlet adapter 1528, and a second pressure-monitoring adapter 1530 removably connectable to the first outlet adapter 1512, the second outlet adapter 1514, the first inlet adapter 1516, and the first pressure-monitoring adapter 1518, respectively. The first umbilical connector 1506 can include a first data connector 1531 (FIG. 10B) and the second umbilical connector 1520 can include a second data connector 1532 (FIG. 10A). The first and second data connectors 1531, 1532 can be a variety of suitable connector types (e.g., USB), and can be configured to provide a data connection between the console 1502 and the cryotherapeutic device. Such a data connection can be useful, for example, to convey data from sensors (e.g., temperature, pressure, and/or flow-rate sensors) within the cryotherapeutic device to the console 1502 for display, processing, and/or control. As shown in FIGS. 10A-10B, the user interface 1508 can include an initiation button 1533, a termination button 1534, and a display 1536. The initiation button 1533 and the termination button 1534 can be configured, respectively, to initiate and terminate flow of refrigerant through the first outlet adapter 1512. The display 1536 can be configured to indicate status and/or operational information to an operator.

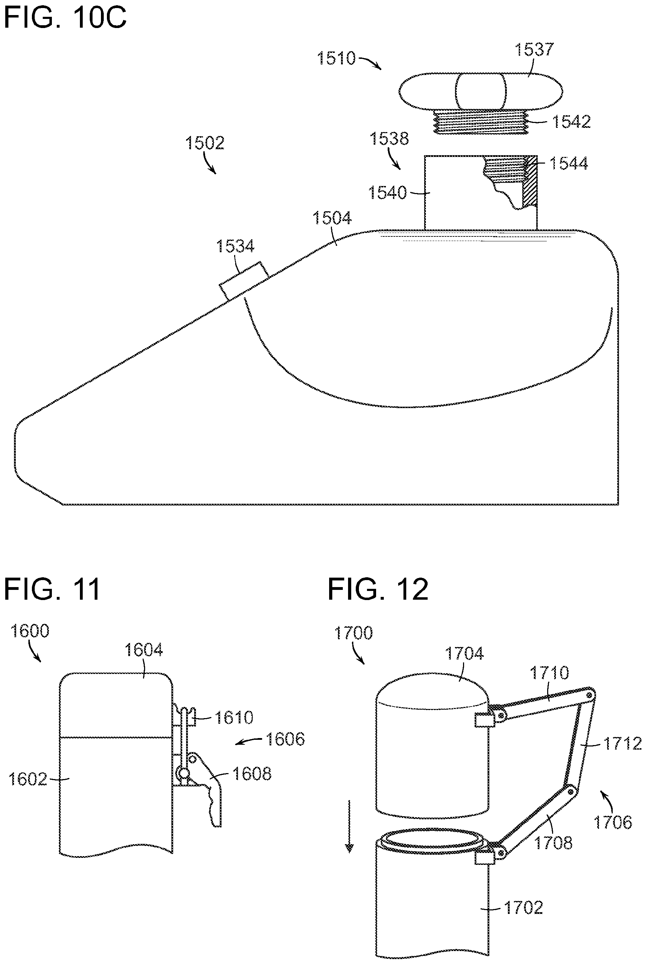

The cartridge housing 1510 can include a lid 1537 and a main portion 1538 having a cylindrical extension 1540. The lid 1537 can be removably connectable to the main portion 1538. FIG. 10C is a partially exploded side-profile view illustrating the console 1502 with the lid 1537 spaced apart from the cylindrical extension 1540. The lid 1537 and the cylindrical extension 1540 can include cooperating coupling members configured to releasably lock the lid 1537 to the cylindrical extension 1540. For example, the lid 1537 can include a first threaded portion 1542, and the cylindrical extension 1540 can include a second threaded portion 1544. The first and second threaded portions 1542, 1544 can be configured to form a high-pressure seal. In some embodiments, rotating the lid 1537 can move the lid 1537 toward the main portion 1538, which can drive a cartridge (not shown) against a connector (not shown) or other coupling member.

FIG. 11 is a profile view illustrating a cartridge housing 1600. FIG. 12 is a perspective view illustrating a cartridge housing 1700. The cartridge housings 1600, 1700 can be well suited for use with the console assembly 1500 of FIGS. 10A-10C. As shown in FIG. 11, the cartridge housing 1600 can include a main portion 1602, a lid 1604, and a latch clamp 1606 extending between the main portion 1602 and the lid 1604. The latch clamp 1606 can include a first coupling member 1608 attached to the main portion 1602 and a second coupling member 1610 attached to the lid 1604. As shown in FIG. 12, the cartridge housing 1700 can include a main portion 1702, a lid 1704, and a coupling assembly 1706 extending between the main portion 1702 and the lid 1704. The coupling assembly 1706 can include a first coupling member 1708 attached to the main portion 1702, a second coupling member 1710 attached to the lid 1704, and a third coupling member 1712 extending between the first and second coupling members 1708, 1710. The first, second, and third coupling members 1708, 1710, 1712 can be configured such that moving the third coupling member 1712 downward can drive the lid 1704 toward the main portion 1702. The coupling assembly 1706 can snap into a locked position when the first, second, and third coupling members 1708, 1710, 1712 are generally in vertical alignment. The latch clamp 1606 (FIG. 11) and the coupling assembly 1706 (FIG. 12) can be configured, respectively, to drive the lids 1604, 1704 toward the main portions 1602, 1702.

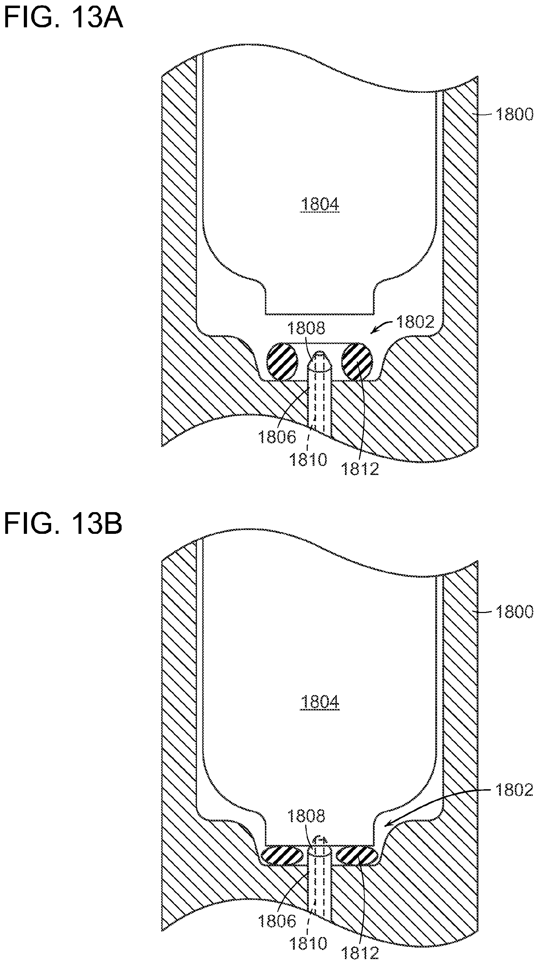

FIGS. 13A-13B are partially cross-sectional views illustrating a cartridge housing 1800, a cartridge connector 1802, and a cartridge 1804. In FIG. 13A, the cartridge 1804 is shown spaced apart from the cartridge connector 1802. In FIG. 13B, the cartridge 1804 is shown coupled to the cartridge connector 1802. The cartridge connector 1802 can include a first coupling member 1806 configured to interact with a second coupling member (not shown) of the cartridge 1804 to open a fluidic connection with a cartridge chamber (not shown) within the cartridge 1804. As shown in FIGS. 13A-13B, the first coupling member 1806 can be elongated and can include a conical tip 1808 and a coupling-member lumen 1810. The second coupling member can include, for example, a membrane (not shown) and/or a check valve (not shown). When the cartridge 1804 is pressed into the cartridge housing 1800, the first coupling member 1806 can be configured to pierce the membrane and/or open the check valve.

The cartridge connector 1802 can include a gasket 1812 around the first coupling member 1806. The gasket 1812 can have an uncompressed state (FIG. 13A) and a compressed state (FIG. 13B). The first coupling member 1806 can be recessed relative to the gasket 1812 in the uncompressed state and protruding relative to the gasket 1812 in the compressed state. The cartridge 1804 can have a contact portion (not shown) around the second coupling member. The contact portion can be configured to press the gasket 1812 from the uncompressed state to the compressed state. The cartridge connector 1802 and the cartridge 1804 of FIGS. 13A-13B can be portions of the cartridge housing 1510 of FIGS. 10A-10C. With reference to FIGS. 10A-10C and 18-19, securing the lid 1537 to the main portion 1538 of the cartridge housing 1510 can cause and/or maintain a connection between the first coupling member 1806 and the second coupling member. For example, the cartridge housing 1510 and/or the cartridge 1804 can be sized such that screwing the lid 1537 to the cylindrical extension 1540 can force the second coupling member of the cartridge 1804 toward the first coupling member 1806 of the cartridge connector 1802. In some embodiments, the cartridge connector 1802 can be configured such that the weight of the cartridge 1804 alone is not sufficient to press the gasket 1812 from the uncompressed state to the compressed state. This can help to ensure that fluidic connection of the cartridge 1804 is deliberate.

Cartridges (e.g., the cartridge 1804 of FIGS. 13A-13B) configured in accordance with embodiments of the present technology can include both liquid and gaseous refrigerant. Cartridge housings (e.g., the cartridge housing 1510 of FIGS. 10A-10C) configured in accordance with embodiments of the present technology can be configured to position cartridges such that liquid refrigerant exits the cartridges before gaseous refrigerant. Gravity typically causes liquid refrigerant to settle at lower portions of cartridges. Accordingly, cartridge housings configured in accordance with embodiments of the present technology can be configured to receive cartridges oriented with coupling members of the cartridges at lowermost portions of the cartridges when structures (e.g., the console 1502 of FIG. 10C) including the cartridge housings are positioned upright on flat surfaces. For example, cartridge connectors (e.g., the cartridge connector 1802 of FIGS. 13A-13B) configured in accordance with embodiments of the present technology can be at lower portions of cartridge housings. Moreover, cartridge housings configured in accordance with embodiments of the present technology can include lids (e.g., the lid 1537 of FIGS. 10A-10C) at first end portions of the cartridge housings and cartridge connectors at second, opposite end portions of the cartridge housings. The second end portions can be lower than the first end portions when structures including the cartridge housings are positioned upright on flat surfaces. In some embodiments, consoles configured in accordance with embodiments of the present technology can include tilt sensors or accelerometers to indicate when tilting or other movement has occurred that may affect the orientation of cartridges and/or the flow of refrigerant from the cartridges. Such sensors can be proximate cartridge housings and can be configured to trigger an alarm, terminate a procedure, or cause another result if tilting or other movement is detected.

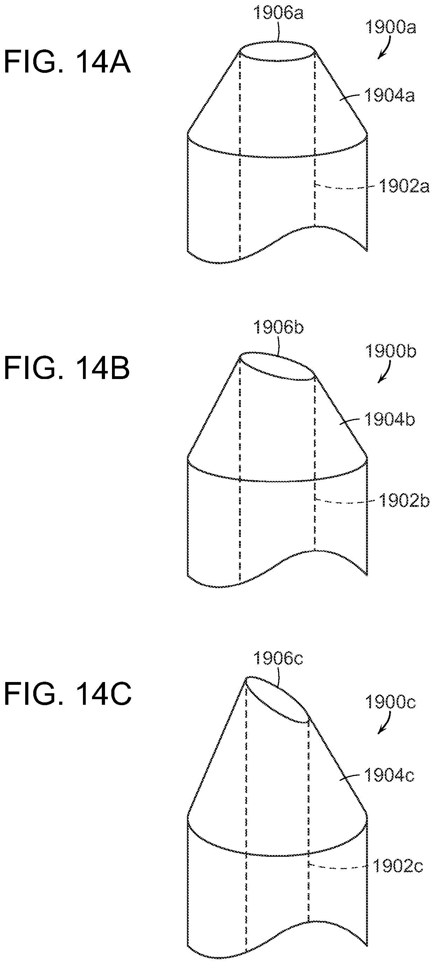

FIGS. 14A-14C are perspective views illustrating coupling members 1900a-1900c that can include coupling-member lumens 1902a-1902c and tips 1904a-1904c having rims 1906a-1906c around the coupling-member lumens 1902a-1902c. As shown in FIG. 14A, the rim 1906a can be generally flat. As shown in FIG. 14B, the rim 1906b can be oriented at a first angle relative to a length of the coupling member 1900b. As shown in FIG. 14C, the rim 1906c can be oriented at a second angle relative to a length of the coupling member 1900c, and the second angle can be less than the first angle. Different tip configurations, such as those shown in FIGS. 14A-14C can be useful to facilitate connection to different types of coupling members of cartridges. For example, the tip 1900a can be particularly useful for opening a check valve, and the tip 1900c can be particularly useful for piercing a membrane.

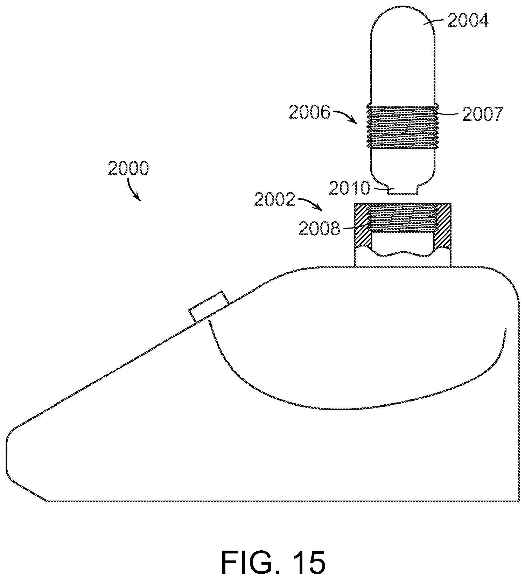

Cartridges configured in accordance with embodiments of the present technology can be configured to lock in fixed positions relative to cartridge connectors of cartridge housings. FIG. 15 is a partially cross-sectional view illustrating a console 2000 similar to the console 1502 of FIGS. 10A-10C, but with a cartridge housing 2002 that does not include a lid. FIG. 15 also shows a cartridge 2004 that can include a coupling member (not shown) and a locking member 2006. In some embodiments, the locking member 2006 can include a first threading 2007 configured to cooperate with a second threading 2008 of the cartridge housing 2002. As the cartridge 2004 is rotated into the cartridge housing 2002, the coupling member of the cartridge 2004 can move toward and eventually engage a coupling member (not shown) of the cartridge housing 2002. In other embodiments, the cartridge housing 2002 and/or the cartridge 2004 can have different mechanisms for releasably locking the cartridge 2004 in a fixed position relative to a cartridge connector of the cartridge housing 2002. For example, the cartridge housing 2002 and the cartridge 2004 can include portions of a spring-lock mechanism (not shown). The console 2000 can include a release switch (not shown) operably connected to the spring-lock mechanism and configured to release the spring-lock mechanism so that the cartridge 2004 can be moved away from the cartridge connector of the cartridge housing 2002. The coupling member of the cartridge 2004 can have various suitable positions on the cartridge 2004. For example, the cartridge 2004 can have a neck portion 2010 that can include threading or another suitable type of coupling member.

Consoles and other cryotherapeutic-system components configured in accordance with embodiments of the present technology can have various positions in an operating room during a treatment procedure. For example, consoles can be configured to rest on an operating table, to rest under an operating table, to hang from an I.V. pole, or to rest on a dedicated or shared equipment bench. In some embodiments, it can be desirable to locate consoles near patients. This can reduce the lengths of refrigerant supply lines and reduce supply-line pressure drops as well as potential warming and expansion of refrigerant before it reaches cooling assemblies within the vasculature. During a treatment procedure, locations near a patient typically are within a sterile field. Accordingly, consoles configured in accordance with embodiments of the present technology can be configured for use within a sterile field. Instead of or in addition to sterilizing the consoles, sterile barriers can be provided around all or portions of the consoles.

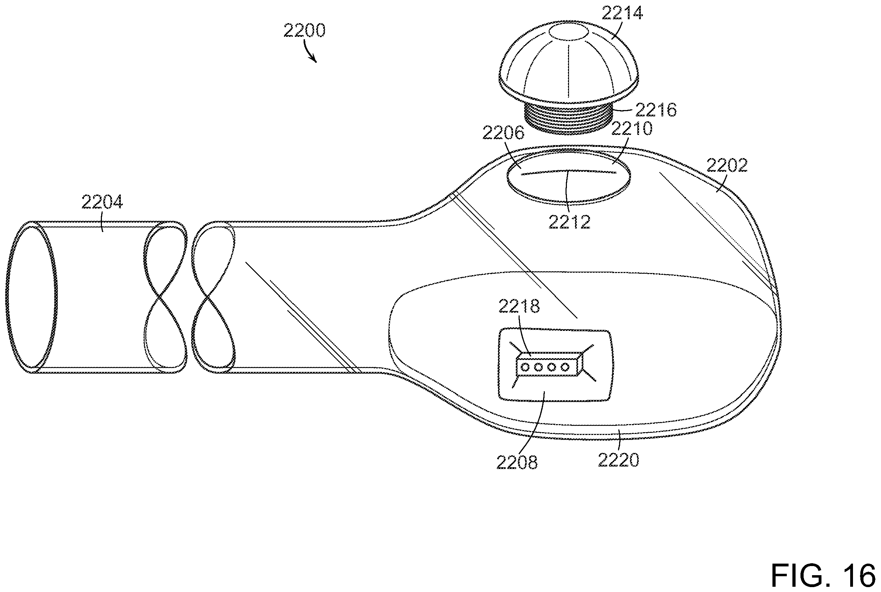

FIG. 16 is a partially exploded perspective view illustrating a bag 2200 that can be configured to form a sterile barrier around the console 1502 of FIGS. 10A-10C. With reference to FIGS. 10A-10C and 16, the bag 2200 can include a main portion 2202 configured to fit around the console 1502 and an elongated extension 2204 configured to fit around a power cord (not shown) connected to the console 1502. The extension 2204 can have a length sufficient to contain the power cord until it reaches a receptacle or exits the sterile field. The main portion 2202 can include a first sterile-barrier portal 2206 and a second sterile-barrier portal 2208. The first sterile-barrier portal 2206 can be configured to allow passage of a cartridge (e.g., the cartridge 1804 of FIGS. 13A-13B) generally without disrupting a sterile barrier around the console 1502. For example, the first sterile-barrier portal 2206 can include a membrane 2210 having a slit 2212 or another suitable sterile-barrier configuration.

As shown in FIG. 16, the bag 2200 can include a sterile cap 2214 having a threaded coupling member 2216. With reference to FIGS. 10A-10C and 16, the cap 2214 can be used in place of the lid 1537. For example, the coupling member 2216 can extend through the slit 2212 and screw into the main portion 1538. In other embodiments, the bag 2200 can include a collar shaped to fit around the cylindrical extension 1540. The inside of the cartridge housing 1510 can be sterile and the collar can fit snugly around the cartridge housing 1510 so as to form a sterile barrier around the outside of the cartridge housing 1510. In still other embodiments, the first sterile-barrier portal 2206 can be eliminated and one or more cartridges can be included within the bag 2200. The inside surface of the cartridge housing 1510 and the outside surface of the cartridges can be non-sterile. The bag 2200 can be sufficiently deformable to allow the cartridges to be grasped through the bag 2200 and moved into or out of the cartridge housing 1510 while remaining contained within the bag 2200. Depleted cartridges can remain in the bag 2200 during a treatment procedure. In some embodiments, the bag 2200 or the console 1502 can include a pocket (not shown) or another suitable structure configured to hold charged and/or depleted cartridges when not in use.

The second sterile-barrier portal 2208 can include a molded insert 2218 configured to allow passage of adapters (e.g., the adapters of the second umbilical connector 1520) generally without disrupting a sterile barrier around the console 1502. In other embodiments, the second sterile-barrier portal 2208 can include a membrane and a slit similar to the membrane 2210 and the slit 2212 of the first sterile-barrier portal 2206. As shown in FIG. 16, the bag 2200 can include a stiffening member 2220, such as a generally flat cardboard or plastic sheet positioned at a base portion of the bag 2200. The stiffening member 2220 can be configured to support the console 1502, to shape the bag 2200, and/or to orient the bag 2200. The bag 2200 can be made of transparent plastic or another material well suited for forming a sterile barrier. In some embodiments, the bag 2200 can be disposable. In other embodiments, the bag 2200 or portions of the bag 2200 can be reusable. For example, the cap 2214 and/or the molded insert 2218 can be reusable and other portions of the bag 2200 can be disposable.

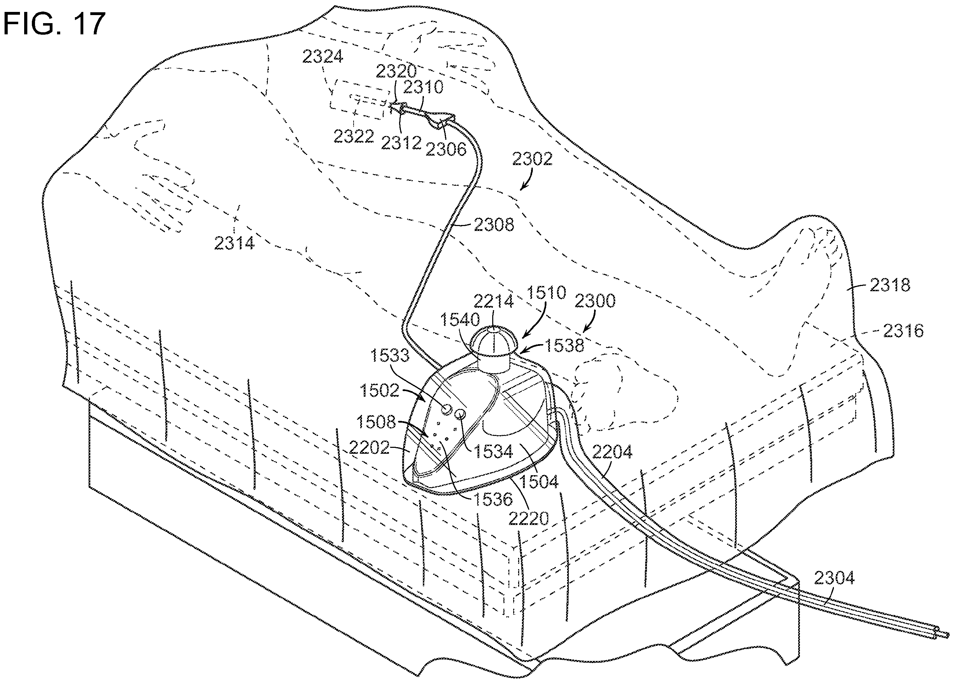

FIG. 17 is a perspective view illustrating a console assembly 2300 and portions of a cryotherapeutic device 2302 connected to the console assembly 2300 during a treatment procedure. The console assembly 2300 can include the bag 2200 of FIG. 16, the console 1502 of FIGS. 10A-10C (without the lid 1537), and a power cord 2304. The main portion 2202 of the bag 2200 can be positioned around the console 1502. The cap 2214 of the bag 2200 can extend through the first sterile-barrier portal (not shown) of the bag 2200 and connect to the cylindrical extension 1540 of the main portion 1538 of the cartridge housing 1510 of the console 1502. The extension 2204 of the bag 2200 can be positioned around the power cord 2304. As shown in FIG. 17, the user interface 1508 of the console 1502 can be visible through the bag 2200. The cryotherapeutic device 2302 can include a hub 2306 and an umbilical cord 2308 extending between the hub 2306 and the console 1502. The cryotherapeutic device 2302 can further include an entry trocar 2312 and an elongated shaft 2310 extending through the entry trocar 2312. FIG. 17 also shows a patient 2314 on a surgical table 2316 and under a surgical sheet 2318 including an entry slit 2320 with the entry trocar 2312 positioned in the slit 2320. Under the surgical sheet 2318, the shaft 2310 and the entry trocar 2312 can enter the vasculature of the patient 2314 at an entry point 2322 that can be covered by surgical tape 2324.

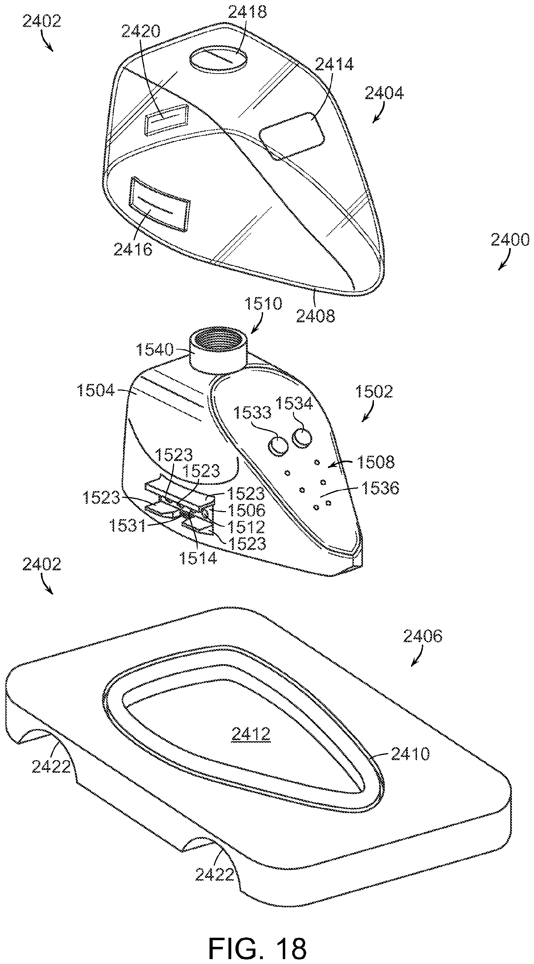

As shown in FIG. 17, the console 1502 can rest on the surgical table 2316 near the patient 2314 and over the surgical sheet 2318. Other embodiments can include components configured to facilitate other placements of the console 1502. These and other embodiments can include different structures configured to form a sterile barrier around portions of the console 1502. For example, FIG. 18 is an exploded perspective view illustrating a console assembly 2400 including a shell 2402 and the console 1502 of FIGS. 10A-10C (without the lid 1537). The shell 2402 can include a cover 2404 and a base 2406 and can be configured to form a sterile barrier around the console 1502. The cover 2404 can include a lower rim 2408 and the base 2406 can include a channel 2410 configured to receive the lower rim 2408. For example, the channel 2410 can be configured to deform slightly around the lower rim 2408 to hold the cover 2404 to the base 2406 by friction. The base 2406 can include a central recess 2412 configured to receive the console 1502. Placing the console 1502 in the central recess 2412 can reduce the possibility of the console 1502 shifting relative to the base 2406 during a treatment procedure.

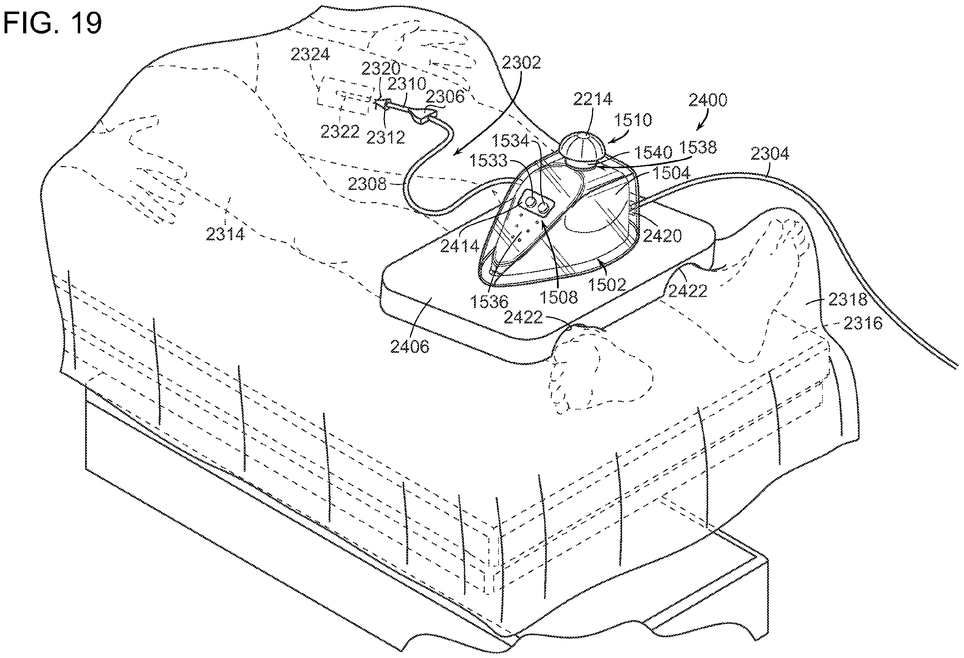

The cover 2404 can be generally rigid and transparent (e.g., composed of a generally rigid and transparent material) with components allowing for interaction with the console 1502 generally without disrupting a sterile barrier around the console 1502. For example, the cover 2404 can include a deformable membrane 2414 configured to be proximate the initiation button 1533 and the termination button 1534 of the console 1502, such that the initiation button 1533 and the termination button 1534 can be operated through the deformable membrane 2414. The shell 2402 can include a first sterile-barrier portal 2416, a second sterile-barrier portal 2418, and a third sterile-barrier portal 2420 configured to be proximate, respectively, the first umbilical connector 1506, the cartridge housing 1510, and a power cord connected to the console 1502. The base 2406 can include recesses 2422 configured to fit over a patient's legs. FIG. 19 is a perspective view illustrating the console assembly 2400 positioned over a patient's legs during a treatment procedure. Placement of the console assembly 2400 over a patient's legs can be useful, for example, when space on the surgical table 2316 is limited. As shown in FIG. 19, the power cord 2304 can extend through the third sterile-barrier portal 2420.

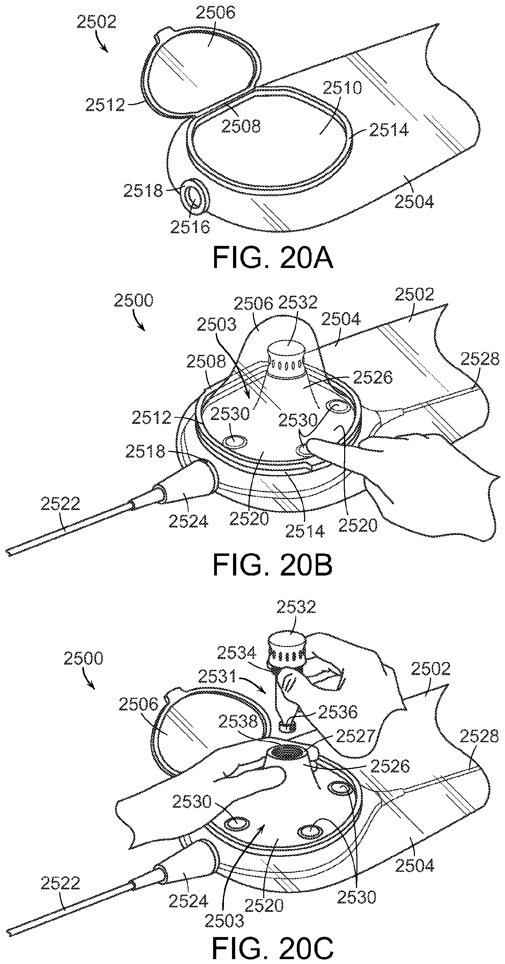

FIGS. 20A-20C are perspective views of a console assembly 2500 or a portion thereof. The console assembly 2500 can include a bag 2502 and a console 2503. FIG. 20A shows the bag 2502 prior to loading and use. The bag 2502 can include a main portion 2504, a dome-shaped flap 2506, and a living hinge 2508 between the main portion 2504 and the flap 2506. The flap 2506 can be configured to fit over a relatively large upper opening 2510 of the main portion 2504. The flap 2506 and the upper opening 2510, respectively, can include a first gasket 2512 and a second gasket 2514 configured to come together when the flap 2506 is closed so as to form a sterile seal. The first and second gaskets 2512, 2514 can be, for example, rubber gaskets. In other embodiments, the first and second gaskets 2512, 2514 can be replaced with zipper seals or other suitable sealing members. With reference again to FIG. 20A, the bag 2502 can further include an umbilical-cord opening 2516 having a third gasket 2518.

FIG. 20B shows the console assembly 2500 with the console 2503 loaded in the bag 2502. The console 2503 can include a primary housing 2520 and the bag 2502 can extend around the primary housing 2520 to form a sterile barrier around the primary housing 2520 when the flap 2506 is closed. As shown in FIG. 20B, the console assembly 2500 can include an umbilical cord 2522 having a second umbilical connector 2524 adjacent to the third gasket 2518. The primary housing 2520 can include a first umbilical connector (not shown) attached to the second umbilical connector 2524 through the umbilical-cord opening 2516. The primary housing 2520 also can include a cartridge housing 2526 having a rim 2527 (FIG. 20C). The cartridge housing 2526 can be shaped to extend upwards through the upper opening 2510 when the flap 2506 is open. When the flap 2506 is closed, the dome shape of the flap 2506 can be configured to accommodate the cartridge housing 2526. The console 2503 can include a power cord 2528 extending away from the primary housing 2520 within the bag 2502. The primary housing 2520 can include buttons 2530 configured to control operation of the console 2500. As shown in FIG. 20B, the flap 2506 can be transparent and sufficiently flexible to allow the buttons 2530 to be pressed when the flap 2506 is closed.

The console assembly 2500 can further include a cartridge 2531 (FIG. 20C). FIG. 20C illustrates loading of the cartridge 2531 into the cartridge housing 2526. The cartridge 2531 can include a cap 2532 and a threaded collar 2534 below the cap 2532. The cap 2532 can be at a first end of the cartridge 2531, and the cartridge 2531 can include a coupling member 2536 at a second, opposite end of the cartridge 2531. The cartridge housing 2526 can include internal threading 2538 configured to receive the threaded collar 2534. As the cartridge 2531 is twisted into the cartridge housing 2526, the coupling member 2536 can move toward a corresponding coupling member (not shown) of a cartridge connector (not shown) at a lowermost portion of the cartridge housing 2526. As shown in FIG. 20B, when the cartridge 2532 is fully inserted within the cartridge housing 2526, the cap 2532 can be adjacent to the rim 2527 (FIG. 20C).

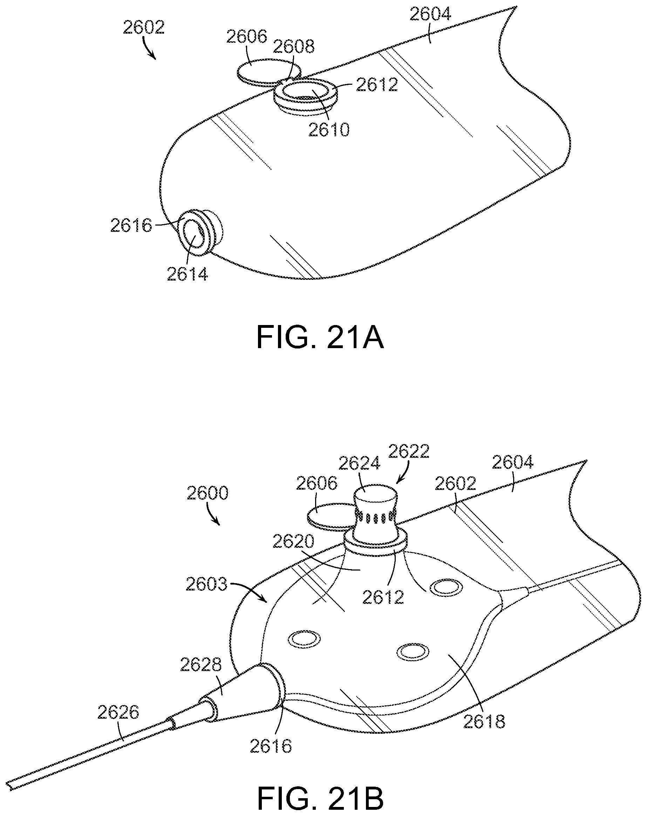

FIGS. 21A-21B are perspective views of a console assembly 2600 or a portion thereof. In the console assembly 2500 of FIGS. 20A-20C, loading the cartridge 2531 can be a non-sterile operation (e.g., performed by non-sterile personnel). The console assembly 2600 can be configured such that loading a cartridge can be a sterile operation (e.g., performed by sterile personnel). The console assembly 2600 can include a bag 2602 and a console 2603. FIG. 21A illustrates the bag 2602 prior to loading and use. The bag 2602 can include a main portion 2604, a circular flap 2606, and a living hinge 2608 between the main portion 2604 and the flap 2606. The bag 2602 also can include a cartridge opening 2610 having a first gasket 2612 and an umbilical-cord opening 2614 having a second gasket 2616.

As shown in FIG. 21B, the console 2603 can include a primary housing 2618, and the bag 2602 can extend around the primary housing 2618 to form a sterile barrier around the primary housing 2618. The primary housing 2618 can include a cartridge housing 2620 and the first gasket 2612 can fit around a rim (not shown) of the cartridge housing 2620 so that generally all of the primary housing 2618 other than an internal portion (not shown) of the cartridge housing 2620 is within a sterile barrier formed by the bag 2602 when the flap 2606 is open or closed. As shown in FIG. 21B, the console assembly 2600 can include a cartridge 2622 having a cap 2624. The cartridge 2622 can be positioned in the cartridge housing 2620 such that the cap 2624 is adjacent to the first gasket 2612. The console assembly 2600 can further include an umbilical cord 2626 having an umbilical connector 2628 adjacent to the second gasket 2616 (FIG. 21A). The cartridge 2622 and the internal portion of the cartridge housing 2620 can be sterile so that the cartridge 2622 can be loaded within the sterile field (e.g., by sterile personnel).

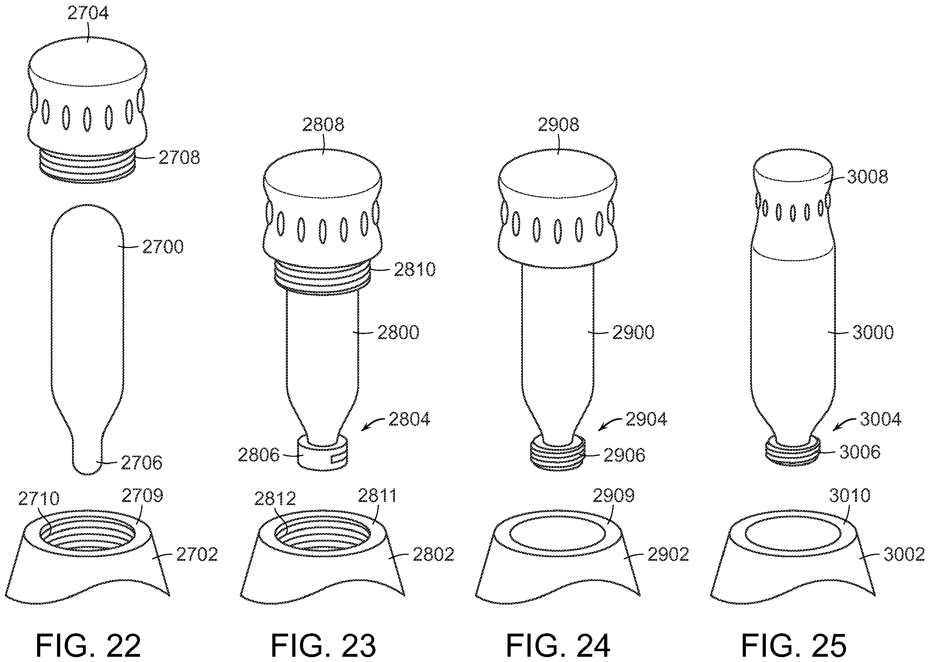

FIGS. 22-25 are perspective views of cartridges and corresponding cartridge housings. Together, FIGS. 22-25 illustrate several examples of possible interacting structures of the cartridges and corresponding cartridge housings. FIG. 22 shows a cartridge 2700, a cartridge housing 2702, and a cap 2704. The cartridge 2700 can include a tip portion 2706 having a coupling member (not shown) configured to engage a coupling member (not shown) within the cartridge housing 2702 to open a fluidic connection. The cap 2704 can be separate from the cartridge 2700 and can include a first threaded portion 2708. The cartridge housing 2702 can include a rim 2709 and a second threaded portion 2710 configured to receive the first threaded portion 2708. The cartridge 2700 and the cartridge housing 2702 can be sized such that rotating the cap 2704 onto the cartridge housing 2702 while the cartridge 2700 is within the cartridge housing 2702 can cause the coupling members of the cartridge 2700 and the cartridge housing 2702 to engage. When the cap 2704 contacts the rim 2709, the cartridge 2700 can have a desired position within the cartridge housing 2702.

FIG. 23 shows a cartridge 2800 and a cartridge housing 2802. The cartridge 2800 can include a tip portion 2804 having a coupling member 2806 configured to engage a coupling member (not shown) within the cartridge housing 2802 to open a fluidic connection. The cartridge 2800 also can include a cap 2808 having a first threaded portion 2810. The cartridge housing 2802 can include a rim 2811 and a second threaded portion 2812 configured to receive the first threaded portion 2810. The cartridge 2800 and the cartridge housing 2802 can be sized such that rotating the cartridge 2800 into the cartridge housing 2802 can cause the coupling member 2806 of the cartridge 2800 and the coupling member of the cartridge housing 2802 to engage. When the cap 2808 contacts the rim 2811, the cartridge 2800 can have a desired position within the cartridge housing 2802.

FIG. 24 shows a cartridge 2900 and a cartridge housing 2902. The cartridge 2900 can include a tip portion 2904 having a coupling member (not shown) and a threaded locking member 2906 configured, respectively, to engage a coupling member (not shown) and a threaded locking member (not shown) within the cartridge housing 2902. The cartridge 2900 also can include a cap 2908, and the cartridge housing 2902 also can include a rim 2909. The cartridge 2900 and the cartridge housing 2902 can be sized such that rotating the cartridge 2900 into the cartridge housing 2902 can cause the coupling members of the cartridge 2900 and the cartridge housing 2902 to engage. When the cap 2908 contacts the rim 2909, the cartridge 2900 can have a desired position within the cartridge housing 2902.

FIG. 25 shows a cartridge 3000 and a cartridge housing 3002. The cartridge 3000 can include a tip portion 3004 having a coupling member (not shown) and a threaded locking member 3006 configured, respectively, to engage a coupling member (not shown) and a threaded locking member (not shown) within the cartridge housing 3002. The cartridge 3000 also can include a gripping portion 3008 at an end of the cartridge 3000 opposite to an end including the tip portion 3004. The cartridge housing 3002 can include a rim 3010, and the cartridge 3000 can be generally independent of the rim 3010. Rotating the cartridge 3000 into the cartridge housing 3002 can cause the coupling members of the cartridge 3000 and the cartridge housing 3002 to engage.

The coupling member 2806 of the cartridge 2800 of FIG. 23 and the coupling members (not shown) of the cartridges 2700, 2900, 3000 of FIGS. 22, 24, and 25 can include membranes, check valves, or other suitable structures. With reference to FIGS. 22-25, the coupling members (not shown) at or near the cartridge housings 2702, 2802, 2902, 3002 can include various suitable structures configured to cooperate with the coupling member 2806 of the cartridge 2800 of FIG. 23 and the coupling members (not shown) of the cartridges 2700, 2900, 3000 of FIGS. 22, 24, and 25 to open fluidic connections. Examples of such structures include the first coupling member 1806 of FIGS. 13A-13B and the coupling members 1900a-c of FIGS. 14A-14C.

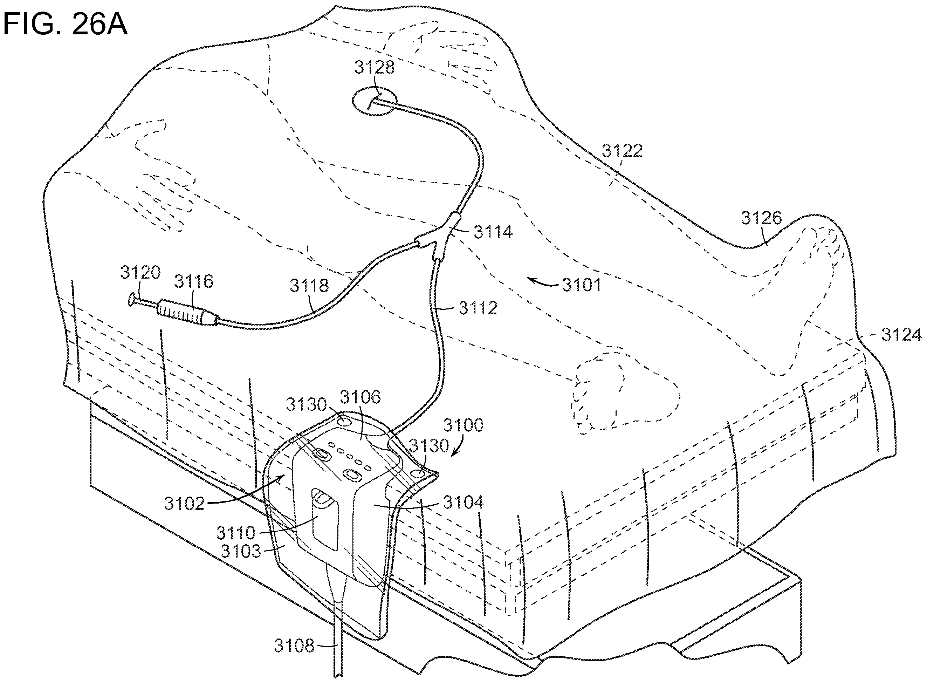

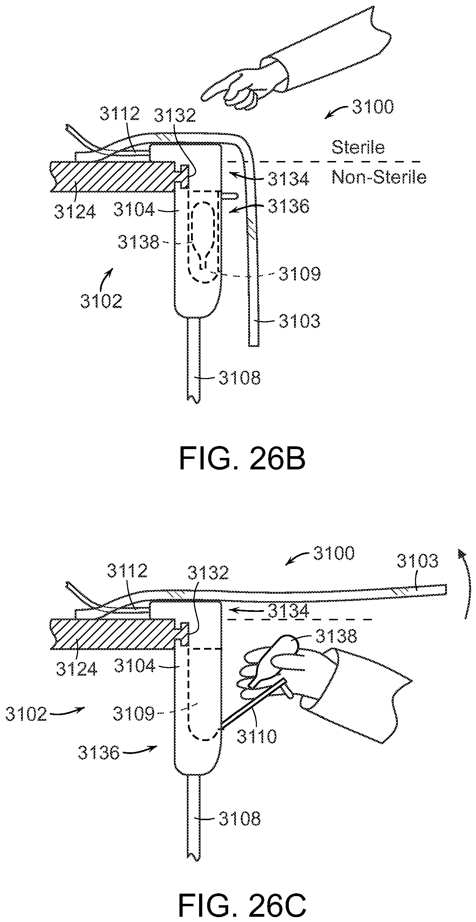

FIG. 26A is a perspective view illustrating a console assembly 3100 and portions of a cryotherapeutic device 3101. FIGS. 26B-26C are partially schematic side views of the console assembly 3100. The console assembly 3100 can include a console 3102 and a drape 3103 configured to form a sterile barrier over the console 3102 during a treatment procedure. The console 3102 can include a primary housing 3104 having a user interface 3106. The console assembly 3100 can include a power cord 3108 connected to the primary housing 3104. The primary housing 3104 can include a cartridge housing 3109 (FIGS. 26B-26C) and a hatch 3110, and the cartridge housing 3109 can be behind the hatch 3110. As shown in FIG. 26A, the cryotherapeutic device 3100 can include an umbilical cord 3112 having a hub 3114, a syringe 3116, and an umbilical branch 3118 extending between the hub 3114 and the syringe 3116. The syringe 3116 can include a plunger 3120 and can be fluidly connected to a balloon (not shown) within the vasculature via lumens along the umbilical branch 3118, the umbilical cord 3112, and an elongated shaft (not shown) of the cryotherapeutic device 3101. Pulling the plunger 3120 can cause the balloon to deflate. FIG. 26A also shows a patient 3122 on a surgical table 3124 and under a surgical sheet 3126 including an entry slit 3128 through which the umbilical cord 3112 can extend. The primary housing 3104 can be positioned over the surgical sheet 3126 and under the drape 3103. The console assembly 3100 can include drape connectors 3130 (e.g., buttons, snaps, or magnets) configured to connect the drape 3103 to the surgical sheet 3126 and/or the surgical table 3124.

As shown in FIGS. 26B-26C, the surgical table 3124 can include a flanged rail 3132 and the primary housing 3104 can be configured to attach to the flanged rail 3132. When attached to the flanged rail 3132, the primary housing 3104 can include an upper portion 3134 and a lower portion 3136. In other embodiments, the primary housing 3104 can have a different edge-mounting configuration. With reference again to FIGS. 26B-26C, the upper portion 3134 can include the user interface 3106 (FIG. 26A) and the lower portion 3136 can include the cartridge housing 3109. With reference to FIGS. 26A-26B, the user interface 3106 can be viewed and/or operated from the sterile field (e.g., by sterile personnel) through the drape 3103. The drape 3103 can be transparent or can include a window (not shown) to facilitate viewing and/or operating the user interface 3106. As shown in FIG. 26C, the console assembly 3100 can include a cartridge 3138 configured to be loaded into the cartridge housing 3109 outside the sterile field (e.g., by non-sterile personnel) under the drape 3103. Loading the cartridge 3138 can include lifting the drape 3103. In some embodiments, the console 3102 can be used without the drape 3103. For example, the console 3102 can be sterile or the user interface 3106 (FIG. 26A) can be sterile and the lower portion 3136 can be non-sterile.

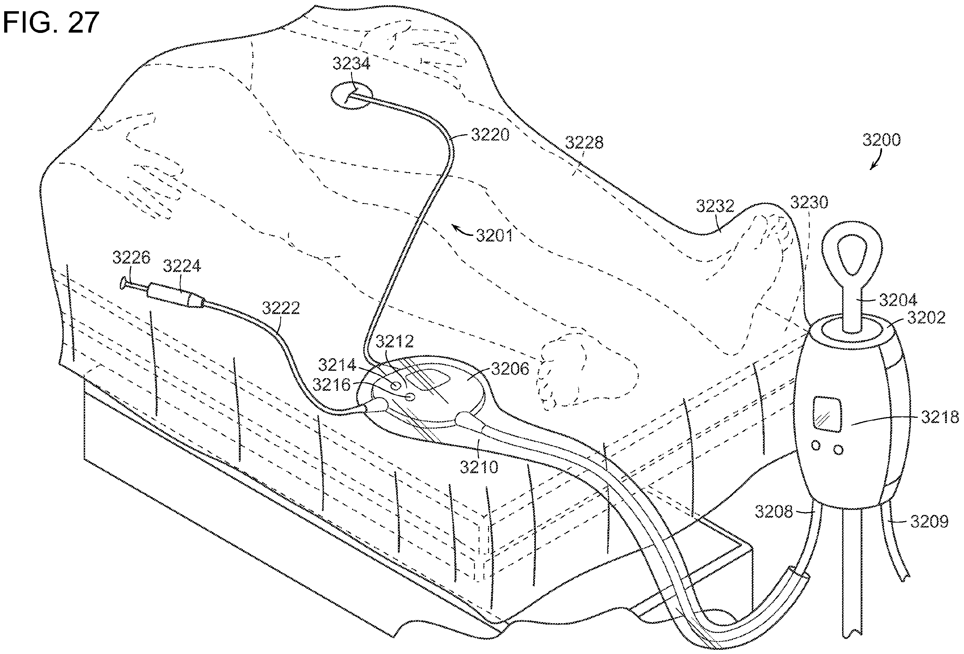

FIG. 27 is a perspective view illustrating a console assembly 3200 and a portion of a cryotherapeutic device 3201. The console assembly 3200 can include a primary housing 3202, a pole 3204 (e.g., a rolling I.V. pole), a hub 3206, and a cord 3208 extending between the primary housing 3202 and the hub 3206. In some embodiments, the primary housing 3202 can be located on the pole 3204 outside the sterile field during a treatment procedure. The console assembly 3200 can also include a power cable 3209 connected to the primary housing 3202 and a bag 3210 extending around the hub 3206 and a portion of the cord 3208. The bag 3210 can be configured to form a sterile barrier around the hub 3206 and the portion of the cord 3208. The primary housing 3202 can include a cartridge housing (not shown). Reloading a cartridge (not shown) can be a non-sterile operation (e.g., performed by non-sterile personnel). In some embodiments, the cord 3208 can be insulated to reduce warming of refrigerant passing from the primary housing 3202 to the hub 3206. The primary housing 3202 can also be replaced with a canister.

With reference to FIG. 27, the hub 3206 can include a first user interface 3212 having an initiation button 3214 and a termination button 3216 configured, respectively, to initiate and terminate cryogenic cooling within the vasculature. The primary housing 3202 can include a second user interface 3218 having the same or different operational and/or display features as the first user interface 3212. In some embodiments, a data connection (not shown) can extend between the hub 3206 and the primary housing 3202 along the cord 3208. As shown in FIG. 27, the cryotherapeutic device 3201 can include an umbilical cord 3220 extending from the hub 3206. The console assembly 3200 can include an umbilical branch 3222 and a syringe 3224 and the umbilical branch 3222 can extend between the hub 3206 and the syringe 3224. The bag 3210 can include sterile-barrier portals (not shown). The umbilical cord 3220 and the umbilical branch 3222 can connect to the hub 3206 through the sterile-barrier portals. The syringe 3224 can include a plunger 3226 and can be fluidly connected to a balloon (not shown) within the vasculature via lumens along the umbilical branch 3222, the umbilical cord 3220, and an elongated shaft (not shown) of the cryotherapeutic device 3201. Pulling the plunger 3226 can cause the balloon to deflate. FIG. 27 also shows a patient 3228 on a surgical table 3230 and under a surgical sheet 3232 including an entry slit 3234 through which the umbilical cord 3220 can extend.

The conceptual division between a console and a cryotherapeutic device associated with the console can vary in cryotherapeutic systems configured in accordance with embodiments of the present technology. For example, umbilical cords and/or hubs can be portions of a console, portions of a cryotherapeutic device, or portions of another related cryotherapeutic-system component. Cryotherapeutic-system components configured in accordance with several embodiments of the present technology can include various suitable cords and connectors (e.g., fluidic and/or electric) allowing for operable connection within cryotherapeutic systems. For example, consoles configured in accordance with embodiments of the present technology can include cords and/or connectors compatible with a variety of suitable cryotherapeutic devices and/or intervening cryotherapeutic-system components. Such cords and/or connectors can facilitate interoperability of cryotherapeutic-system components (e.g., interoperability between disposable and non-disposable cryotherapeutic-system components), customization of cryotherapeutic systems for certain cryotherapeutic applications, and bridging across sterile barriers, among other objectives.

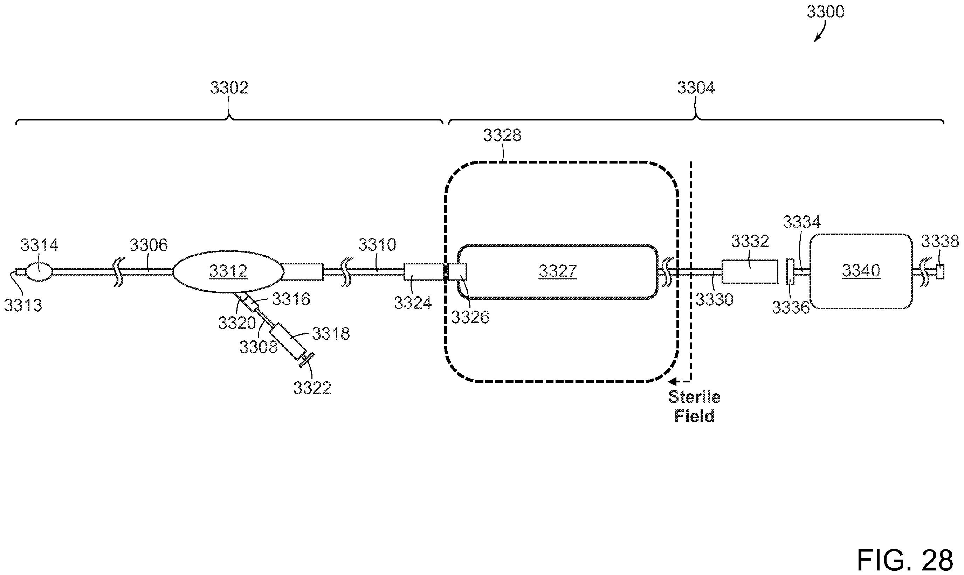

FIG. 28 is a partially schematic view illustrating a cryotherapeutic system 3300 illustrating several examples of interrelationships between cryotherapeutic-system components. The cryotherapeutic system 3300 can include a cryotherapeutic device 3302 and a console assembly 3304. The cryotherapeutic device 3302 can include an elongated shaft 3306 configured to be at least partially within the vasculature during a treatment procedure, an umbilical branch 3308, an umbilical cord 3310, and a hub 3312 at an intersection between the shaft 3306, the umbilical branch 3308, and the umbilical cord 3310. The shaft 3306 can include a distal tip 3313 and a balloon 3314. The balloon 3314 can be a primary balloon configured to generate or deliver therapeutically effective cryogenic neuromodulation of renal nerves. For example, the balloon 3314 can be a portion of an applicator (not separately identified in FIG. 28) of a cooling assembly (not separately identified in FIG. 28) having a delivery state and a deployed state. Furthermore, the balloon 3314 can be at least partially collapsed in the delivery state and expanded in the deployed state. In some embodiments, a length between the distal tip 3313 and the hub 3312 can be, for example, between about 60 cm and about 105 cm (e.g., between about 80 cm and about 85 cm).

The umbilical branch 3308 can include a first umbilical-branch connector 3316 at one end and a syringe 3318 at an opposite end. The hub 3312 can include a second umbilical-branch connector 3320 configured to operably connect to the first umbilical-branch connector 3316. The syringe 3318 can include a plunger 3322 and can be fluidly connected to the balloon 3314 within the vasculature via lumens along the umbilical branch 3308 and the shaft 3306. Pulling the plunger 3322 can cause the balloon 3314 to deflate. In other embodiments, additional and/or different umbilical branches can be connected to the shaft 3306, the umbilical cord 3310, and/or the hub 3312. These umbilical branches can include manual and/or automatic supply and/or evacuation mechanisms (e.g., syringes and/or actuated valves). In some embodiments, an umbilical branch can be configured to facilitate inflation or evacuation of a secondary balloon configured to be within the vasculature. A secondary balloon can be configured, for example, to facilitate a cooling pattern (e.g. a partially circumferential cooling pattern), to facilitate sizing of an applicator, and/or to facilitate a desired level of vessel occlusion. Umbilical branches also can be configured to deliver and/or withdraw drugs (e.g., pain relief drugs) and/or contrast agents.

The umbilical cord 3310 can be integrally connected to the hub 3312 at one end and can include a first umbilical-cord connector 3324 at an opposite end. A length of the umbilical cord 3310 between the hub 3312 and the first umbilical-cord connector 3324 can be, for example, between about 4 feet and about 10 feet (e.g., between about 6 feet and about 8 feet). The console assembly 3304 can include a second umbilical-cord connector 3326 configured to operably connect to the first umbilical-cord connector 3324. As shown in FIG. 28, the console assembly 3304 can include a primary housing 3327 and a bag 3328 configured to form a sterile barrier around the primary housing 3327. The first and second umbilical-cord connectors 3324, 3326 can be configured to bridge across a sterile barrier formed by the bag 3328. The console assembly 3304 can further include a cartridge (not shown), a user interface (not shown), and a power cord 3330 having a first power-cord connector 3332 at an end opposite to an end integrally connected to the console assembly 3304. The bag 3328 can extend around a portion of the power cord 3330, and the first power-cord connector 3332 can be outside the sterile field. A length of the power cord 3330 between the console assembly 3304 and the first power-cord connector 3332 can be, for example, between about 6 feet and about 10 feet (e.g., about 8 feet). The console assembly 3304 can also include an electrical adapter cord 3334 having a second power-cord connector 3336, a plug 3338, and an electrical adapter 3340 (e.g., a transformer) between the second power-cord connector 3336 and the plug 3338. The first and second power-cord connectors 3332, 3336 can be operably connectable, and the plug 3338 can be configured to connect to a standard wall receptacle. A length of the electrical adapter cord 3334 between the second power-cord connector 3336 and the plug 3338 can be, for example, between about 6 feet and about 10 feet (e.g., about 8 feet).

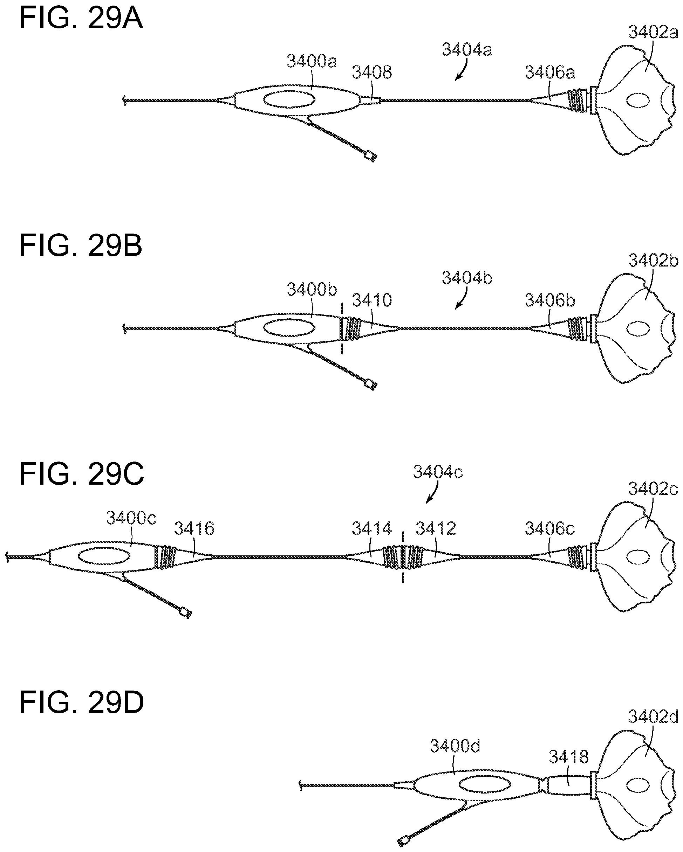

FIGS. 29A-29D are plan views of hubs 3400 (individually identified as 3400a-d) and primary housings 3402 (individually identified as 3402a-d) illustrating several examples of umbilical configurations. FIGS. 29A-29C also show umbilical cords 3404 (individually identified as 3404a-c) extending between the hubs 3400a-c and the primary housings 3402a-c. The umbilical cords 3404a-c can include proximal connectors 3406 (individually identified as 3406a-c) operably connected to the primary housings 3402a-c. As shown in FIG. 29A, the umbilical cord 3404a can include an integral distal connection 3408 operably connected to the hub 3400a. As shown in FIG. 29B, the umbilical cord 3404b can include a distal connector 3410 operably connected the hub 3400b. As shown in FIG. 29C, the umbilical cord 3404c can include a first intermediate connector 3412, a second intermediate connector 3414 operably connected to the first intermediate connector 3412, and a distal connector 3416 operably connected to the hub 3400c. FIG. 29D shows an integral umbilical connection 3418 between the hub 3400d and the primary housing 3402d instead of an umbilical cord.

Similar to the umbilical cord 3404c of FIG. 29C, umbilical cords configured in accordance with embodiments of the present technology can include multiple segments (e.g., to allow customization of length and/or bridging across sterile barriers). The hubs 3400a-c and the umbilical cords 3404a-c of FIG. 29A-29C can be disposable and/or provided in sterile packages (not shown). Similarly, the hub 3400d and the integral umbilical connection 3418 of FIG. 29D can be disposable and/or provided in a sterile package (not shown). With reference to FIGS. 29B-29C, in some embodiments, the hubs 3400b, 3400c can be non-disposable and the umbilical cords 3404b, 3404c can be disposable. In these and other embodiments, the distal connectors 3410, 3416 and the first and second intermediate connectors 3412, 3414 can be sterile, which can allow the hubs 3400b, 3400c to be disconnected and reconnected without breaking a sterile barrier around the primary housings 3402b, 3402c.

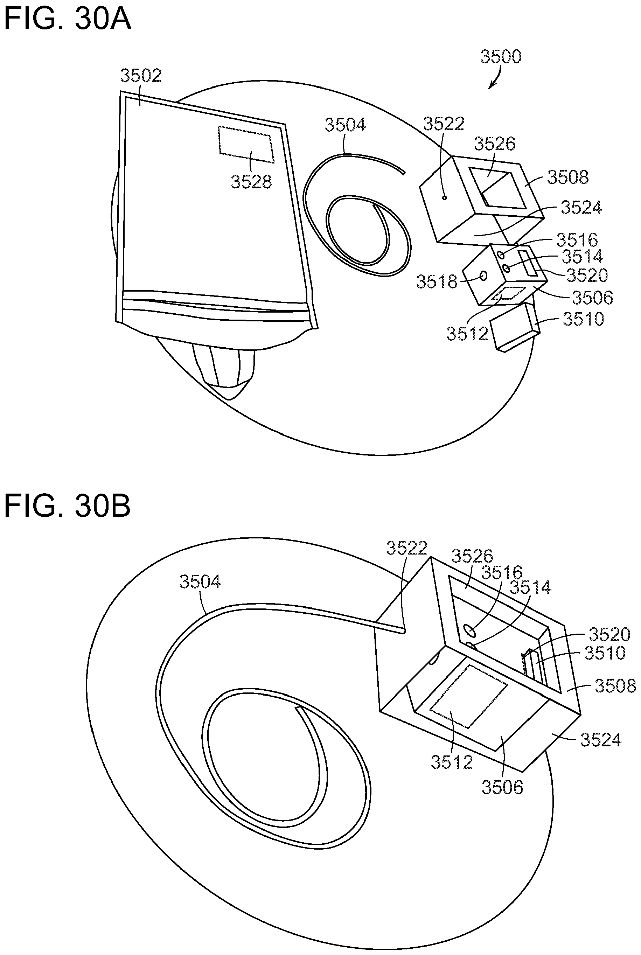

FIGS. 30A-30B are perspective views illustrating a kit 3500 including a bag 3502, a cryotherapeutic device 3504, a console 3506, a shell 3508, and a pack 3510. The console 3506, the shell 3508, and the pack 3510 can be generally shaped as rectangular solids with the console 3506 sized to fit within the shell 3508. In other embodiments, the console 3506, the shell 3508, and the pack 3510 can have other suitable shapes and sizes. The console 3506 can include a user interface (not separately identified) having a display 3512, an initiation button 3514, and a termination button 3516. The console 3506 can include a first connection port 3518 configured for connection to the cryotherapeutic device 3504, and a second connection port 3520 configured for connection to the pack 3510. The shell 3508 can include a sterile portal 3522, a first window 3524, and a second window 3526. Prior to a treatment procedure, the pack 3510 can be connected to the second connection port 3520. The connected console 3506 and pack 3510 can then be placed into the shell 3508 before being introduced into a sterile field. After connection to the console 3506, a portion of the pack 3510 can remain protruding from the console 3506 to facilitate removal and replacement. In some embodiments, the shell 3508 can act as a protective skin and can be waterproof.

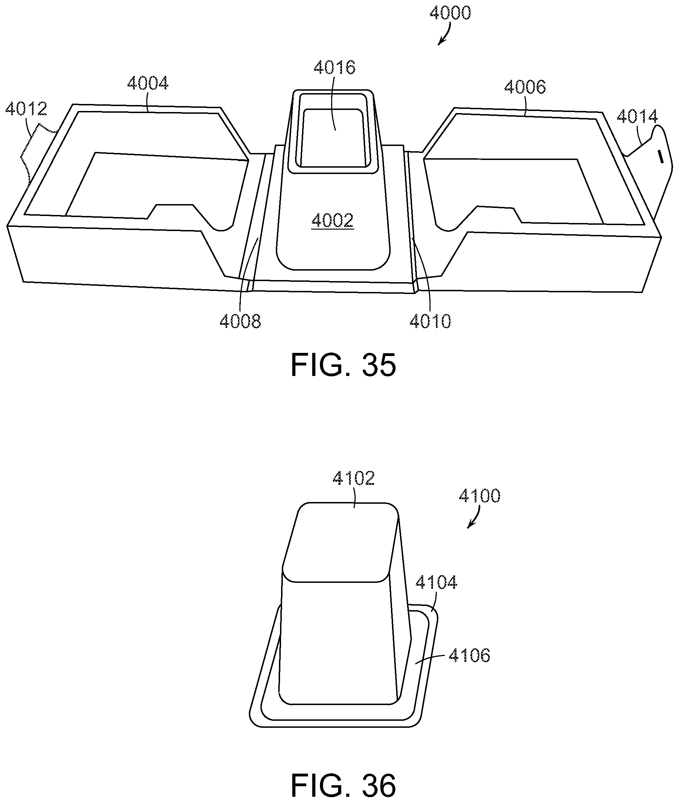

The cryotherapeutic device 3504, the shell 3508, and the pack 3510 can be supplied sterile in the bag 3502, which can be opened, for example, during a treatment procedure or during staging prior to a treatment procedure. Accordingly, the cryotherapeutic device 3504, the shell 3508, and the pack 3510 can be configured for exposure within the sterile field during a treatment procedure. The console 3506 can be configured to be non-sterile during a treatment procedure. In other embodiments, the console 3506 can be configured for sterilization between treatment procedures. After sterilization, the console 3506 can be sealed in a container (not shown) prior to use in subsequent treatment procedures. Examples of suitable containers for this use are described, for example, below with reference to FIGS. 35-36. In some embodiments, more than one cryotherapeutic device 3504 can be included in the kit 3500. For example, the kit 3500 can include a plurality of cryotherapeutic devices 3504 having different properties (e.g., shaft lengths, shaft diameters, balloon lengths, or balloon diameters) to accommodate the anatomies of different patients. In other embodiments, multiple kits 3500 can individually include single cryotherapeutic devices 3504 having different properties. Such kits 3500 can be labeled (e.g., with sizes) according to the properties of the included cryotherapeutic devices 3504.

As shown in FIG. 30B, when the console 3506 is within the shell 3508, the cryotherapeutic device 3504 can extend through the sterile portal 3522 and connect to the first connection port 3518 of the console 3506 within the shell 3508. In some embodiments, the cryotherapeutic device 3504 can be attached to the shell 3508, such as via interlocking members (not shown) on the cryotherapeutic device 3504 and the shell 3508. This can be useful, for example, to prevent the cryotherapeutic device 3504 from becoming disconnected from the console 3506 during a treatment procedure. The display 3512 can be visible through the first window 3524 of the shell 3508. In some embodiments, the first window 3524 can be configured to open to allow the console 3506 to be moved into the shell 3508. For example, the first window 3524 can be at least a portion of a wall assembly (not shown) removably connectable to the shell 3508. The second window 3526 can be flexible to allow operation of the initiation and termination buttons 3514, 3516 when the console 3506 is within the shell 3508. In other embodiments, the second window 3526 can include a deformable membrane, as described, for example, above with reference to FIG. 18.

In some embodiments, the console 3506 can be a durable component configured for indefinite use without the need for replacement or refurbishment. In contrast, the cryotherapeutic device 3504, the shell 3508, and the pack 3510 can be configured for use in a limited number of treatment procedures prior to disposal or refurbishment. For example, the cryotherapeutic device 3504, the shell 3508, and the pack 3510 can be disposable or refurbishable after use on a single patient. Disposal or refurbishment can occur at the site of the treatment procedure or at a different site. In some embodiments, the bag 3502 can be configured to be reused as a container for shipping one or more used components of the kit 3500 to a location equipped for specialized disposal, recycling, or refurbishment of the components. For example, the bag 3502 can include a shipping label 3528 addressed to such a location. The pack 3510 can include a cartridge (not shown), a battery (not shown), and an electronics package (not shown) (e.g., including a memory device). These features and other pack configurations are described, for example, above with reference to FIG. 7B. Refurbishing the pack 3510 can include sterilization, recharging the cartridge, recharging the battery, retrieving data from the memory device, clearing the memory device, loading information (e.g., a date of refurbishment, a new expiration date for the pack 3510, etc.) onto the memory device, other suitable acts, or any combination thereof. In some embodiments, multiple packs 3510 can be used to complete multiple treatment procedures. For example, multiple packs 3510 can be included in the kit 3500.

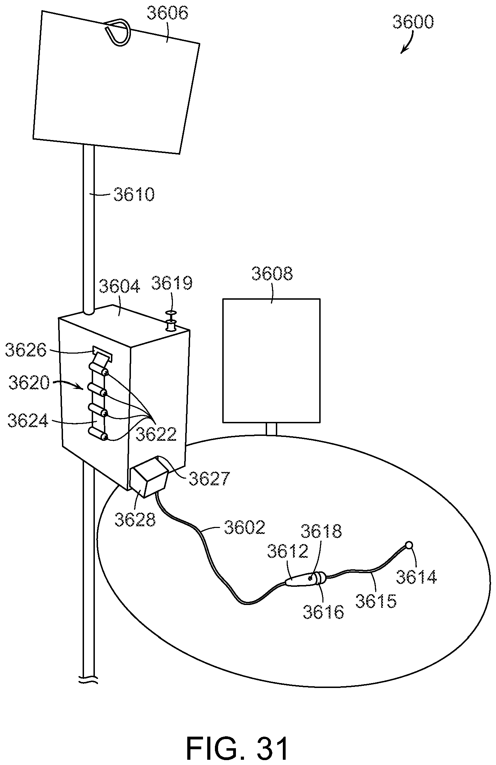

FIG. 31 is a perspective view illustrating a cryotherapeutic system 3600 that can include a cryotherapeutic device 3602, a console 3604, a first display 3606, and a second display 3608. As shown in FIG. 31, the cryotherapeutic system 3600 can be used during a treatment procedure with the console 3604 and the first display 3606 mounted on an I.V. pole 3610 outside the sterile field. For example, the console 3604 and the first display 3606 can include adapters (e.g., clamps) configured for connection to a standard I.V. pole. The first display 3606 can be connected wirelessly (e.g., via Wi-Fi) to the console 3604 and can be mounted higher on the I.V. pole 3610 than the console 3604 for enhanced visibility. The second display 3608 can also be connected wirelessly (e.g., via Wi-Fi) to the console 3604. In other embodiments, the first display 3606 and/or the second display 3608 can have wired connections to the console 3604. As shown in FIG. 31, the second display 3608 can be at approximately the same level as the console 3604, but spaced apart from the console 3604.

The cryotherapeutic device 3602 can be connected to the console 3604 and can include a handle 3612, a cooling assembly 3614, and an elongated shaft 3615 extending between the handle 3612 and the cooling assembly 3614. The handle 3612 can include an indicator 3616 (e.g., a red light, an audio indicator, etc.) configured, for example, to indicate when a portion of the cooling assembly 3614 is cryogenically adhered to a vessel during a treatment procedure. When this occurs, it can be useful to reduce movement of the cryotherapeutic device 3602 so as not to strain the connection between the cooling assembly 3614 and the vessel. Locating the indicator 3616 on the handle 3612 can increase the likelihood that an operator will notice the indicator 3616 prior to moving the cryotherapeutic device 3602. In some embodiments, one or more additional or alternative indicators can be included on the console 3604, on the first display 3606, and/or on the second display 3608. The handle 3612 can also include a first abort button 3618, which can be configured, for example, to stop the flow of refrigerant to the cooling assembly 3614 or to otherwise stop, slow, or reverse cooling by the cooling assembly 3614. As shown in FIG. 31, the console 3604 can include a second abort button 3619 that can have the same functionality as the first abort button 3618. In some embodiments, the second abort button 3619 or a third abort button (not shown) can be included on a satellite (not shown) between the handle 3612 and the console 3604.

The cryotherapeutic system 3600 can include elements that are removably connectable to the console 3604 for supplying power, refrigerant, data, or a combination thereof to the console 3604. For example, the cryotherapeutic system 3600 can include a ribbon 3620 having multiple cartridges 3622 and a webbing 3624, with the cartridges 3622 detachably or integrally connected to the webbing 3624. The console 3604 can include a first inlet port 3626 configured to receive the ribbon 3620. The console 3604 can also include a feeder (not shown) configured to advance the ribbon 3620 as refrigerant in the cartridges 3622 is consumed or in response to another factor. For example, the feeder can be configured to advance the ribbon 3620 to load a new cartridge 3622 in response to a signal from a user interface (not shown), in response to a signal indicating depletion of a loaded cartridge 3622, in response to a signal indicating a duration or volume of refrigerant flow from a loaded cartridge 3622, and/or each time a treatment procedure is completed regardless of a level of depletion of a loaded cartridge 3622. In some embodiments, portions of the ribbon 3620 including consumed cartridges 3622 can exit the console 3604 through an outlet port (not shown) or accumulate within the console 3604 (e.g., within a chamber (not shown) accessible during maintenance of the console 3604).

The console 3604 can also include a second inlet port 3627 and the cryotherapeutic system 3600 can include a pack 3628 removably connectable to the console 3604 at the second inlet port 3627. When used in conjunction with the ribbon 3620, the pack 3628 can supply power and/or information. For example, the pack 3628 can include a battery (not shown) and/or a memory device (not shown). In some embodiments, the pack 3628 can include a cartridge and substitute for or provide a backup for the ribbon 3620. The pack 3628 can also have other configurations. In some embodiments, the console 3604 can include a power cord (not shown) for connection to an external power source. In these embodiment and other embodiments, the pack 3628 can be eliminated or can serve as a backup or alternative power source.

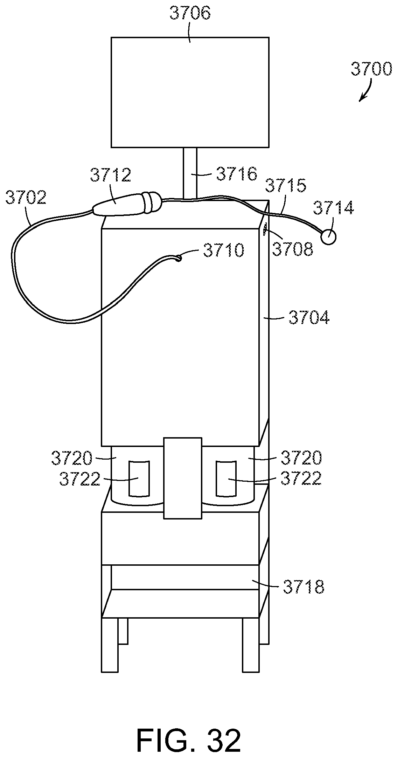

FIG. 32 is a perspective view illustrating a cryotherapeutic system 3700 that can include a cryotherapeutic device 3702, a console 3704, and a display 3706. As shown in FIG. 32, the console 3704 can include a data port 3708 (e.g., a USB connector) for receiving data (e.g., patient data) and/or transmitting data (e.g., procedure data). In some embodiments, the console 3704 can include more than one power supply (e.g., a rechargeable battery (not shown)), and a power cord (not shown). The power cord can be retractably connected to the console 3704 to reduce tangling. The console 3704 can include a connector 3710 and the cryotherapeutic device 3702 can be attached to the console 3704 at the connector 3710. In some embodiments, the cryotherapeutic device 3702 can be similar to the cryotherapeutic device 3602 of FIG. 31. The cryotherapeutic device 3702 can include a handle 3712 having controls (e.g., pre-inflate, on, off, abort, other controls, or a combination thereof), a cooling assembly 3714, and an elongated shaft 3715 extending between the handle 3712 and the cooling assembly 3714. In some embodiments, all or most controls for normal operation of the cryotherapeutic device 3702 can be located on the handle 3712.

The cryotherapeutic system 3700 can include an adjustable-height mount 3716 between the console 3704 and the display 3706. The display 3706 can also be detachable from the console 3704 for mounting at different locations and can be connected to the console 3704 wirelessly (e.g., via Wi-Fi) or through a wired connector. In some embodiments, during a treatment procedure, the display 3706 can be positioned in an operator's field of view and/or proximate a fluoroscopy screen. The console 3704 can be a self-supporting cabinet with a height, for example, from about 2 feet to about 3 feet. In some embodiments, the console 3704 can include wheels (not shown) that allow the console 3704 to be moved over power cords or other irregularities on the floor of a treatment area with reduced disruption. For example, the wheels can be resiliently mounted (e.g., spring loaded). In some embodiments, the wheels can be knobbled or made up partially or entirely of brushes. The console 3704 can have a square footprint or another suitable footprint and can have features to facilitate convenient placement and/or storage. For example, the back of the console 3704 can include a cutout (not shown) configured to fit over a skirting board and facilitate placement of the console 3704 flush or nearly flush against a wall. As shown in FIG. 32, the console 3704 can include a storage space 3718 sized to hold consumable portions of the cryotherapeutic system 3700 (e.g., sterile kits containing disposable cryotherapeutic devices). Bar code, radiofrequency, or other suitable identification systems can be used to monitor stock levels of components within the storage space 3718.

The cryotherapeutic system 3700 can be used with two canisters 3720 and the console 3704 can be configured to hold both canisters 3720, allowing one canister 3720 to be refilled while the other remains in use. The canisters 3720 can have sufficient capacity for multiple treatment procedures. In some embodiments, the canisters 3720 can be a standard size used for medical applications. The console 3704 can include a valve (not shown) and a manual or automatic actuator (not shown) configured to change the supply of refrigerant to the connector 3710 from one canister 3720 to the other. As shown in FIG. 32, the canisters 3720 can include windows 3722 allowing internal portions of the canisters 3720 to be viewed. The internal portions of the canisters can include level indicators (not shown). In addition or alternatively, the console 3704 can include one or more sensors (not shown) configured to detect the level of refrigerant remaining in one or both of the canisters 3720. Examples of suitable sensors include weight sensors (e.g., scales operably connected to one or both of the canisters 3720), flow meters fluidly connected to one or both of the canisters 3720, and pressure sensors fluidly connected to one or both of the canisters 3720. In some embodiments, the cryotherapeutic system 3700 can include a tracking system for the canisters 3720. For example, the canisters 3720 can have identifiers (e.g., radiofrequency or e-ink identifiers), and the tracking system can associate the identifiers with canister data (e.g., a time or number of uses since the most recent recharging). In these and other embodiments, the console 3704 can be configured to send a notification (e.g., through an Internet connection) to a supplier when the level of refrigerant remaining in one or both of the canisters 3720 drops below a threshold value or range.

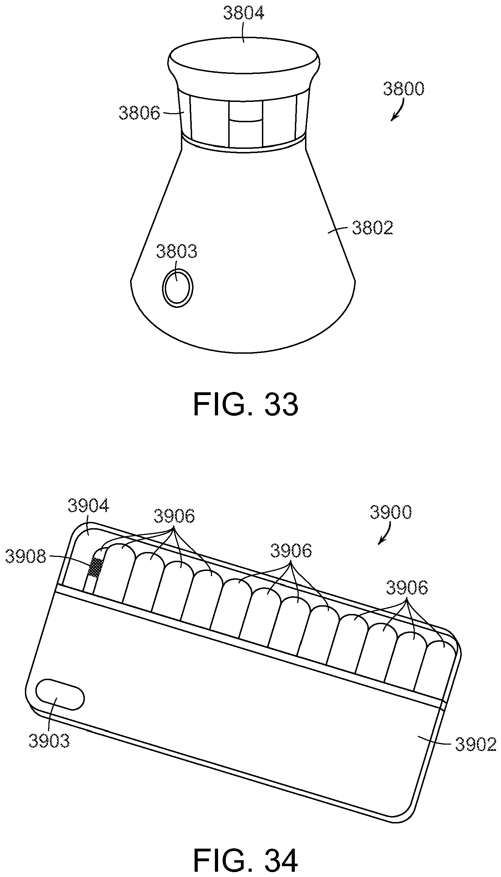

FIG. 33 is a perspective view illustrating a console 3800 that can include a body 3802 having an upwardly tapering truncated, conical shape and a connection port 3803 near the lowermost portion of the body 3802 for connection to a cryotherapeutic device (not shown). The console 3800 can include a lid 3804 and a neck 3806 between the body 3802 and the lid 3804. The console 3800 can be configured to hold a plurality of cartridges (not shown) (e.g., two, three, four, or a greater number of cartridges). For example, the lid 3804 can be detachable from the body 3802 to allow the cartridges to be loaded. The neck 3806 can be at least partially transparent allowing portions of the cartridges or indicators (not shown) corresponding to the cartridges to be viewed without detaching the lid 3804. In some embodiments, the cartridges can be selectively deployed through the neck 3806 (e.g., through actuators (not shown) on the neck 3806). The neck 3806 and/or the lid 3804 can also be rotatable to facilitate transition from one cartridge to another cartridge. In these and other embodiments, the body 3802 and the neck 3806 can include markings (not shown) and/or tactile engagement mechanisms indicating when proper alignment of a cartridge has been achieved to allow deployment of the cartridge. Deployment of the cartridge can include opening a fluidic connection with a passage (not shown) within the body 3802. As discussed above with reference to FIGS. 13A-13B, this can involve, for example, opening a check valve or puncturing a membrane of the cartridge.

FIG. 34 is a perspective view illustrating a console 3900 that can include a body 3902 generally shaped as a rectangular solid and a connection port 3903 near the lowermost portion of the body 3902 for connection to a cryotherapeutic device (not shown). The connection port 3903 can be oblong (or asymmetrical in some embodiments), which can facilitate alignment of an adapter (not shown) requiring a particular radial orientation. The console 3900 can also include a cap 3904 removably connectable to the body 3902. In some embodiments, the cap 3904 can be at least partially transparent. The console 3900 can include a plurality of cartridges 3906 that can be visible through the cap 3904. Each of the cartridges 3906 can include a coupling member (not shown) positioned within the body 3902. The body 3902 can include corresponding coupling members (not shown) aligned with the cartridges 3906. Opening the cap 3904 and pressing one or more of the cartridges 3906 toward the body 3902 can cause the pressed cartridges 3906 to become deployed. Cartridges 3906 that have not yet been deployed can be identifiable by their position within the console 3900. For example, deployed cartridges 3906 can be farther inset into the body 3902 than cartridges 3906 that have not yet been deployed. In some embodiments, the cartridges 3906 can be configured to be deployed in a particular order and the final cartridge 3906 can include a marking 3908 alerting an operator that replacement cartridges 3906 should be obtained. Multiple markings 3908 can also be used on the same or different cartridges 3906. For example, multiple markings 3908 can be used to indicate a desired order of deployment. In both the console 3800 of FIG. 33 and the console 3900 of FIG. 34, exhausted cartridges 3906 can remain in place or be withdrawn prior to deploying additional cartridges 3906.

FIG. 35 is a perspective view illustrating a shell 4000 that can be used, for example, to form a sterile barrier around the console 3900 of FIG. 34. As shown in FIG. 35, the shell 4000 can include a base 4002, a first wing 4004, and a second wing 4006. The base 4002 can be made, for example, from a rigid, opaque polymer. The first and second wings 4004, 4006 can be made, for example, from a rigid, transparent polymer. The shell 4000 can further include a first living hinge 4008 and a second living hinge 4010 that can, respectively, connect the first wing 4004 and the second wing 4006 to opposite sides of the base 4002. The first wing 4004 can include a first flap 4012 and the second wing 4006 can include a second flap 4014. The first and second flaps 4012, 4014 can be interlocking and configured to hold the shell 4000 together in a clamshell configuration. Alternatively or in addition, the first and second flaps 4012, 4014 can have other interlocking features. The base 4002 can include a recess 4016 shaped to receive the console 3900 of FIG. 34 and to hold the console 3900 in an upright configuration. In other embodiments, the base 4002 can have a different shape, such as a shape corresponding to another console configured in accordance with embodiments of the present technology. Furthermore, the first and second flaps 4012, 4014 can be shaped to conform relatively closely to the shape of a suitable console (e.g., the console 3900 of FIG. 34). In this way, the shell 4000 can help to protect the console during handling prior to use. In some embodiments, the shell 4000 can be configured to hold a console such that the console does not need to be removed from the base 4002 during use in a treatment procedure. For example, the base 4002 and the first and second wings 4004, 4006 can support and stabilize the console after the shell 4000 is opened and the console is placed in use.

FIG. 36 is a perspective view illustrating a shell 4100 that can be used, for example, to form a sterile barrier around the console 3900 of FIG. 34. As shown in FIG. 36, the shell 4100 can include a container 4102 and a sealing member 4104. In some embodiments, the container 4102 can be at least partially transparent. The container 4102 can have a rim 4106 configured to connect to the sealing member 4104 (e.g., with an adhesive or thermal bond). The console 3900 of FIG. 34 or another suitable console configured in accordance with embodiments of the present technology can be sealed within the container 4102 to maintain the console 3900 in a sterile condition. The sealing member 4104 can be peeled from the rim 4106 to access the console 3900 prior to use. In some embodiments, the console can be resealed within the container 4102 after sterilization between treatment procedures. Similar to the shell 4000 shown in FIG. 35, the shell 4100 can be shaped to conform relatively closely to the shape of a suitable console (e.g., the console 3900 of FIG. 34). In this way, the shell 3900 can help to protect the console during handling prior to use.

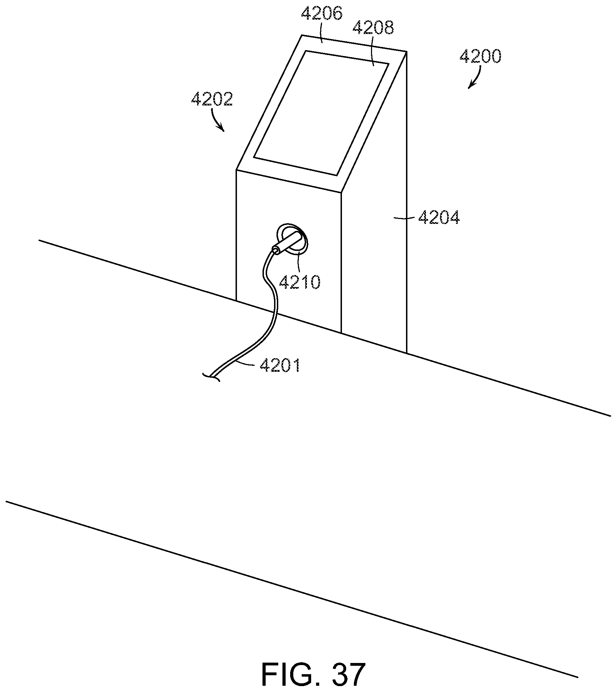

FIG. 37 is a perspective view illustrating a cryotherapeutic system 4200 that can include a cryotherapeutic device 4201 and a console 4202. The console 4202 can be self-supporting and can include a base (not shown) and a tower 4204 having an angled upper portion 4206 with a display 4208. The tower 4204 can also include a door (not shown) covering a compartment (not shown) configured to hold a canister (not shown). The cryotherapeutic system 4200 can include other features corresponding to use of relatively large-capacity canisters, such as those described, for example, above with reference to FIG. 32. In some embodiments, the tower 4204 can be attached to the base for enhanced stability. The base can include wheels (not shown) and a locking mechanism (not shown). As shown in FIG. 37, the tower 4204 can include an opening 4210 through which the cryotherapeutic device 4201 can be routed.

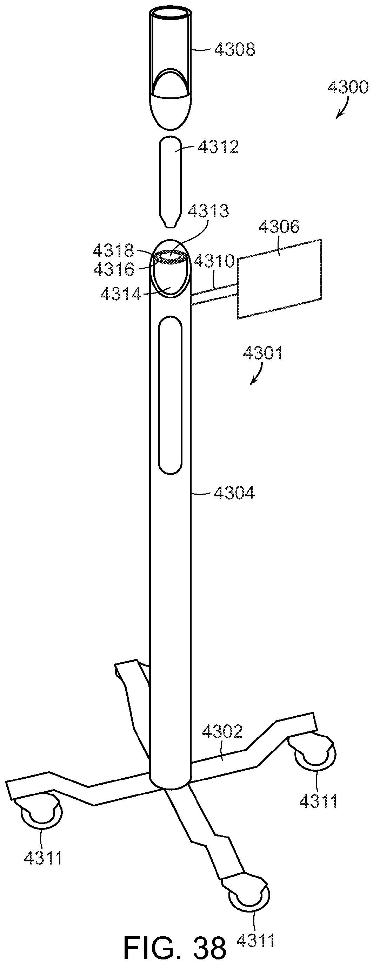

FIG. 38 is a partially exploded view illustrating a console assembly 4300 that can include a console 4301 and a base 4302. The console 4301 can include a pole 4304, a display 4306, and a cap 4308. The console 4300 can further include an arm 4310 extending between the display 4306 and the pole 4304. In some embodiments, the arm 4310 can be vertically adjustable relative to the pole 4304. Furthermore, the display 4306 can be tiltable relative to the arm 4310. The base 4302 can be self-supporting and include wheels 4311 for mobility. The console assembly 4300 can include a cartridge 4312 and the console 4301 can include an opening 4313 at the top of the pole 4304. The opening 4313 can be configured to receive the cartridge 4312 in a vertical orientation. In some embodiments, the cap 4308 can be removably connectable to the pole 4304. For example, the console 4301 can include a ledge 4314 at the top of the pole 4304 and the cap 4308 can be configured to rest on the ledge 4314. The bottom edge of the cap 4308 and the ledge 4314 can be shaped to cause the cap 4308 to have a specific radial alignment relative to the pole 4304. For example, the bottom edge of the cap 4308 and the ledge 4314 can be slanted or curvilinear. In some embodiments, the cap 4308 can be at least partially transparent (e.g., to facilitate viewing the presence or absence of the cartridge 4312).

The opening 4313 can extend into a cartridge housing (not shown) of the console 4301 and the cartridge housing can include a coupling member (not shown) configured to engage a coupling member (not shown) of the cartridge 4312. The console 4301 can include a connector (not shown) on the pole 4304 configured to connect to a cryotherapeutic device (not shown). The console 4301 can further include a supply line (not shown) extending between the connector and the coupling member of the cartridge housing. Engaging the coupling member of the cartridge 4312 and the coupling member of the cartridge housing can include, for example, pressing the cartridge 4312 into the opening 4313 or screwing the cartridge 4312 into the opening 4313. In some embodiments, the console 4301 can be configured to automatically load the cartridge 4312. For example, the console 4301 can include a motorized loading mechanism (not shown) configured to automatically lower the cartridge 4312 into the cartridge housing until the coupling member of the cartridge 4312 and the coupling member of the cartridge housing are fully engaged. Such a mechanism can include a gripper (not shown) configured to grip the cartridge 4312 and an actuator (not shown) (e.g., a solenoid actuator) configured to lower the cartridge 4312 into the opening 4313. Engaging the coupling member of the cartridge 4312 and the coupling member of the cartridge housing can facilitate consistent and reliable connection of the cartridge 4312, such as by controlling the coupling force and the alignment of the cartridge 4312. In some embodiments, loading the cartridge 4312 can include manually placing or securing (e.g., screwing) the cartridge 4312 into an initial position in the opening 4313 and then initiating the actuator. The console 4301 can also be configured to detect the presence of the cartridge 4312 and initiate the actuator automatically.

As shown in FIG. 38, the console 4301 can include an indicator loop 4316 at the top portion of the pole 4304 around the opening 4313. The indicator loop 4316 can include a plurality of light-emitting diodes 4318 configured to illuminate when the coupling member of the cartridge 4312 and the coupling member of the cartridge housing are fully engaged. In other embodiments, the console 4301 can include another visible, audible, or other indication that the cartridge 4312 is properly loaded. Moreover, the indicator loop 4316 can have another suitable shape or size. For example, the indicator loop 4316 can be replaced with a non-looped indicator. In some embodiments, the console 4301 can include control circuitry (not shown) connected to the indictor loop 4316 and configured to illuminate the light-emitting diodes 4318 in response to a detected condition (e.g., detected full engagement of the cartridge 4312, detected pressure of refrigerant within the supply line, or detected flow of refrigerant from the cartridge 4312). The indicator loop 4316 can be configured to display different colors corresponding to different levels of depletion of refrigerant within the cartridge 4312 (e.g., green for low depletion and red for high depletion).

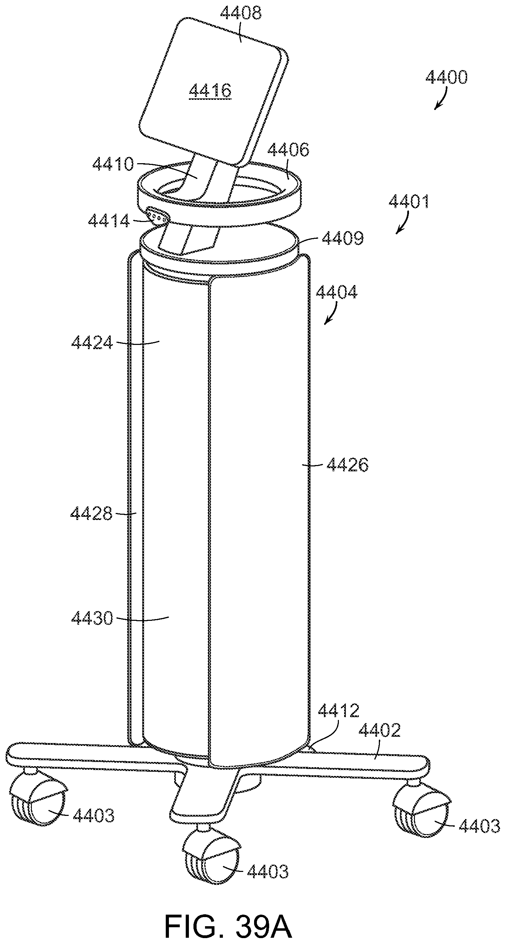

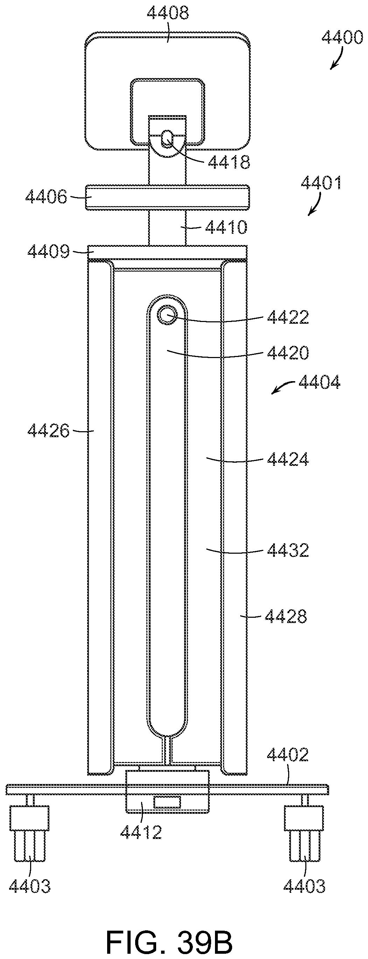

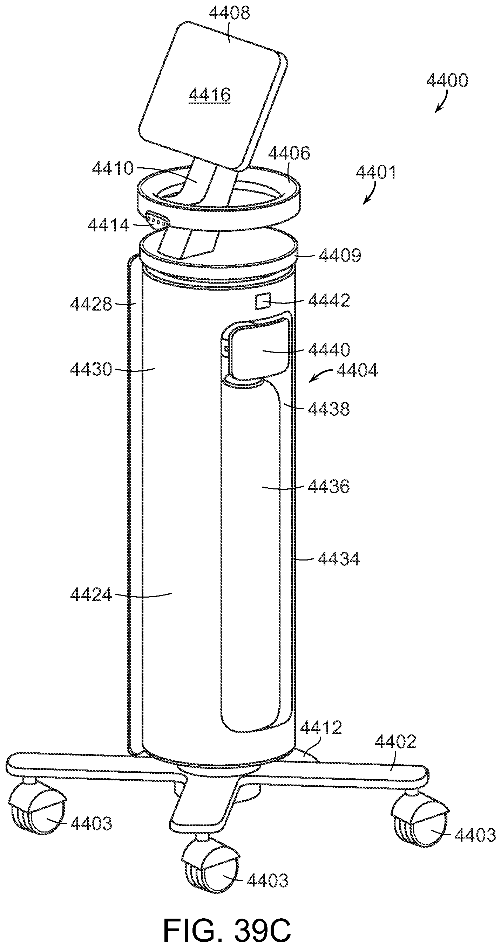

FIGS. 39A and 39C are perspective views and FIG. 39B is a profile view illustrating a console assembly 4400 that can include a console 4401 and a base 4402 having wheels 4403. The console 4401 can include a main housing 4404, a handle 4406, and a display 4408. The main housing 4404 can include an upper portion 4409, and the console 4401 can further include a neck 4410 extending between the upper portion 4409 and the display 4408. The handle 4406 can be attached to the neck 4410 between the upper portion 4409 and the display 4408. As shown in FIGS. 39A and 39C, the neck 4410 can extend at an angle between the main housing 4404 and the display 4408, which can cause the display 4408 to have a non-vertical viewing angle. The display 4408 can also be tiltable relative to the neck 4410. The main housing 4404 can be centrally supported on the base 4402, and the wheels 4403 can be laterally spaced apart from the main housing 4404. The lateral spacing of the wheels 4403 can improve the stability of the console 4401. A distance between wheels 4403 on generally opposite sides of the base 4402 can be at least about 30% of a distance between the upper portion 4409 and the floor (e.g., at least about 40% or at least about 50%). In some embodiments, the base 4402 can include an extender (not shown) configured to adjust the height of the console 4401 relative to the base 4402. As shown in FIG. 39B, the base 4402 can include a pedal 4412 configured to adjust the extender and/or to lock the wheels 4403. For example, the extender can be pneumatic and the pedal 4412 can be configured to increase or decrease the pressure in the extender to increase or decrease, respectively, the height of the extender. In some embodiments, the base 4402 can include more than one pedal 4412 (e.g., one pedal configured to increase the height of the extender, one pedal configured to decrease the height of the extender, and one pedal configured to lock the wheels 4403). The wheels 4403 can include individual locking mechanisms (not shown).

As shown in FIGS. 39A and 39C, the handle 4406 can be circular and can extend around the neck 4410. In some embodiments, the console 4401 can include a connector 4414 on the handle 4406 (e.g., below an active side 4416 of the display 4408). The connector 4414 can be configured for connection to a mating connector (not shown) of a cryotherapeutic device (not shown). The connector 4414 can be asymmetrical to facilitate connection to the mating connector in a particular orientation. Furthermore, the connector 4414 and/or the handle 4406 proximate the connector 4414 can include one or more lighting elements (not shown) configured to illuminate or change color (e.g., red to green) when a mating connector is properly (e.g., fully) connected. For example, connecting the mating connector can complete a circuit that causes the lighting elements to illuminate. In some embodiments, the lighting elements can be within the connector 4414 and the connector 4414 can be at least partially transparent to allow passage of light from the lighting elements when a mating connector is properly connected. In these and other embodiments, the connector 4414 can be protruding relative to the handle 4406 and act as an overall male connector with female sub-connectors. In other embodiments, the connector 4414 can have a different configuration.

As shown in FIG. 39B, the console 4401 can include a power switch 4418 at the top of the neck 4410 proximate a joint between the neck 4410 and the display 4408. The power switch 4418 can be configured to turn on the display 4408 and/or to otherwise prepare the console 4401 for operation. The main housing 4404 can include an elongated recess 4420 and a post 4422 at an upper portion of the recess 4420. The recess 4420 can be configured to hold an electrical cord (not shown) or another type of line (e.g., suction, refrigerant supply, refrigerant exhaust, etc.) with the cord or line looped around the post 4422. The main housing 4404 can include a core 4424, a first sliding door 4426, and a second sliding door 4428. The first and second sliding doors 4426, 4428 can be rotatable individually or jointly relative to the core 4424. For example, FIGS. 39A-39B show the main housing 4404 in a first configuration, with the first and second sliding doors 4426, 4428 rotated so that the core 4424 has an exposed forward portion 4430 (FIG. 39A) and an exposed rear portion 4432 (FIG. 39B). The forward portion 4430 can be generally below the active side 4416 of the display 4408 and the rear portion 4432 can be generally opposite to the forward portion 4430. FIG. 39C shows the main housing 4404 in a second configuration, with the second sliding door 4426 (FIG. 39A) rotated so that the core 4424 has an exposed side portion 4434.

The console assembly 4400 can be used with two canisters 4436 (one shown in FIG. 39C) and the main housing 4404 can include two canister housings 4438 (one shown in FIG. 39C) configured to hold the canisters 4436. As shown in FIG. 39C, one of the canisters 4436 and one of the canister housings 4438 can be at the side portion 4434. The other canister and the other canister housing can be at an opposite side (not shown) of the core 4424. Including two canisters 4436 can allow one to be replaced while the other remains in service. In other embodiments, the canister housings 4438 can have different locations in the main housing 4404. For example, the main housing 4404 can include two sections (not shown), each including one of the canister housings 4438, and one or both of the sections can be configured to rotate outward relative to a central axis of the console assembly 4400 to expose the canister housings 4438. For example, in these and other embodiments, the console 4401 can include a support pole (not shown) about which the sections can rotate. When the sections are rotated together, openings into the canister housings 4438 can meet within the main housing 4404.

With reference again to FIGS. 39A-39C, the console 4401 can include shields 4440 (one shown in FIG. 39C) at upper portions of the canister housings 4438. In some embodiments, the shields 4440 can be hingedly mounted to the main housing 4404, and lifting a lower portion of the shields 4440 can allow the canisters 4436 to be replaced. For example, lifting one of the shields 4440 can allow access to a coupling member (not shown) of the canister housing 4438 and a coupling member (not shown) of the canister 4436 within the canister housing 4438. In some embodiments, lifting the shield 4440 can automatically disengage the coupling members. In other embodiments, lifting the shield 4440 can provide access for manually disengaging the coupling members. As shown in FIG. 39C, the console 4401 can include capacity indicators 4442 (one shown in FIG. 39C) on the core 4424 above the canister housings 4438. The capacity indicators 4442 can be configured to indicate the amount of refrigerant remaining in the canisters 4436. In some embodiments, the capacity indicators 4442 can be configured to indicate a number of treatment procedures and/or cooling cycles that can be completed before replacing the canister 4436 or switching to the other canister 4436.

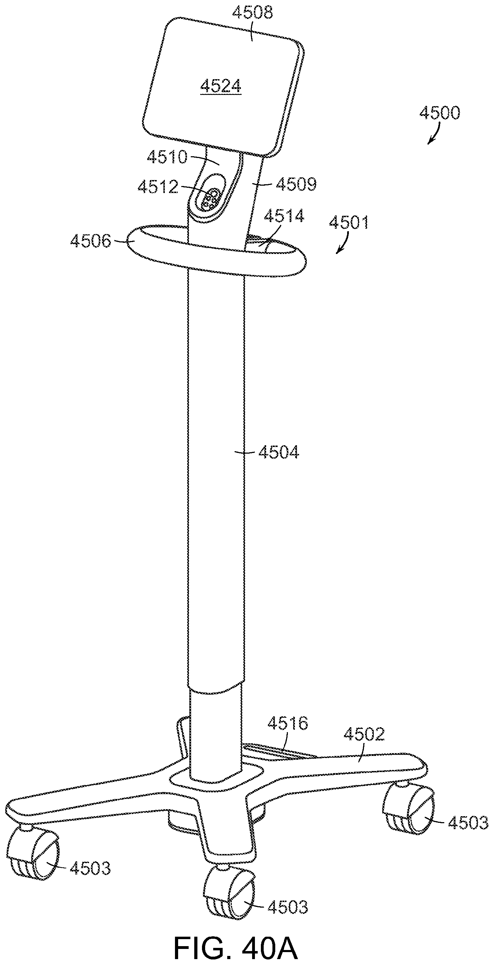

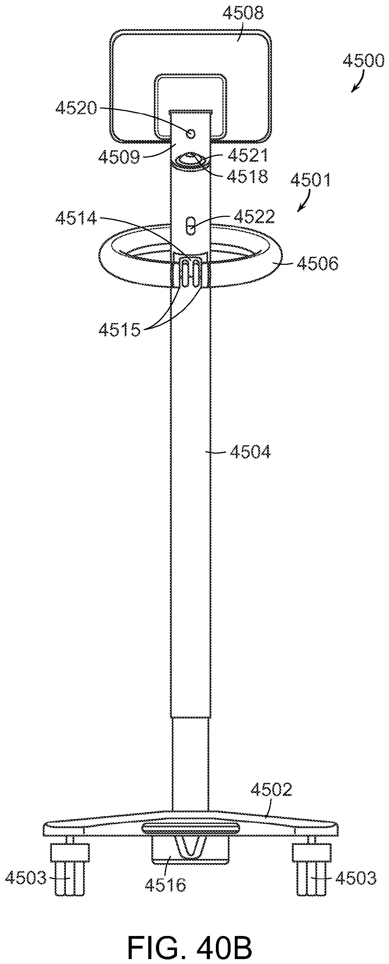

FIG. 40A is a perspective view and FIG. 40B is a profile view illustrating a console assembly 4500 that can include a console 4501 and a base 4502 having wheels 4503. The console assembly 4500 can be similar to the console assembly 4400 of FIGS. 39A-39C, but configured to hold a cartridge instead of canisters. Similar to the console 4401 of FIGS. 39A-39C, the console 4501 can include a body 4504, a handle 4506, and a display 4508. The body 4504 can include an upper portion 4509 having an angled upper surface 4510. The console 4501 can further include a connector 4512 at the angled upper surface 4510. The connector 4512 can be configured for connection to a cryotherapeutic device (not shown). In other embodiments, the connector 4512 can have a different position. For example, the body 4504 can include a recess (not shown) at a front side of the body 4504 and the connector 4512 can be at a top portion of the recess (e.g., at an angled top portion of the recess). The recess can be configured, for example, to hold the cryotherapeutic device when not in use. With reference again to FIGS. 40A-40B, the display can be connected (e.g., tiltably connected) to the upper portion 4509 above the connector 4512.

As shown in FIG. 40B, the console 4501 can include an arm 4514 extending between the body 4504 and the handle 4506. A cord or line (not shown) of the console 4501 can be configured to be looped around or over the arm 4514. In some embodiments, the arm 4514 can include one or more curved recesses 4515 configured to receive the cord or line. For example, each curved recess 4515 can be configured to receive a different cord or line to keep the different cords or lines separated. The handle 4506 can be circular and can extend around the body 4504 below the upper portion 4509. In some embodiments, the handle 4506 can be tiltably connected to the arm 4514 and/or the arm 4514 can be tiltably connected to the body 4504 to allow adjustment of the handle 4506. In other embodiments, the handle 4506 and the display 4508 can have different configurations. For example, the handle 4506 can extend laterally from one side of the body 4504 and the display 4508 can be connected to an opposite side of the body 4504. With reference again to FIGS. 40A-40B, the body 4504 can be centrally supported on the base 4502, and the wheels 4503 can be laterally spaced apart from the body 4504. The base 4502 can include a pedal 4516 configured to adjust the height of the body 4504 relative to the base 4502 and/or to lock the wheels 4503.

As shown in FIG. 40B, the console assembly 4500 can include a cartridge 4518, and the console 4501 can include a cartridge housing (not shown) at the upper portion 4509 of the body 4504. The console 4501 can further include a load/eject button 4520 above an opening (not shown) of the cartridge housing as well as an indicator loop 4521 around the opening. The load/eject button 4520 can be configured to initiate an automatic (e.g., actuated and/or motorized) loading or unloading sequence. Such a sequence can include, for example, opening or closing a fluidic connection between a supply line (not shown) of the console 4501 and the cartridge 4518. The indicator loop 4521 can be configured to illuminate when the cartridge 4518 is properly connected. A power switch 4522 of the console 4501 can be between the arm 4514 and the opening of the cartridge housing. In some embodiments, the load/eject button 4520, the opening of the cartridge housing, the power switch 4522, the arm 4514, and the pedal 4516 can be primarily accessible from a rear side of the console 4501. Furthermore, an active side 4524 (FIG. 40A) of the display 4508 can be visible, and the connector 4512 (FIG. 40A) can be primarily accessible, from a front side of the console 4501 opposite the rear side. This arrangement can reduce interference of support operations (e.g., changing the cartridge 4518) with visibility of the active side 4524 of the display 4508 and/or reduce the possibility of a cryotherapeutic device (not shown) being inadvertently disconnected from the connector 4512.

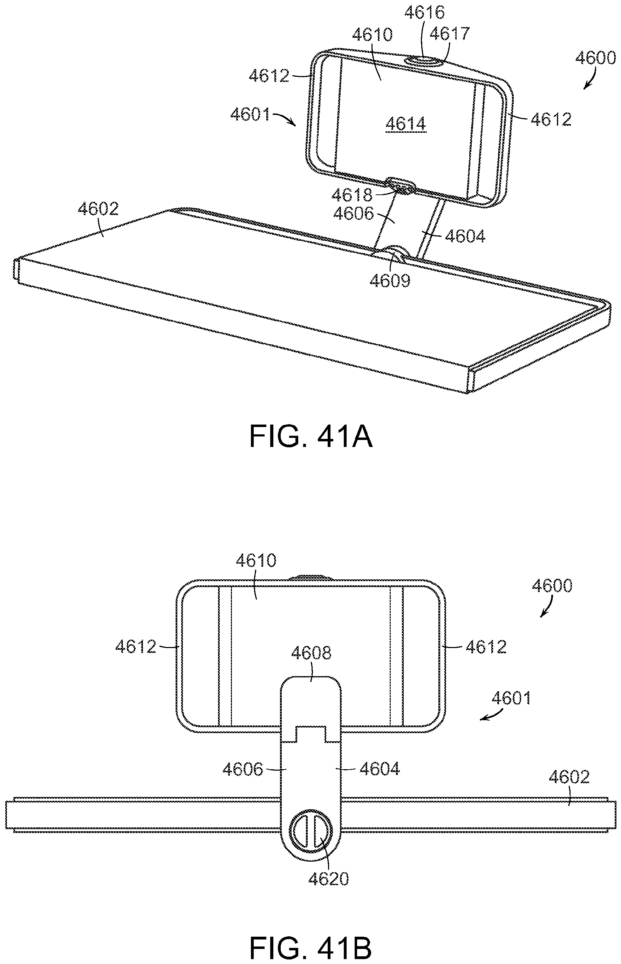

FIG. 41A is a perspective view and FIG. 41B is a profile view illustrating a console assembly 4600 that can include a console 4601, a table 4602, and an arm 4604 extending between the console 4601 and the table 4602. The console assembly 4600 can have a relatively compact configuration and can be configured to rest on a structure (e.g., a bed, a pole, or an instrument cabinet) in an operating room. In some embodiments, the table 4602 can be eliminated and the arm 4604 can be configured to mount directly to the structure in the operating room. For example, the arm 4604 can be configured to mount to a rail of the bed. The arm 4604 can include a first portion 4606 connected to the table 4602 and a second portion 4608 extending between the first portion 4606 and the console 4601. The first portion 4606 can be hingedly attached to the second portion 4608 and/or the console 4601 can be hingedly attached to the first portion 4606 to allow adjustment of the height and/or the angle of the console 4601.

The second portion 4608 of the arm 4604 can include a clamp 4609 configured to releasably secure the arm 4604 to the table 4602 or to another structure. The console 4601 and the arm 4604 can be rotatable relative to the clamp 4609. For example, when the clamp 4609 is attached to a rail of a bed, the console 4601 and the arm 4604 can be rotated to an upright position generally above the rail during a treatment procedure and rotated to an upside down position generally below the rail when not in use. As shown in FIG. 41B, the clamp 4609 can include a generally circular grip 4620 configured to lock and unlock the clamp 4609 relative to the table 4602 and/or to lock and unlock the arm 4604 relative to the clamp 4609. For example, turning the grip 4620 in different directions can release or engage the clamp 4609 from the table 4602 and/or allow or prevent rotation of the console 4601 and the arm 4604 relative to the clamp 4609. In other embodiments, the clamp 4609 can be replaced with a hinge configured to allow the console 4601 and the arm 4604 to rotate outward relative to the table 4602 between the upright and upside-down positions.

The console 4601 can include a display 4610 and handles 4612 on either side of the display 4610. The console 4601 can further include a cartridge housing (not shown) behind an active side 4614 of the display 4610. The console assembly 4600 can include a cartridge 4616 configured to fit in the cartridge housing, and the console 4601 can include an indicator loop 4617 around an opening (not shown) of the cartridge housing. As shown in FIG. 41A, the console 4601 can further include a connector 4618 below the active side 4614 of the display 4610. A supply line (not shown) of the console 4601 extending between the connector 4618 and a cartridge connector (not shown) of the console 4601 can be relatively short, which can reduce premature warming of refrigerant passing through the supply line. In some embodiments, the console assembly 4600 can be configured to receive refrigerant from a canister (e.g., a canister under the bed or in a cabinet spaced apart from the bed). In these embodiments, the canister can be a backup or alternative refrigerant source. The canister can also replace the cartridge 4616 and the cartridge housing. In other embodiments, the cartridge housing can have a different position. For example, the cartridge housing can be within the arm 4604. For example, the opening to the cartridge housing can be accessible from the back side of the arm 4604. In these and other embodiments, the connector 4618 can be at a front side of the arm 4604.

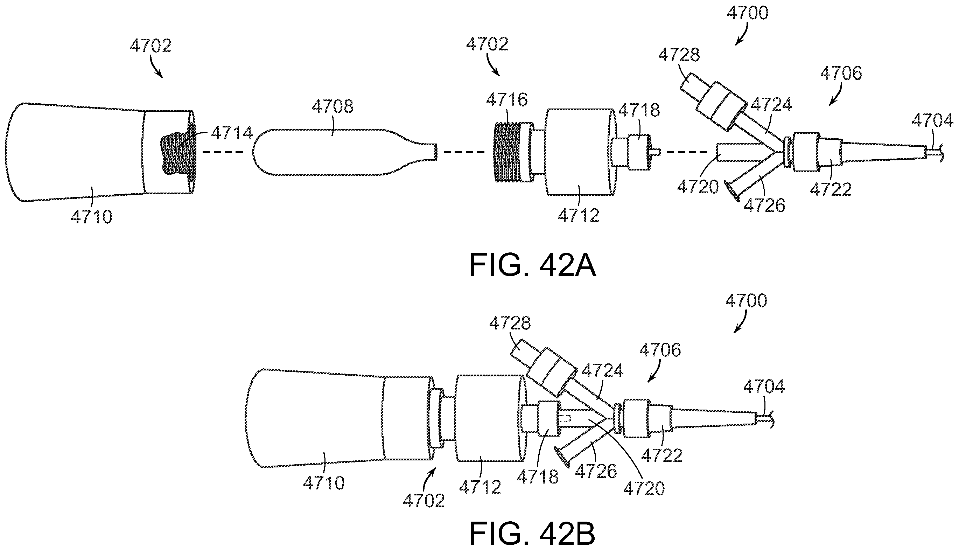

FIG. 42A is an exploded perspective view and FIG. 42B is a perspective view illustrating a cryotherapeutic system 4700 that can include a handle assembly 4702 and a cryotherapeutic device (not separately identified) having a shaft 4704. The cryotherapeutic system 4700 can further include a coupling member 4706 between the handle assembly 4702 and the shaft 4704. As shown in FIG. 42A, the cryotherapeutic system 4700 can include a cartridge 4708 and can be configured to contain the cartridge 4708 in the handle assembly 4702. For example, the handle assembly 4702 can include a cartridge housing 4710 and a cap 4712 configured to fit around the cartridge 4708. Securing the cap 4712 to the cartridge housing 4710 can cause a coupling member (not shown) within the cap 4712 to engage a coupling member (not shown) of the cartridge 4708. As shown in FIG. 42A, the cartridge housing 4710 can include a first threaded portion 4714 and the cap 4712 can include a corresponding second threaded portion 4716. The first threaded portion 4714 can be female and the second threaded portion 4716 can be male. In other embodiments, the first threaded portion 4714 can be male and the second threaded portion 4716 can be female. The cap 4712 can include a first adapter 4718 (e.g., a luer) and the coupling member 4706 can include a corresponding second adapter 4720. The coupling member 4706 can further include a third adapter 4722 configured to connect to the shaft 4704. In some embodiments, connections between the first and second adapters 4718, 4720 and between the third adapter 4722 and the shaft 4704 can be permanent. In other embodiments, one or both of the connections can be releasable. The coupling member 4706 can include a first branch 4724 and a second branch 4726, which can allow connection to various devices, valves, and lines. For example, the coupling member 4706 can include a syringe adapter 4728 connected to the first branch 4724.

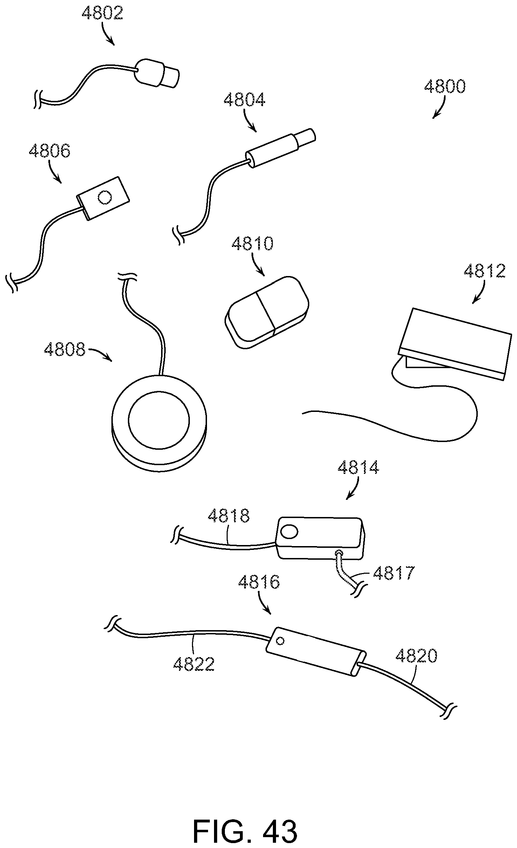

Cryotherapeutic systems configured in accordance with embodiments of the present technology can include one or more user-interface devices other than displays. User-interface devices, for example, can be configured to activate and/or terminate cooling cycles, which can include activating and/or terminating flow of refrigerant from consoles to cryotherapeutic devices. In other embodiments, this functionality can be included in handles of the cryotherapeutic devices and/or combined with display functionality (e.g., in touch screens). FIG. 43 is a set of perspective views illustrating a selection of user-interface devices 4800 having a variety of configurations. Among the user-interface devices 4800 are a first plunger 4802, a second plunger 4804, a relatively small wired push button 4806, a relatively large wired push button 4808, a wireless push button 4810, and a foot pedal 4812. The first and second plungers 4802, 4804, the push buttons 4806, 4808, 4810, and the foot pedal 4812 can be configured for operable connection to consoles and can be configured to control operation of supply control valves along supply lines extending from the consoles to the cryotherapeutic devices. These and other embodiments can be configured to default to positions (e.g., neutral positions) corresponding to closed configurations of the supply control valves and to move into activated positions in response to continuously applied force. For example, maintaining corresponding supply control valves in open configurations can include actively keeping the first and second plungers 4802, 4804, the push buttons 4806, 4808, 4810, and the foot pedal 4812 pressed. In other embodiments, the first and second plungers 4802, 4804, the push buttons 4806, 4808, 4810, and the foot pedal 4812 can be configured to initiate a cooling cycle when pressed and to terminate the cooling cycle when pressed again. In still other embodiments, the first and second plungers 4802, 4804, the push buttons 4806, 4808, 4810, and the foot pedal 4812 can be configured to initiate or to terminate a cooling cycle, but not both.

In some embodiments, the first and second plungers 4802, 4804, the push buttons 4806, 4808, 4810, and the foot pedal 4812 can be separate from the supply lines. In other embodiments, these and other user-interface devices 4800 can be along the supply lines. Also among the user-interface devices 4800 are a first inline device 4814 and a second inline device 4816. The first inline device 4814 can include a first inlet line 4817 and a first outlet line 4818. Similarly, the second inline device 4816 can include a second inlet line 4820 and a second outlet line 4822. The first and second inline devices 4814, 4816 can be configured, respectively, to change and maintain the direction of corresponding supply lines. For example, the first inline device 4814 can act as an angled junction between the first inlet line 4817 and the first outlet line 4818, and the second inline device 4816 can act as a generally straight junction between the second inlet line 4820 and the second outlet line 4822. Changing and/or maintaining the direction of corresponding supply lines can prevent tangling and enhance control of cryotherapeutic devices connected to the supply lines depending on the positions of corresponding consoles. For example, the first inline device 4814 can be well suited for use with a console positioned to the side of a patient and the second inline device 4816 can be well suited for use with a console positioned at the foot of a patient.

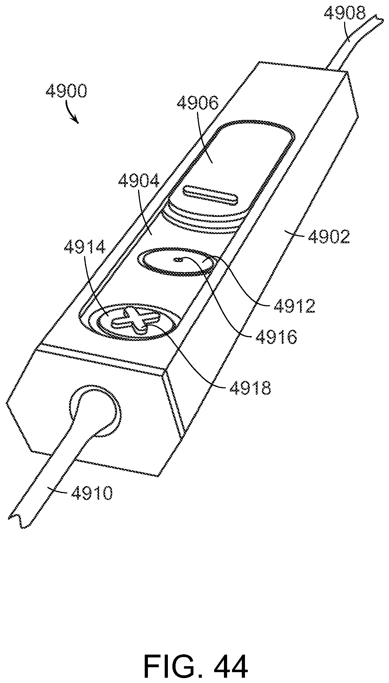

In some embodiments, a user-interface device can include separate buttons and/or other controls corresponding to initiating and terminating a cooling cycle. FIG. 44 is a perspective view of a user-interface device 4900 that can include an elongated body 4902 having a recess 4904 and a slidable cover 4906. The user-interface device 4900 can further include an inlet line 4908 and an outlet line 4910. The user-interface device 4900 can be an inline device configured to be along a supply line (not shown) extending through the inlet line 4908 and the outlet line 4910. The user-interface device 4900 can include an initiation button 4912 and a termination button 4914 in the recess 4904. The initiation button 4912 can include a circular indentation 4916 or another tactile identifier associated with initiating the flow of refrigerant. Similarly, the termination button 4914 can include an X-shaped projection 4918 or another tactile identifier associated with terminating the flow of refrigerant. Tactile identifiers can be useful to facilitate operation of the user-interface device 4900 while focusing elsewhere (e.g., on a display). The cover 4906 can be configured to slide over the initiation button 4912 and the termination button 4914 to reduce the likelihood that the initiation button 4912 and the termination button 4914 will be activated inadvertently. The user-interface device 4900 can be shaped as an elongated polygonal solid (e.g., an elongated hexagonal solid). In other embodiments, the user-interface device 4900 can have another shape (e.g., another non-round shape that allows the user-interface device 4900 to rest flat on a surface without rolling when not in use). Furthermore, the user-interface device 4900 can include other control features. For example, in some embodiments, the user-interface device 4900 can include a rotatable portion (not shown) at the outlet line 4910 and the rotatable portion can rotatably click into a plurality of positions corresponding to a plurality of control settings (e.g., refrigerant flow rates).

### E. Selected Examples of Pre-Cooling in Cryotherapeutic Systems

Selected examples of pre-cooling in cryotherapeutic systems configured in accordance with embodiments of the present technology are described in this section with reference to FIGS. 45A-50. It will be appreciated that specific elements, substructures, advantages, uses, and/or other features of the embodiments described with reference to FIGS. 45A-50 can be suitably interchanged, substituted, or otherwise configured with one another and/or with the embodiments described with reference to FIGS. 1A-44 and 51-67 in accordance with additional embodiments of the present technology. Furthermore, suitable elements of the embodiments described with reference to FIGS. 45A-50 can be used as stand-alone and/or self-contained devices.

In cryogenic renal nerve modulation, the volume of refrigerant available for cooling can be limited (e.g., due to limited supply or limited flow-passage dimensions). Accordingly, it can be useful to increase the cooling capacity of the refrigerant. Pre-cooling the refrigerant before expanding the refrigerant in a cooling assembly is one example of a process that can increase a refrigerant's cooling capacity. Even when cooling occurs primarily through phase change, using colder refrigerant before the phase change can increase the amount of cooling. Pre-cooling can reduce the volume of refrigerant needed for therapeutically effective cryogenic renal nerve modulation, which can allow smaller and more flexible shafts to be used within the vasculature. Pre-cooling also can mitigate reductions in cooling capacity associated with other components of cryotherapeutic systems, such as thermally insulative members within an applicator and in-line solenoid valves that release heat during operation. In some embodiments, a supply tube can be in thermal communication with an exhaust tube, and refrigerant exhausted from an associated cooling assembly can be used to pre-cool refrigerant supplied to the cooling assembly. Alternatively or in addition (e.g., at a different position along the lengths of supply and exhaust tubes), precooled refrigerant in the supply tube can cool the refrigerant exhaust in the exhaust tube. This can reduce back pressure in the cooling assembly, which can further increase cooling capacity.

Pressurized refrigerant used in cryogenic renal nerve modulation can be supplied outside the vasculature at room temperature (e.g., from a room-temperature dewar). As the pressurized refrigerant travels along a supply tube within the vasculature, it can increase in temperature via heat transfer with warm blood and tissue. For example, as pressurized refrigerant supplied at about room temperature (e.g., about 23°C) passes through the vasculature at body temperature (e.g., about 37°C), the temperature of the pressurized refrigerant can increase to about 25°C to 37°C before reaching a cooling assembly. Cryotherapeutic devices configured in accordance with embodiments of the present technology can include a pre-cooling assembly configured to cool pressurized refrigerant before the pressurized refrigerant expands in an associated cooling assembly. For example, pressurized refrigerant can be cooled to have a temperature just before expansion in an associated cooling assembly that is less than body temperature (e.g., less than about 20°C or less than about 10°C). Such pre-cooling assemblies can be configured to be outside the vasculature and/or to utilize the same refrigerant supply as an associated cooling assembly. In some embodiments, pre-cooling can be useful to maintain refrigerant in liquid form until it reaches a cooling assembly where cryogenic cooling is desired. For example, evaporation associated with warming of refrigerant passing through portions of a cryotherapeutic device proximal to a cooling assembly can be reduced.

FIG. 45A is a plan view and FIG. 45B is a cross-sectional view illustrating a pre-cooling assembly 6000 and associated cryotherapeutic-system components. The pre-cooling assembly 6000 can include a hub 6002, an adapter 6004, and a flexible tubular member 6006 extending between the hub 6002 and the adapter 6004. The adapter 6004 can be configured to connect to a pressurized-refrigerant source (not shown). The pre-cooling assembly 6000 can be a portion of a cryotherapeutic system (not separately identified) including a control-wire conduit 6008 and a cryotherapeutic device (not separately identified) having an elongated shaft 6010. The control-wire conduit 6008 and the shaft 6010 can be connected to the hub 6002. For example, the hub 6002 can include a primary connector 6012 attached to the shaft 6010, an exhaust portal 6014 configured to vent to the atmosphere, a first branch 6016 connected to the tubular member 6006, and a second branch 6018 connected to the control-wire conduit 6008. In some embodiments, the hub 6002 can include one or more additional branches, such as a branch including a tube (not shown) fluidly connected to a proximal syringe adapter (not shown) (e.g., a proximal syringe adapter including a diaphragm configured to be punctured with a needle of a syringe). A syringe can be used, for example, to introduce contrast agent in the vicinity of a cooling assembly within the vasculature and/or to introduce filler material into a filler lumen of a cooling assembly within the vasculature.

With reference again to FIGS. 45A-45B, the cryotherapeutic system can include two control wires 6020 (FIG. 45B) extending from the control-wire conduit 6008, through the hub 6002, and into the shaft 6010. The hub 6002 can define a generally straight exhaust flow path from the shaft 6010 to the atmosphere through the exhaust portal 6014. The tubular member 6006 can include a proximal portion 6022 at the adapter 6004 and a distal portion 6024 at the first branch 6016. As shown in FIG. 45B, the proximal portion 6022 can include a first plug 6026 and a second plug 6028, and the adapter 6004 can include an opening 6030 proximate the second plug 6028. The adapter 6004 can include a variety of suitable structures for connection to a pressurized-refrigerant source, such as a threaded fitting, a compression fitting, or a barbed fitting.

As shown in FIG. 45B, the pre-cooling assembly 6000 can include a first supply tube 6032 and a second supply tube 6034. The first supply tube 6032 and the second supply tube 6034 can include a first proximal opening 6036 and a second proximal opening 6038, respectively, at the second plug 6028. The first proximal opening 6036 and the second proximal opening 6038 can fluidly connect the first supply tube 6032 and the second supply tube 6034, respectively, to a passage defined by the opening 6030. From the second plug 6028, the first supply tube 6032 and the second supply tube 6034 can extend through the proximal portion 6022 and through the first plug 6026. The distal portion 6024 can define a pre-cooling expansion chamber (not separately identified) extending from the first plug 6026 to the exhaust flow path within the hub 6002. The first supply tube 6032 can extend through the pre-cooling expansion chamber, through the hub 6002 and into the shaft 6010. The portion of the first supply tube 6032 extending from first proximal opening 6036 to the shaft 6010 can be a first portion of the first supply tube 6032. A second portion (not shown) of the first supply tube 6032 can be proximate a cooling assembly (not shown) configured to be within the vasculature.

Expanding pressurized refrigerant into the pre-cooling expansion chamber from the second supply tube 6034 can cool the pre-cooling expansion chamber and thereby cool the first supply tube 6032 and liquid refrigerant within the first supply tube 6032. If pre-cooling is performed distant from an entry point into the vasculature (e.g., if pressurized refrigerant is cooled in a console (not shown) before being transported to an entry point into the vasculature), heat from the atmosphere can cause undesirable warming of the pre-cooled pressurized refrigerant. Positioning the pre-cooling expansion chamber proximate the hub 6002 can reduce such undesirable warming. The pre-cooling assembly 6000 can have a length sufficient to allow heat-transfer between expanded refrigerant within the pre-cooling expansion chamber and pressurized refrigerant within a portion of the first supply tube 6032 within the pre-cooling expansion chamber. For example, the pre-cooling expansion chamber can have a length greater than about 10 cm (e.g., greater than about 15 cm or greater than about 25 cm). In some embodiments, the pre-cooling expansion chamber can have a length from about 20 cm to about 30 cm.

After cooling the first supply tube 6032, refrigerant from the pre-cooling expansion chamber can join the exhaust flow path within the hub 6002 and vent out the exhaust portal 6014 to the atmosphere. FIG. 45B shows a first arrow 6042 indicating a flow direction of refrigerant through the exhaust portal 6014 and a second arrow 6044 indicating a flow direction of refrigerant through the pre-cooling expansion chamber. The exhaust flow path within the hub 6002 through the exhaust portal 6014 can be generally aligned with the flow of exhaust through the shaft 6010. In contrast, the flow path of refrigerant through the pre-cooling expansion chamber can be out of alignment with the exhaust flow path within the hub 6002 through the exhaust portal 6014 and/or the flow of exhaust through the shaft 6010.

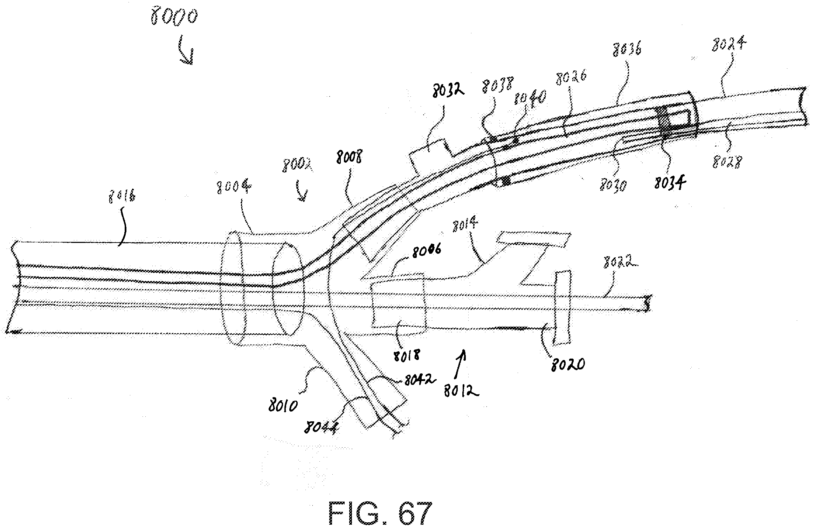

FIG. 67 is a partially schematic view illustrating a pre-cooling assembly 8000 configured in accordance with another embodiment of the present technology. The pre-cooling assembly 8000 can include a hub 8002 having a primary connector 8004, a central branch 8006, a first side branch 8008, and a second side branch 8010. The pre-cooling assembly 8000 can further include a Y-connector 8012 connected to the central branch 8006. The Y-connector 8012 can include a lateral extension 8014 configured to vent refrigerant exhaust. In some embodiments, the pre-cooling assembly 8000 can be a portion of a cryotherapeutic system (not separately identified) including a cryotherapeutic device (not separately identified) having an elongated shaft 8016 connected to the primary connector 8004 of the hub 8002. Refrigerant exhaust from the cryotherapeutic device can flow through the shaft 8016, through the hub 8002, into the Y-connector 8012, and out the lateral extension 8014 to the atmosphere or to a containment system (not shown). The Y-connector 8012 can include a distal portion 8018 and a proximal portion 8020 generally in alignment with one another. A pressure-monitoring tube 8022 of the cryotherapeutic system can extend through the Y-connector 8012 between the distal portion 8018 and the proximal portion 8020. The pressure-monitoring tube 8022, for example, can have an inner diameter of about 0.008 inches, or another suitable dimension. In some embodiments, the pressure-monitoring tube 8022 can extend through the hub 8002, into the shaft 8016, and terminate within an intravascular portion of the cryotherapeutic device.

As shown in FIG. 67, the pre-cooling assembly 8000 can include a pre-cooling tube 8024 extending from the first side branch 8008 of the hub 8002. A first supply tube 8026 of the pre-cooling assembly 8000 can extend through the pre-cooling tube 8024, through the hub 8002, and into the shaft 8016. The first supply tube 8026, for example, can be configured to supply refrigerant to an intravascular portion of the cryotherapeutic device. A second supply tube 8028 of the pre-cooling assembly 8000 can have a distal opening 8030 within the pre-cooling tube 8024. Joule-Thomson expansion and/or phase change of refrigerant from the distal opening 8030 of the second supply tube 8028 can cool a space within the pre-cooling tube 8024 and pre-cool pressurized refrigerant within the first supply tube 8026. A lateral outlet 8032 of the pre-cooling tube 8024 can be configured to exhaust expanded refrigerant from the second supply tube 8028. The pre-cooling assembly 8000 can further include a plug 8034 within the pre-cooling tube 8024 around the first and second supply tubes 8026, 8028.

The pre-cooling tube 8024 can be configured to contain high-pressure refrigerant proximal to the plug 8034 and expanded refrigerant distal to the plug 8034. In some embodiments, the pre-cooling tube 8024 can have different wall thicknesses proximal and distal to the plug 8034. For example, the pre-cooling tube 8024 can have an inner diameter of about 0.025 inches and an outer diameter of about 0.55 inches proximal to the plug 8034 and an inner diameter of about 0.039 inches and an outer diameter of about 0.55 inches distal to the plug 8034. A greater wall thickness of the pre-cooling tube 8024 proximal to the plug 8034 than distal to the plug 8034 can enhance the ability of the pre-cooling tube 8024 to contain high-pressure refrigerant proximal to the plug 8034 and/or enhance the area available for refrigerant expansion distal to the plug 8034. In some embodiments, portions of the pre-cooling tube 8024 having different wall thicknesses can be joined using adhesive (e.g., UV light-cured adhesive). Suitable adhesives include LOCTITE® 3211 adhesive, among others. In other embodiments, the portions of the pre-cooling tube 8024 having different wall thicknesses can be integrally formed (e.g., using variable extrusion). The pre-cooling tube 8024 can be made of a variety of suitable materials, such as polyamide (e.g., GRILAMID® polyamide), among others.

As shown in FIG. 67, the pre-cooling assembly 8000 can include a jacket 8036 around a portion of the pre-cooling tube 8024. The jacket 8036 can be configured to thermally insulate the pre-cooling tube 8024 to reduce or eliminate ice build-up on the exterior of the pre-cooling tube 8024 due to condensation and freezing of atmospheric moisture. Ice build up can cause the pre-cooling tube 8024 to be difficult to handle during use and can cause unwanted water to form when the pre-cooling tube 8024 is not in use and the ice build up melts. In some embodiments, the jacket 8036 can include heat-shrink tubing (e.g., heat-shrink polyolefin tubing). In other embodiments, the jacket 8036 can have other suitable configurations and/or compositions. As shown in FIG. 67, the pre-cooling assembly 8000 can include adhesive 8038 between the jacket 8036 and the pre-cooling tube 8024. The adhesive 8038, for example, can secure the jacket 8036 to the pre-cooling tube 8024. The adhesive 8038 can be positioned entirely outside the pre-cooling tube 8024 so as not to interfere with movement of expanded refrigerant through the pre-cooling tube 8024 and out the lateral outlet 8032.

A thermocouple 8040 of the pre-cooling assembly 8000 can be positioned within the pre-cooling tube 8024 near the adhesive 8038 (e.g., about 1 cm proximal to the adhesive 8038). The thermocouple 8040 can be configured to measure a temperature within the pre-cooling tube 8024 (e.g., to monitor pre-cooling activity). The pre-cooling assembly 8000 can include a first thermocouple lead 8042 extending from the thermocouple 8040, through the hub 8002, and exiting the second side branch 8010 of the hub 8002. As shown in FIG. 67, a second thermocouple lead 8044 of the cryotherapeutic system can extend from into the second side branch 8010 of the hub 8002, through the hub 8002, and into the shaft 8016. The second thermocouple lead 8044, for example, can connect to a second thermocouple (not shown) in an intravascular portion of the cryotherapeutic device. In some embodiments, the second thermocouple lead 8044 can be secured to the pressure-monitoring tube 8022 (e.g., using adhesive) at one or more points along the lengths of the second thermocouple lead 8044 and the pressure-monitoring tube 8022.

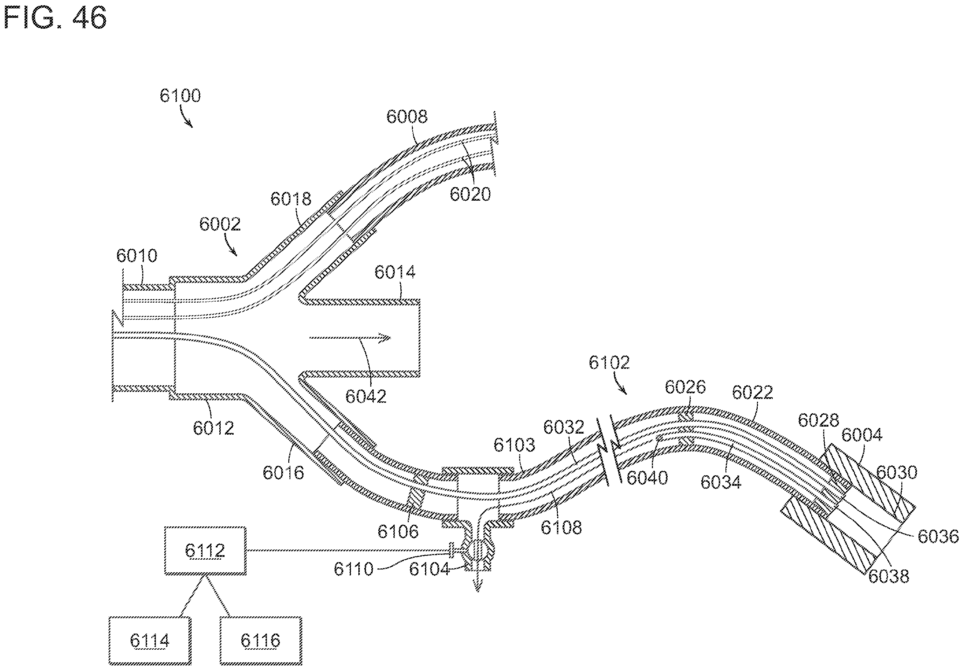

FIG. 46 is a cross-sectional view illustrating a pre-cooling assembly 6100 similar to the pre-cooling assembly 6000 of FIGS. 45A-45B. In some embodiments, the pre-cooling assembly 6100 can include a pre-cooling expansion chamber fluidly separate from an exhaust passage. As shown in FIG. 46, the pre-cooling assembly 6100 can include a flexible tabular member 6102 having a distal portion 6103 and extending between the hub 6002 and the adapter 6004. The tubular member 6102 can include a valve 6104 and a third plug 6106 fluidly separating the pre-cooling expansion chamber from internal portions of the shaft 6010 and the hub 6002. The first supply tube 6032 can extend through the third plug 6106 before extending through the hub 6002 and into the shaft 6010.

FIG. 46 shows an arrow 6108 indicating a flow direction of refrigerant through the pre-cooling expansion chamber when the valve 6104 is open. When the valve 6104 is closed, pressure within the pre-cooling expansion chamber can increase until it equilibrates with the second supply tube 6034, thereby causing flow through the second supply tube 6034 to stop. In this way, opening and closing the valve 6104 can turn pre-cooling on or off. Partially opening the valve 6104 can regulate pressure within the pre-cooling expansion chamber and thereby regulate refrigerant flow through the second supply tube 6034 and an associated pre-cooling temperature. For example, the pre-cooling assembly 6100 can include an actuator 6110 operably connected to the valve 6104 and a processor 6112 configured to signal the actuator 6110. The pre-cooling assembly 6100 can further include a user interface 6114 and a sensor 6116 configured to direct the actuator 6110 via the processor 6112 to open or close the valve 6104 fully or incrementally. In some embodiments, the pre-cooling assembly 6100 can include the user interface 6114 and not the sensor 6116 or include the sensor 6116 and not the user interface 6114. The sensor 6116, for example, can be a temperature sensor of an associated cooling assembly (not shown). In some embodiments, a temperature sensor can send a signal to the processor 6112 that can cause the valve 6104 (a) to open and pre-cooling to increase if a detected temperature of the cooling assembly or tissue proximate the cooling assembly is higher than a desired value, or (b) to close and pre-cooling to decrease if a detected temperature of the cooling assembly or tissue proximate the cooling assembly is lower than a desired value.

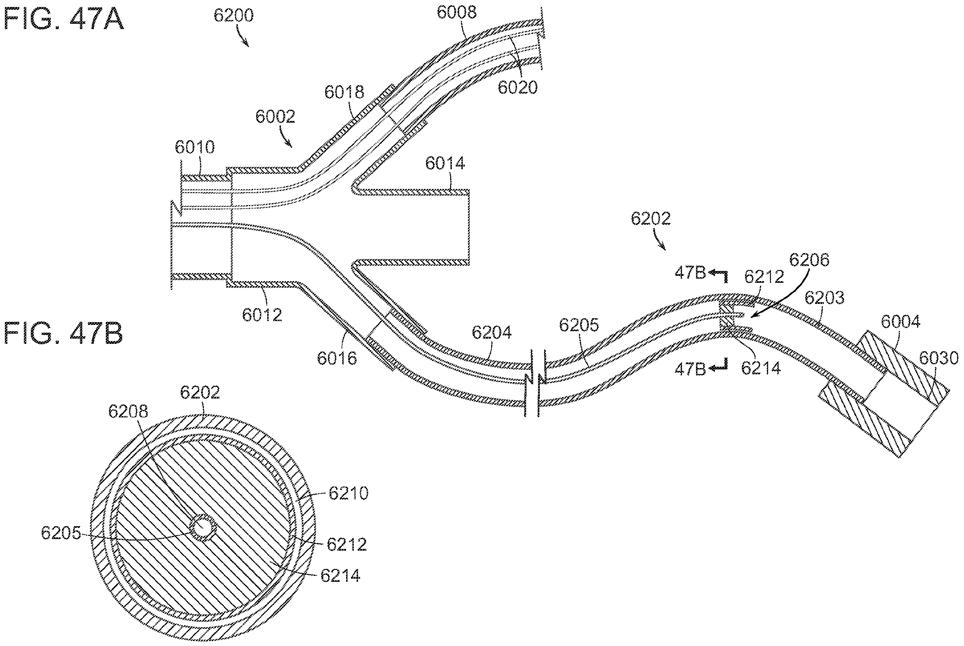

FIG. 47A is a cross-sectional view illustrating a pre-cooler 6200 and associated cryotherapeutic-system components. FIG. 47B is a cross-sectional view illustrating the pre-cooler 6200 taken along the line 47B-47B. The pre-cooler 6200 can include a container 6202 having a proximal portion 6203 and a distal portion 6204 and extending between the hub 6002 and the adapter 6004. With reference to FIG. 45B, accessing an internal portion of the tubular member 6006 to form the first plug 6026 can be challenging. With reference again to FIGS. 47A-47B, instead of the first plug 6026 (FIG. 45B) and the second supply tube 6034 (FIG. 45B), the pre-cooler 6200 can include a supply tube 6205 and a flow separator 6206 attached to the supply tube 6205. In some embodiments, the flow separator 6206 can divide the container 6202 into the proximal portion 6203 and the distal portion 6204. The proximal portion 6203 can define a proximal chamber or a combined supply lumen between the opening 6030 and the flow separator 6206. The distal portion 6204 can define a pre-cooling expansion chamber. As most clearly shown in FIG. 47B, the flow separator 6206 can include a primary passage 6208 fluidly connected to the supply tube 6205 and the pre-cooler 6200 can include a secondary passage 6210 along a periphery of the flow separator 6206. The secondary passage 6210 can be sized to cause a pressure drop sufficient to expand refrigerant and cool the pre-cooling expansion chamber. In some embodiments, the flow separator 6206 can include a tubular segment 6212 and a flow-separator plug 6214. The flow-separator plug 6214 can be positioned between an outer surface of the supply tube 6205 and an inner surface of the container 6202. The tubular segment 6212 can be selected to have an outer cross-sectional dimension (e.g., diameter) slightly smaller than an inner cross-sectional dimension (e.g., diameter) of the container 6202. The flow-separator plug 6214 can include, for example, an adhesive material configured to bond to the outer surface of the supply tube 6205 and the inner surface of the container 6202.

In some embodiments, the flow separator 6206 can float in the container 6202 (e.g., the flow separator 6206 can be non-fixed within the container 6202) such that the secondary passage 6210 is an annular space between the flow separator 6206 and an inner surface of the container 6202. In other embodiments, the flow separator 6206 can have a different configuration. For example, flow separators configured in accordance with embodiments of the present technology can be fixed to containers, and pre-cooling passages can extend through the flow separators around only portions (e.g., curved portions) of the peripheries of the flow separators. In other examples, flow separators configured in accordance with embodiments of the present technology can be attached to containers around generally their entire circumferences, and the flow separators can include openings spaced inwardly apart from the peripheries of the flow separators. For example, these and other flow separators can include internal openings configured to expand refrigerant into pre-cooling expansion chambers.

FIG. 48A is a cross-sectional view illustrating a pre-cooling assembly 6300 and associated cryotherapeutic-system components. FIG. 48B is a cross-sectional view of the pre-cooling assembly 6300 taken along the line 48B-48B. In some embodiments, the pre-cooling assembly 6300 can be similar to the pre-cooler 6200 of FIGS. 47A-47B, except having different flow-separator and supply-tube configurations. As shown in FIGS. 48A-48B, the pre-cooling assembly 6300 can include a tubular member 6302 having a proximal portion 6303 and a distal portion 6304 and extending between the hub 6002 and the adapter 6004. The pre-cooling assembly 6300 can further include a supply tube 6306 and a flow separator 6308 attached to the supply tube 6306. In some embodiments, the flow separator 6308 can be without a tubular segment and can be constructed, for example, from a cylindrical block of material (e.g., rubber, polymer, metal, or another material) including a hole through which the supply tube 6306 can be threaded or otherwise attached. As most clearly shown in FIG. 48B, the pre-cooling assembly 6300 can include a passage 6310 along a periphery of the flow separator 6308. The supply tube 6306 can extend through the flow separator 6308 and can be attached to an inner surface of the proximal portion 6303 of the tubular member 6302 proximate the opening 6030. Attaching the supply tube 6306 to an accessible portion of the tubular member 6302 can be useful to prevent or reduce undesirable longitudinal movement of the flow separator 6308 and the supply tube 6306 when the proximal chamber is at high pressure.

Pre-cooling assemblies configured in accordance with embodiments of the present technology can be arranged in compact configurations. For example, these pre-cooling assemblies can be entirely or partially contained within the handles of cryotherapeutic devices. FIG. 49 is a partially cross-sectional view illustrating a pre-cooling assembly 6400 and related cryotherapeutic-system components. The pre-cooling assembly 6400 can be partially contained within a handle 6402 of a cryotherapeutic device (not separately identified) and can include a hub 6404, an adapter 6406, and a tubular member 6408 extending between the hub 6404 and the adapter 6406. The handle 6402 can include a proximal portion 6410, and the tubular member 6408 can extend through the proximal portion 6410. The hub 6404 can include an elongated exhaust portal 6412 extending through the proximal portion 6410, and the cryotherapeutic device can include a control-wire conduit 6414 extending from the hub 6404 through the proximal portion 6410. In some embodiments, the tubular member 6408 can be coiled around the exhaust portal 6412. Furthermore, the handle 6402 can be insulated to prevent heat loss to the atmosphere and to improve pre-cooling efficiency.

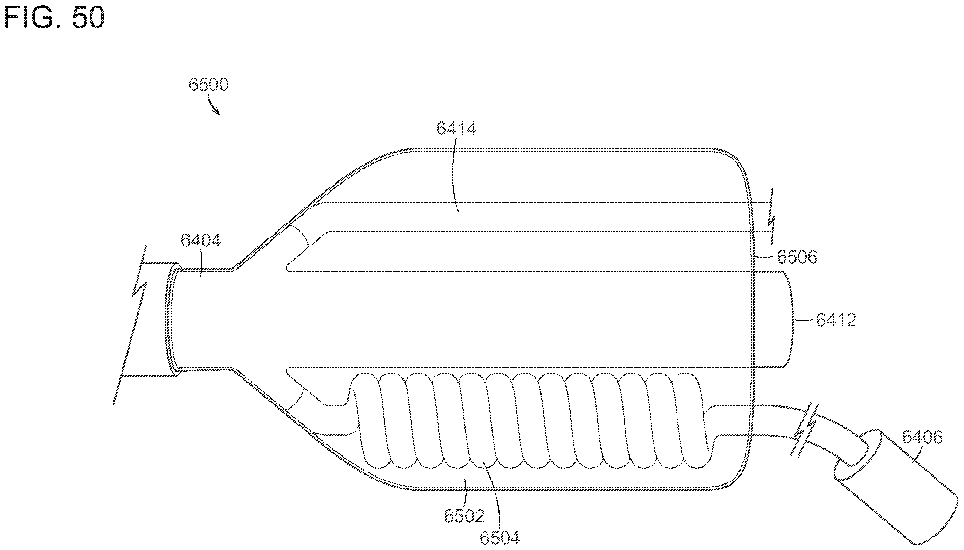

FIG. 50 is a partially cross-sectional view illustrating a pre-cooling assembly 6500 similar to the pre-cooling assembly 6400 of FIG. 49. The pre-cooling assembly 6500 can be partially contained within a handle 6502 of a cryotherapeutic device (not separately identified) and can include a tubular member 6504 extending between the hub 6404 and the adapter 6406. The handle 6502 can include a proximal portion 6506, and the tubular member 6504 can extend through the proximal portion 6506. In some embodiments, a helical portion of the tubular member 6504 can be spaced apart from the exhaust portal 6412.

### F. Selected Examples of Displays in Cryotherapeutic Systems

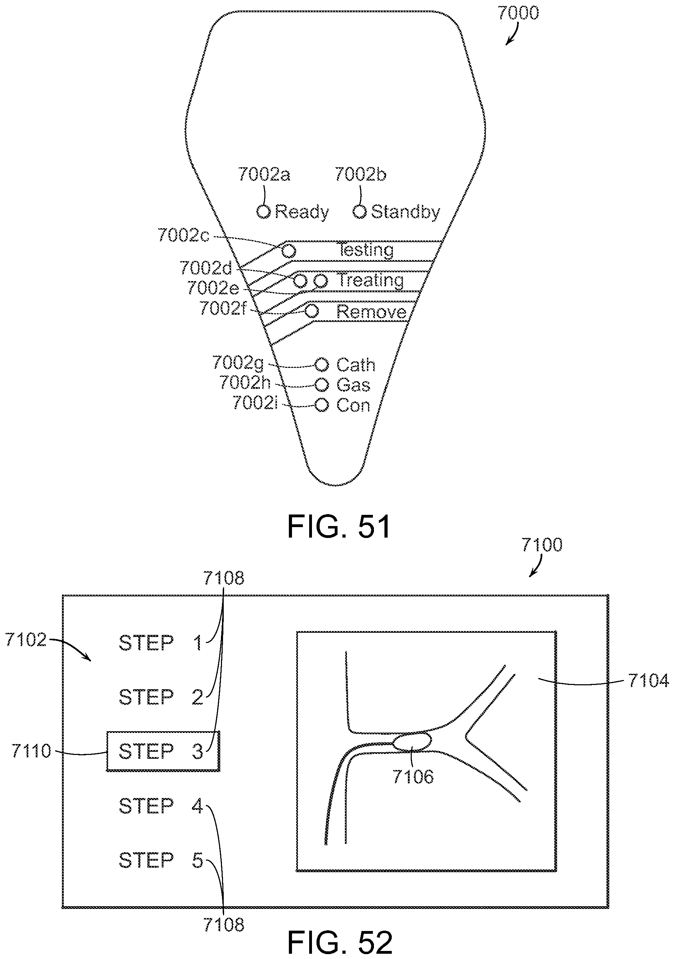

Selected examples of displays in cryotherapeutic systems configured in accordance with embodiments of the present technology are described in this section with reference to FIGS. 51-56. It will be appreciated that specific elements, substructures, advantages, uses, and/or other features of the embodiments described with reference to FIGS. 51-56 can be suitably interchanged, substituted, or otherwise configured with one another and/or with the embodiments described with reference to FIGS. 1A-50 and 57-67 in accordance with additional embodiments of the present technology. Furthermore, suitable elements of the embodiments described with reference to FIGS. 51-56 can be used as stand-alone and/or self-contained devices.

Cryotherapeutic systems configured in accordance with embodiments of the present technology can include machine displays incorporated directly into one or more portions of a machine housing (e.g., a console housing). FIG. 51 is a plan view illustrating a machine display 7000 that can include indicators 7002 (individually identified as 7002a-i). The machine display 7000 can be incorporated, for example, into the console 1502 of FIGS. 10A-10C or another suitable console configured in accordance with embodiments of the present technology. In some embodiments, the indicators 7002 can be lights (e.g., light-emitting diodes). The indicator 7002a can be configured, for example, to indicate that the console is powered on and ready to be used. The indicator 7002b can be configured, for example, to indicate that the console is powered on, but not yet functional. The indicator 7002c can be configured, for example, to indicate that a pressure-decay test or a pre-inflation of a balloon (not shown) within a connected cryotherapeutic device (not shown) is in progress. The indicator 7002d can be configured, for example, to indicate that a first cooling period is in progress. The indicator 7002e can be configured, for example, to indicate that a second cooling period is in progress. The indicator 7002f can be configured, for example, to indicate that an applicator (not shown) of the cryotherapeutic device has warmed sufficiently after a cooling period to be removed from a treatment site within the vasculature. The indicators 7002g, 7002h, and 7002i can be configured, for example, to indicate that an error has occurred in a catheter (not shown), a cartridge (not shown), or the console, respectively. Other embodiments of the machine display 7000 can have different configurations and/or can provide more or less information. In some embodiments, one or more of the indicators 7002 can display different colors (e.g., by activating different-color light-emitting diodes) to convey information. For example, the indicators 7002d-7002e can display blue to indicate cooling, orange to indicate warming, and green to indicate readiness.

In addition to or instead of machine displays, cryotherapeutic systems configured in accordance with embodiments of the present technology can include displays configured to appear on screens or monitors. These displays, for example, can include coded instructions on computer-readable media configured to generate various dynamic and/or static display elements. FIG. 52 is a profile view of a display 7100 that can include a primary-stage list 7102, an anatomical image 7104, and a device image 7106 registered with the anatomical image 7104. The primary-stage list 7102 can include primary stages 7108 (e.g., portions of the method 600 of FIG. 6) of an overall treatment procedure and an indicator box 7110 around a currently occurring one of the primary stages 7108. In some embodiments, the primary stages 7108 can include a first cryotherapeutic-cycling stage at a first treatment site within a first renal artery and a second cryotherapeutic-cycling stage at a second treatment site within a second renal artery. The anatomical image 7104 can be an angiographic, fluoroscopic, or another suitable type of image showing one or both renal arteries of a patient. In some embodiments, the device image 7106 can be a graphic registered with the anatomical image 7104 or it can be an angiographic or fluoroscopic image that indicates a size and/or position of a shaft, a cooling assembly, a balloon, and/or an applicator at a treatment site within the vasculature. The display 7100 can also include other information (e.g., patient-status information (e.g., blood pressure) and/or patient-identification information).

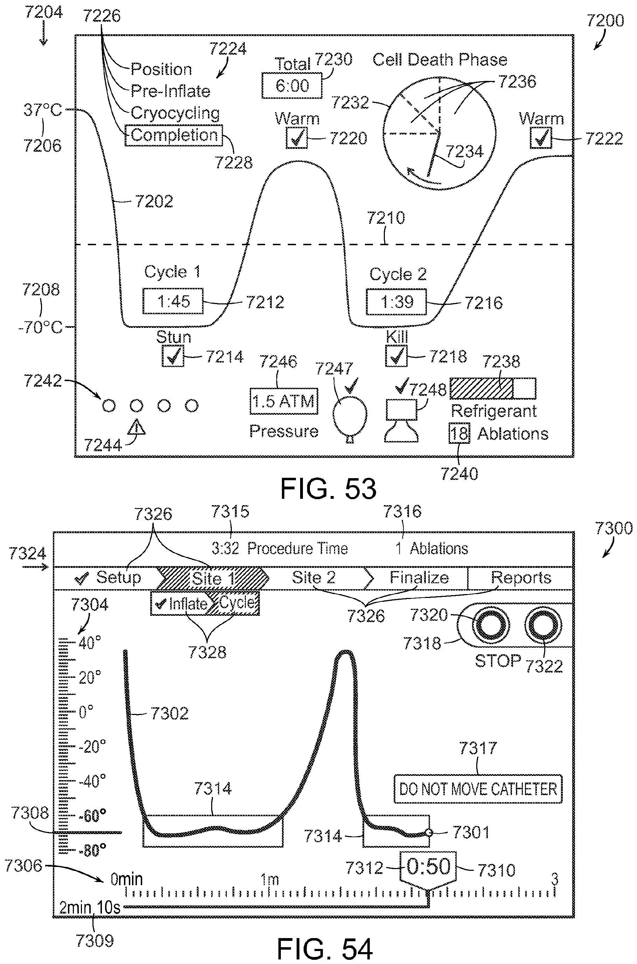

FIG. 53 is a profile view of a display 7200 that can include a plot 7202 of temperature versus time for a cryotherapeutic-cycling stage of a treatment procedure. The temperature can correspond, for example, to a measured temperature at or near a cooling assembly at a treatment site (e.g., at or near a balloon or an applicator of the cooling assembly at the treatment site). In one embodiment, the temperature is measured within the shaft along a portion of the exhaust lumen. The plot 7202 can be generated (e.g., left to right) in real time, near real time, or delayed time and can begin, for example, when refrigerant flow is first initiated. In some embodiments, the plot 7202 can begin after positioning a cooling assembly at a treatment site, pre-inflating a balloon, and/or another preliminary operation. The plot 7202 can be configured to refresh between cryotherapeutic-cycling stages performed at different treatment sites (e.g., in different renal arteries of a patient during a bilateral treatment procedure). The display 7200 can include a temperature scale 7204 (e.g., on the y-axis) and a time scale (not shown) (e.g., on the x-axis). The temperature scale 7204 can include a starting temperature 7206 and a target temperature 7208 (e.g., a target temperature for therapeutically effective cryogenic nerve modulation). In some embodiments, the resolution of the temperature scale 7204 can be higher around the target temperature 7208 (e.g., plus or minus about 10 or about 20 degrees of the target temperature 7208) than at other portions of the temperature scale 7204. For example, the temperature scale 7204 can be enlarged around the target temperature 7208 relative to other portions of the temperature scale 7204. The display 7200 can include a line 7210 corresponding to a threshold temperature below which therapeutically effective cryogenic nerve modulation begins to occur.

The display 7200 can further include a first cooling-period timer 7212, a first cooling-period checkbox 7214, a second cooling-period timer 7216, and a second cooling-period checkbox 7218 proximate corresponding portions of the plot 7202. The first cooling-period timer 7212 and the second cooling-period timer 7216 can be configured to display the amount of time the temperature is below the line 7210 during a first cooling period and a second cooling period, respectively, of the cryotherapeutic-cycling stage of the treatment procedure. When a sufficient time has elapsed, the first cooling-period checkbox 7214 and the second cooling-period checkbox 7218 can be configured to indicate completion of the first and second cooling periods, respectively. The display 7200 can further include a first warming checkbox 7220, a second warming checkbox 7222, and a secondary-stage list 7224. The first and second warming checkboxes 7220, 7222 can be configured to indicate when the temperature has increased sufficiently and for a sufficient period of time for cryoadhesion to have ended such that the cooling assembly can be moved within the vasculature. For example, the first and second warming checkboxes 7220, 7222 can indicate when a predetermined period of time has elapsed after the treatment site has returned to body temperature. The secondary-stage list 7224 can include secondary stages 7226 with one being the cryotherapeutic-cycling stage. The secondary-stage list 7224 can further include an indicator box 7228 around a currently occurring one of the secondary stages 7226.

As shown in FIG. 53, the display 7200 can include a procedure timer 7230 and a cell-death indicator 7232. The procedure timer 7230 can be configured to indicate the total time of the treatment procedure. The cell-death indicator 7232 can be configured to indicate the estimated phases of cell death during the cryotherapeutic-cycling stage. For example, the cell-death indicator 7232 can be similar to a clock and can include a hand 7234 and a plurality of radial segments 7236 corresponding to phases that estimate cryogenic cell death at the treatment site. The hand 7234 can be configured to move through the segments 7236 during the cryotherapeutic-cycling stage (e.g., at least partially based on the temperatures and/or durations of the first and second cooling periods of the cryotherapeutic-cycling stage). In some embodiments, the hand 7234 can move through the segments 7236 at least partially based on a calculated cell-death rate depending on both temperature and time during the cryotherapeutic-cycling stage. The display 7200 can include a refrigerant meter 7238 and a remaining count 7240. The refrigerant meter 7238 can be configured to indicate the quantity of refrigerant remaining in an associated refrigerant source (e.g., an associated cartridge or canister) and the remaining count 7240 can be configured to indicate the number of cryotherapeutic-cycling stages (e.g., ablations) that can be completed before the refrigerant source is exhausted (e.g., completely depleted or depleted to a level below which the refrigerant pressure would be inadequate).

The display 7200 can include a variety of suitable status indicators. For example, the display 7200 can include a plurality of operational indicators 7242 corresponding to operational characteristics (e.g., refrigerant pressure, exhaust pressure, etc.) of the cryotherapeutic system. The operational indicators 7242 can be configured to differentiate between normal and abnormal status. For example, the operational indicators 7242 can appear green during normal operation and red during errors. In addition or alternatively, the display 7200 can include warning flags 7244 (one shown in FIG. 53) configured to appear proximate an appropriate operational indicator 7242 during an error. The display 7200 can include a pressure reading 7246 configured to display the pressure within the balloon. A sudden increase or decrease in the pressure reading 7246 can indicate an error (e.g., a balloon rupture) and signal an operator to terminate a cryotherapeutic-cycling stage. The display 7200 can further include an intravascular summary check 7247 and a console summary check 7248 configured, respectively, to provide summary indications that all intravascular and console components of the cryotherapeutic system are functioning properly. In some embodiments, the intravascular summary check 7247 and the console summary check 7248 can include graphical associations (e.g., a balloon representing intravascular components and a canister representing console components).

FIG. 54 is a profile view of a display 7300 that can correspond to a second cryotherapeutic-cycling stage at a first treatment site and a second overall cryotherapeutic-cycling stage of a treatment procedure. The display 7300 can include a point 7301 configured to indicate a real-time status of a cooling assembly during the cryotherapeutic-cycling stage and a plot 7302 configured to indicate the past movement of the point 7301 during the cryotherapeutic-cycling stage. The display 7300 can further include a temperature scale 7304, a time scale 7306, a temperature indicator 7308, a cycling-stage timer 7309, and a cycling-stage time indicator 7310 having a cycling-stage countdown 7312. The temperature indicator 7308 and the cycling-stage time indicator 7310 can be configured, respectively, to indicate the real-time temperature and time corresponding to the point 7301 relative to the temperature scale 7304 and the time scale 7306. The cycling-stage timer 7309 and the cycling-stage countdown 7312 can be configured, respectively, to indicate the amount of time elapsed and the amount of time remaining in the cryotherapeutic-cycling stage. The display 7300 can include boxes 7314 around portions of the plot 7302 corresponding to cooling periods of the cryotherapeutic-cycling stage. The cooling period is the time elapsed while the temperature is within a target range. The display 7300 can further include a procedure timer 7315 and a cryotherapeutic-cycling count 7316 configured to indicate, respectively, the total procedure time and the number of cryotherapeutic-cycling stages (e.g., ablations) previously completed during the treatment procedure.

As shown in FIG. 54, the display 7300 can include a movement warning 7317 configured to alert an operator when the cooling assembly is cryogenically adhered to a vessel such that movement of the cooling assembly is not desirable. In some embodiments, the movement warning 7317 can appear as a red octagon or another easily discernable indication of the desirability of reducing or eliminating movement of the cooling assembly during the period of cryogenic adhesion. The display 7300 can be an interactive touch screen and can include an operation portion 7318 having a first button 7320 and a second button 7322. The first and second buttons 7320, 7322 can be configured to stop and start cooling, respectively. In some embodiments, the first and second buttons 7320, 7322 can be radio buttons with only the button that would cause a change in operation being active at a time. The first and second buttons 7320, 7322 can allow redundant control of a cryotherapeutic device (e.g., as a backup to a user-interface device). For example, the first and second buttons 7320, 7322 can be configured for operation outside a sterile field by a secondary operator (e.g., a nurse) during a treatment procedure and a corresponding user-interface device can be configured for operation inside the sterile field by a primary operator (e.g., a doctor) during the treatment procedure.

The display 7300 can include a menu 7324 having primary stages 7326 and secondary stages 7328. In some embodiments, the secondary stages 7328 can be sub-stages of the primary stages 7326 and can be configured to appear under the primary stages 7326 only when the corresponding secondary stage 7328 is in progress. The primary and secondary stages 7326, 7328 can be highlighted when in progress and checked when completed. Movement from one primary stage 7326 to another or from one secondary stage 7328 to another can correspond to other changes in the display 7300 (e.g., refreshing the time scale 7306, refreshing the plot 7302, and/or showing or hiding the movement warning 7317).

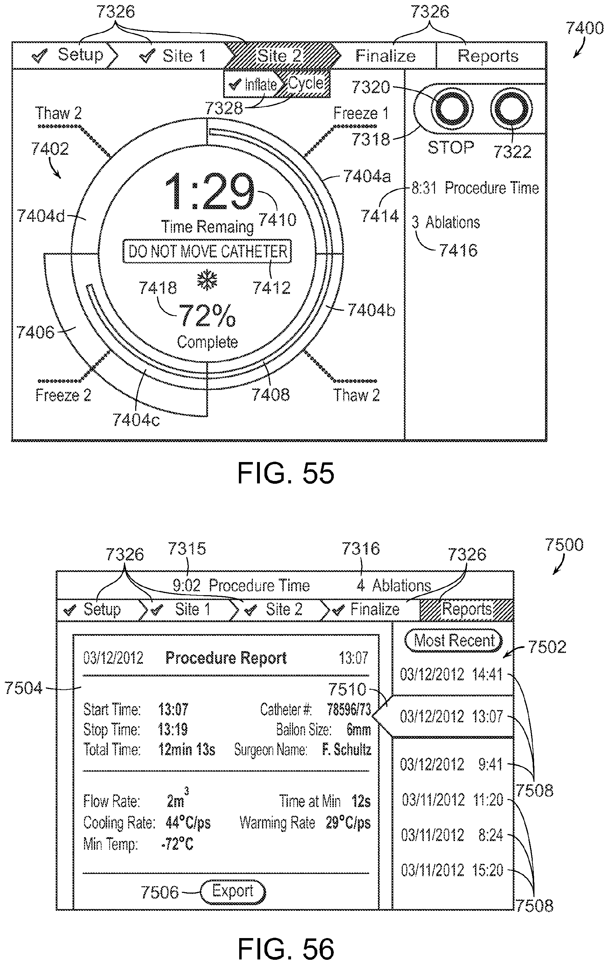

FIG. 55 is a profile view of a display 7400 that can correspond to a second cryotherapeutic-cycling stage at a second treatment site and a fourth overall cryotherapeutic-cycling stage of a treatment procedure. The display 7400 can include a circular area 7402 having a plurality of segments 7404 (individually identified as 7404a-d) corresponding to portions (e.g., cooling periods and warming) of the cryotherapeutic-cycling stage. The display 7400 can include an arc 7406 highlighting the currently active segment 7404. The display 7400 can further include a radial progress indicator 7408 configured to extend (e.g., clockwise) around the circular area 7402 as the cryotherapeutic-cycling stage progresses. Similar to the display 7300 of FIG. 54, the display 7400 can include a cycling-stage countdown 7410, a movement warning 7412, a procedure timer 7414, and a cryotherapeutic-cycling count 7416. As shown in FIG. 55, the circular area 7402 can extend around the cycling-stage countdown 7410 and the movement warning 7412, which can enhance the visibility of the cycling-stage countdown 7410 and the movement warning 7412 to an operator. The procedure timer 7414 and the cryotherapeutic-cycling count 7416 can be below the operation portion 7318. The display 7400 can further include a percentage countdown 7418 configured to indicate a percentage of the cryotherapeutic-cycling stage that has been completed.

FIG. 56 is a profile view of a display 7500 that can be a reporting interface for treatment procedures. The display 7500 can include a procedure list 7502, a procedure report 7504, and an export button 7506. The procedure list 7502 can include a plurality of procedure indicators 7508 corresponding to completed treatment procedures (e.g., completed treatment procedures using a particular cryotherapeutic system). The procedure indicators 7508 can include time information and can be organized chronologically. In other embodiments, the procedure indicators 7508 can include patient names and can be organized alphabetically, or can have other suitable identifiers and/or organizations. The procedure list 7502 can include an indicator box 7510 around the procedure indicator 7508 currently displayed in the procedure report 7504. In some embodiments, selecting (e.g., touching on a touch screen or clicking with a mouse) one of the procedure indicators 7508 can cause a corresponding treatment procedure to display as the procedure report 7504.

The procedure report 7504 can include a variety of summary information corresponding to a treatment procedure (e.g., start time, stop time, total time, patient name, operator name, device characteristics (e.g., balloon size), device identifiers (e.g., catheter serial number), and other suitable information). In some embodiments, the procedure report 7504 can include average, minimum, and/or maximum values for parameters of the treatment procedure (e.g., refrigerant flow rate, cooling rate, warming rate, blood pressure, temperature, and other suitable parameters). The procedure report 7504 can further include times at minimum, and/or maximum values of the parameters (e.g., time at the minimum temperature during the treatment procedure). Selecting the export button 7506 can cause the procedure report 7504 or multiple procedure reports 7504 corresponding to the procedure list 7502 to be transmitted (e.g., via a wired or wireless connection) to a printer or an external device. In some embodiments, the exported data for treatment procedures can include additional information (e.g., anatomical images of treatment sites, plots of temperature versus time for cryotherapeutic cycling stages performed during the treatment procedures, and/or other suitable information).

Cryotherapeutic systems configured in accordance with embodiments of the present technology can include multiple displays. For example, any of the foregoing displays 7000, 7100, 7200, 7300, 7400, and/or 7500 can be combined on a single screen of a single display device (e.g., single monitor, panel, or tablet). The displays 7000, 7100, 7200, 7300, 7400, and/or 7500 can be displayed simultaneously in any combination, or they can be displayed serially or selectably by activating a command or button that switches between one or more of the displays 7000, 7100, 7200, 7300, 7400, and/or 7500 manually or automatically. In additional examples, a first display can include primarily status information and a second display can include primarily dynamic information. As another example, a first display can be primarily for surgical planning (e.g., including an anatomical image), a second display can be primarily for monitoring system status, and a third display can be primarily for monitoring execution of cryotherapeutic periods. In some embodiments, a first display can include a primary-stage list (e.g., the primary-stage list 7102 of FIG. 52) and a second display can include a secondary-stage list (e.g., the secondary-stage list 7224 of FIG. 53). For example, the display 7100 of FIG. 52 can be used in conjunction with the display 7200 of FIG. 53. Displays configured in accordance with embodiments of the present technology can be permanently attached to, removably attached to, connected via wired connections, and/or connected via wireless connections to other cryotherapeutic-system components (e.g., consoles). Furthermore, cryotherapeutic systems configured in accordance with embodiments of the present technology can be configured to interface with centralized display systems of treatment locations (e.g., operating rooms). Furthermore, displays configured in accordance with embodiments of the present technology can include mounting elements configured to facilitate mounting on operating-room walls.

### G. Related Anatomy and Physiology

The sympathetic nervous system (SNS) is a branch of the autonomic nervous system along with the enteric nervous system and parasympathetic nervous system. It is always active at a basal level (called sympathetic tone) and becomes more active during times of stress. Like other parts of the nervous system, the SNS operates through a series of interconnected neurons. Sympathetic neurons are frequently considered part of the peripheral nervous system (PNS), although many lie within the central nervous system (CNS). Sympathetic neurons of the spinal cord (which is part of the CNS) communicate with peripheral sympathetic neurons via a series of sympathetic ganglia. Within the ganglia, spinal cord sympathetic neurons join peripheral sympathetic neurons through synapses. Spinal cord sympathetic neurons are therefore called presynaptic (or preganglionic) neurons, while peripheral sympathetic neurons are called postsynaptic (or postganglionic) neurons.

At synapses within the sympathetic ganglia, preganglionic sympathetic neurons release acetylcholine, a chemical messenger that binds and activates nicotinic acetylcholine receptors on postganglionic neurons. In response to this stimulus, postganglionic neurons principally release norepinephrine. Prolonged activation may elicit the release of adrenaline from the adrenal medulla. Once released, norepinephrine binds adrenergic receptors on peripheral tissues. Binding to adrenergic receptors causes a neuronal and hormonal response. The physiologic manifestations include pupil dilation, increased heart rate, occasional vomiting, and increased blood pressure. Increased sweating is also seen due to binding of cholinergic receptors of the sweat glands.

The SNS is responsible for up and down regulation of many homeostatic mechanisms in living organisms. Fibers from the SNS innervate tissues in almost every organ system, providing at least some regulatory function to physiological features as diverse as pupil diameter, gut motility, and urinary output. This response is also known as the sympatho-adrenal response of the body, as the preganglionic sympathetic fibers that end in the adrenal medulla (but also all other sympathetic fibers) secrete acetylcholine, which activates the secretion of adrenaline (epinephrine) and to a lesser extent noradrenaline (norepinephrine). Therefore, this response that acts primarily on the cardiovascular system is mediated directly via impulses transmitted through the SNS and indirectly via catecholamines secreted from the adrenal medulla.

Science typically looks at the SNS as an automatic regulation system, that is, one that operates without the intervention of conscious thought. Some evolutionary theorists suggest that the SNS operated in early organisms to maintain survival as the SNS is responsible for priming the body for action. One example of this priming is in the moments before waking, in which sympathetic outflow spontaneously increases in preparation for action.

### 1. The Sympathetic Chain

As shown in FIG. 57, the SNS provides a network of nerves that allows the brain to communicate with the body. Sympathetic nerves originate inside the vertebral column, toward the middle of the spinal cord in the intermediolateral cell column (or lateral horn), beginning at the first thoracic segment of the spinal cord and are thought to extend to the second or third lumbar segments. Because its cells begin in the thoracic and lumbar regions of the spinal cord, the SNS is said to have a thoracolumbar outflow. Axons of these nerves leave the spinal cord through the anterior rootlet/root. They pass near the spinal (sensory) ganglion, where they enter the anterior rami of the spinal nerves. However, unlike somatic innervation, they quickly separate out through white rami connectors that connect to either the paravertebral (which lie near the vertebral column) or prevertebral (which lie near the aortic bifurcation) ganglia extending alongside the spinal column.

In order to reach the target organs and glands, the axons travel long distances in the body. Many axon cells relay their messages to second cells through synaptic transmission. For example, the ends of axon cells can link across a space (i.e., a synapse) to dendrites of the second cell. The first cell (the presynaptic cell) can send a neurotransmitter across the synaptic cleft where it activates the second cell (the postsynaptic cell). The message is then carried to the final destination. In the SNS and other components of the PNS, these synapses are made at sites called ganglia, discussed above. The cell that sends its fiber is called a preganglionic cell, while the cell whose fiber leaves the ganglion is called a postganglionic cell. As mentioned previously, the preganglionic cells of the SNS are located between the first thoracic (T1) segment and third lumbar (L3) segments of the spinal cord. Postganglionic cells have their cell bodies in the ganglia and send their axons to target organs or glands. The ganglia include not just the sympathetic trunks but also the cervical ganglia (superior, middle, and inferior), which send sympathetic nerve fibers to the head and thorax organs, and the celiac and mesenteric ganglia, which send sympathetic fibers to the gut.

### 2. Innervation of the Kidneys

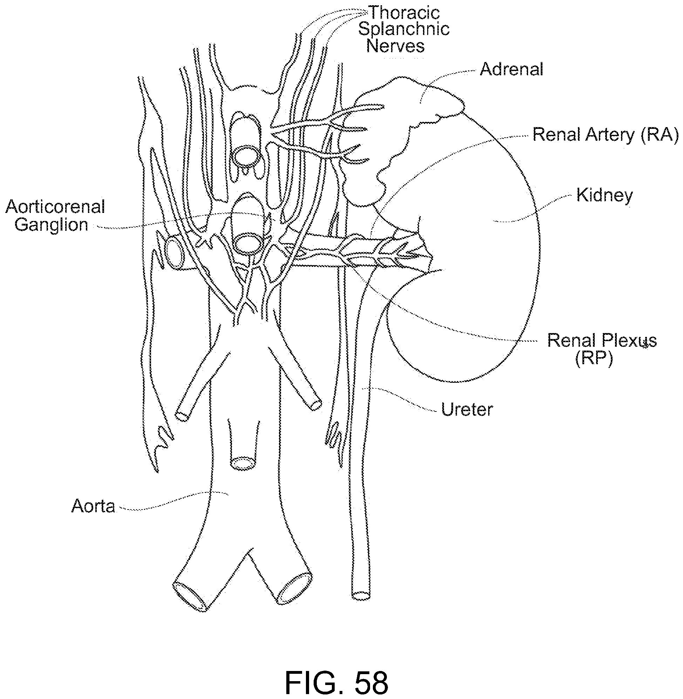

As FIG. 58 shows, the kidney is innervated by the renal plexus, which is intimately associated with the renal artery. The renal plexus is an autonomic plexus that surrounds the renal artery and is embedded within the adventitia of the renal artery. The renal plexus extends along the renal artery until it arrives at the substance of the kidney. Fibers contributing to the renal plexus arise from the celiac ganglion, the superior mesenteric ganglion, the aorticorenal ganglion and the aortic plexus. The renal plexus, also referred to as the renal nerve, is predominantly comprised of sympathetic components. There is no (or at least very minimal) parasympathetic innervation of the kidney.

Preganglionic neuronal cell bodies are located in the intermediolateral cell column of the spinal cord. Preganglionic axons pass through the paravertebral ganglia (they do not synapse) to become the lesser splanchnic nerve, the least splanchnic nerve, the first lumbar splanchnic nerve, and the second lumbar splanchnic nerve, and they travel to the celiac ganglion, the superior mesenteric ganglion, and the aorticorenal ganglion. Postganglionic neuronal cell bodies exit the celiac ganglion, the superior mesenteric ganglion, and the aorticorenal ganglion to the renal plexus and are distributed to the renal vasculature.

### 3. Renal Sympathetic Neural Activity

Messages travel through the SNS in a bidirectional flow. Efferent messages may trigger changes in different parts of the body simultaneously. For example, the SNS may accelerate heart rate, widen bronchial passages, decrease motility (movement) of the large intestine, constrict blood vessels, increase peristalsis in the esophagus, cause pupil dilation, cause piloerection (i.e., goose bumps), cause perspiration (i.e., sweating), and raise blood pressure. Afferent messages carry signals from various organs and sensory receptors in the body to other organs and, particularly, the brain.

Hypertension, heart failure, and chronic kidney disease are a few of many disease states that result from chronic activation of the SNS, especially the renal sympathetic nervous system. Chronic activation of the SNS is a maladaptive response that drives the progression of these disease states. Pharmaceutical management of the renin-angiotensin-aldosterone system (RAAS) has been a longstanding, but somewhat ineffective, approach for reducing overactivity of the SNS.

As mentioned above, the renal sympathetic nervous system has been identified as a major contributor to the complex pathophysiology of hypertension, states of volume overload (such as heart failure), and progressive renal disease, both experimentally and in humans. Studies employing radiotracer dilution methodology to measure overflow of norepinephrine from the kidneys to plasma revealed increased renal norepinephrine spillover rates in patients with essential hypertension, particularly so in young hypertensive subjects, which in concert with increased norepinephrine spillover from the heart, is consistent with the hemodynamic profile typically seen in early hypertension and characterized by an increased heart rate, cardiac output, and renovascular resistance. It is now known that essential hypertension is commonly neurogenic, often accompanied by pronounced SNS overactivity.

Activation of cardiorenal sympathetic nerve activity is even more pronounced in heart failure, as demonstrated by an exaggerated increase of norepinephrine overflow from the heart and the kidneys to plasma in this patient group. In line with this notion is the recent demonstration of a strong negative predictive value of renal sympathetic activation on all-cause mortality and heart transplantation in patients with congestive heart failure, which is independent of overall sympathetic activity, glomerular filtration rate, and left ventricular ejection fraction. These findings support the notion that treatment regimens that are designed to reduce renal sympathetic stimulation have the potential to improve survival in patients with heart failure.

Both chronic and end-stage renal disease are characterized by heightened sympathetic nervous activation. In patients with end-stage renal disease, plasma levels of norepinephrine above the median have been demonstrated to be predictive for both all-cause death and death from cardiovascular disease. This is also true for patients suffering from diabetic or contrast nephropathy. There is compelling evidence suggesting that afferent signals originating from the diseased kidneys are major contributors to initiating and sustaining elevated central sympathetic outflow in this patient group. This facilitates the occurrence of the well-known adverse consequences of chronic sympathetic overactivity, such as hypertension, left ventricular hypertrophy, ventricular arrhythmias, sudden cardiac death, insulin resistance, diabetes, and metabolic syndrome.

### (i) Renal Sympathetic Efferent Nerve Activity

Sympathetic nerves to the kidneys terminate in the blood vessels, the juxtaglomerular apparatus and the renal tubules. Stimulation of the renal sympathetic nerves causes increased renin release, increased sodium (Na⁺) reabsorption, and a reduction of renal blood flow. These components of the neural regulation of renal function are considerably stimulated in disease states characterized by heightened sympathetic tone and clearly contribute to the rise in blood pressure in hypertensive patients. The reduction of renal blood flow and glomerular filtration rate as a result of renal sympathetic efferent stimulation is likely a cornerstone of the loss of renal function in cardio-renal syndrome, which is renal dysfunction as a progressive complication of chronic heart failure, with a clinical course that typically fluctuates with the patient's clinical status and treatment. Pharmacologic strategies to thwart the consequences of renal efferent sympathetic stimulation include centrally acting sympatholytic drugs, beta blockers (intended to reduce renin release), angiotensin converting enzyme inhibitors and receptor blockers (intended to block the action of angiotensin II and aldosterone activation consequent to renin release), and diuretics (intended to counter the renal sympathetic mediated sodium and water retention). However, the current pharmacologic strategies have significant limitations including limited efficacy, compliance issues, side effects, and others.

### (ii) Renal Afferent Nerve Activity

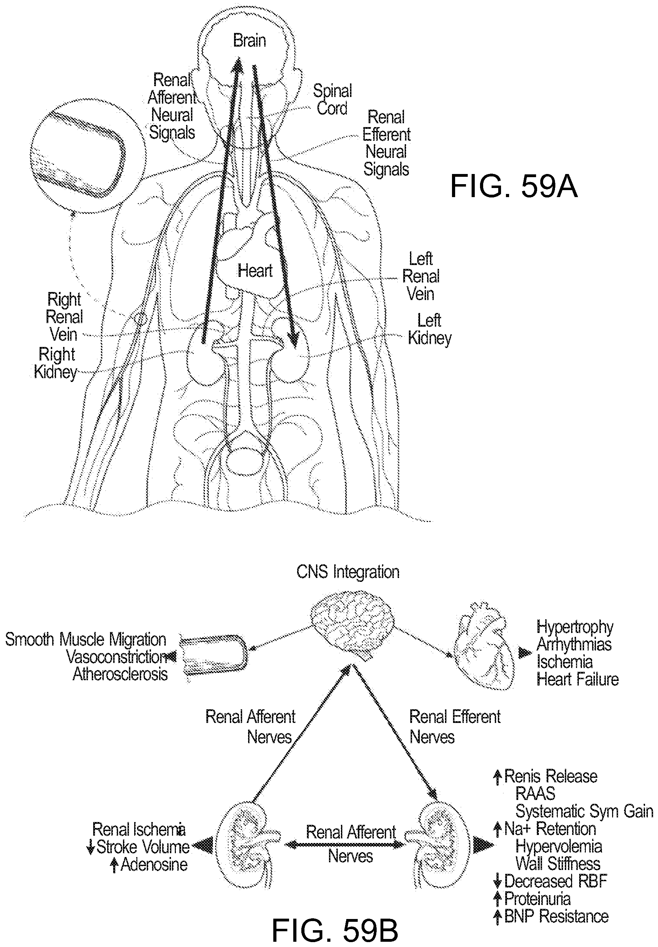

The kidneys communicate with integral structures in the CNS via renal afferent nerves. Several forms of "renal injury" may induce activation of afferent signals. For example, renal ischemia, reduction in stroke volume or renal blood flow, or an abundance of adenosine enzyme may trigger activation of afferent neural communication. As shown in FIGS. 59A and 59B, this afferent communication might be from the kidney to the brain or might be from one kidney to the other kidney (via the CNS). These afferent signals are centrally integrated and may result in increased sympathetic outflow. This sympathetic drive is directed toward the kidneys, thereby activating the RAAS and inducing increased renin secretion, sodium retention, volume retention, and vasoconstriction. Central sympathetic overactivity also impacts other organs and bodily structures innervated by sympathetic nerves such as the heart and the peripheral vasculature, resulting in the described adverse effects of sympathetic activation, several aspects of which also contribute to the rise in blood pressure.

The physiology therefore suggests that (a) modulation of tissue with efferent sympathetic nerves will reduce inappropriate renin release, salt retention, and reduction of renal blood flow, and (b) modulation of tissue with afferent nerves will reduce the systemic contribution to hypertension and other disease states associated with increased central sympathetic tone through its direct effect on the posterior hypothalamus as well as the contralateral kidney. In addition to the central hypotensive effects of afferent renal denervation, a desirable reduction of central sympathetic outflow to various other sympathetically innervated organs such as the heart and the vasculature is anticipated.

### 4. Additional Clinical Benefits of Renal Denervation

As provided above, renal denervation is likely to be valuable in the treatment of several clinical conditions characterized by increased overall and particularly renal sympathetic activity such as hypertension, metabolic syndrome, insulin resistance, diabetes, left ventricular hypertrophy, chronic end-stage renal disease, inappropriate fluid retention in heart failure, cardio-renal syndrome, and sudden death. Since the reduction of afferent neural signals contributes to the systemic reduction of sympathetic tone/drive, renal denervation might also be useful in treating other conditions associated with systemic sympathetic hyperactivity. Accordingly, renal denervation may also benefit other organs and bodily structures innervated by sympathetic nerves, including those identified in FIG. 57. For example, as previously discussed, a reduction in central sympathetic drive may reduce the insulin resistance that afflicts people with metabolic syndrome and Type II diabetes. Additionally, patients with osteoporosis are also sympathetically activated and might also benefit from the down regulation of sympathetic drive that accompanies renal denervation.

### 5. Achieving Intravascular Access to the Renal Artery

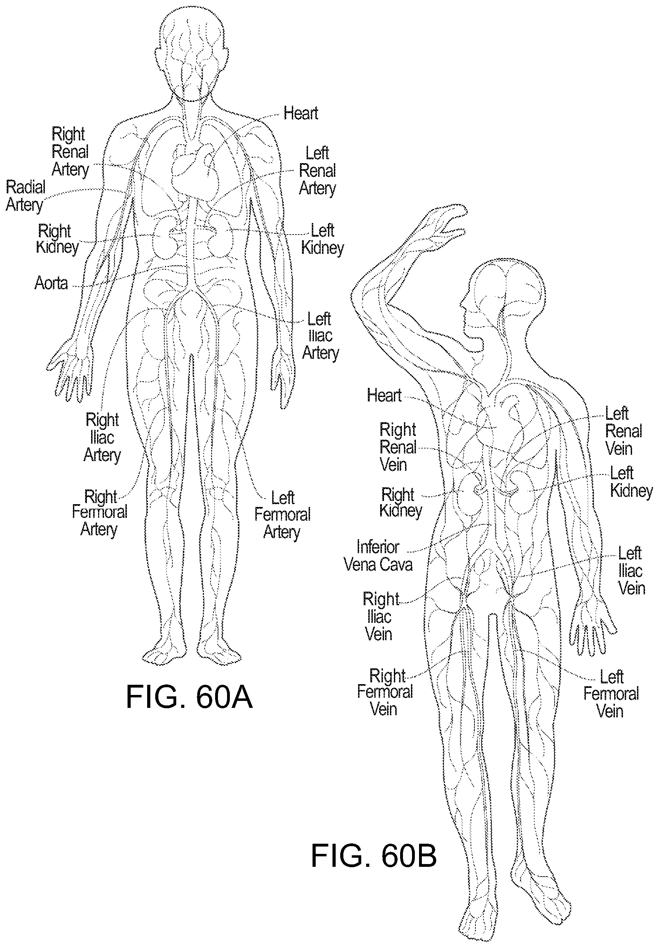

In accordance with the present technology, neuromodulation of a left and/or right renal plexus, which is intimately associated with a left and/or right renal artery, may be achieved through intravascular access. As FIG. 60A shows, blood moved by contractions of the heart is conveyed from the left ventricle of the heart by the aorta. The aorta descends through the thorax and branches into the left and right renal arteries. Below the renal arteries, the aorta bifurcates at the left and right iliac arteries. The left and right iliac arteries descend, respectively, through the left and right legs and join the left and right femoral arteries.

As FIG. 60B shows, the blood collects in veins and returns to the heart, through the femoral veins into the iliac veins and into the inferior vena cava. The inferior vena cava branches into the left and right renal veins. Above the renal veins, the inferior vena cava ascends to convey blood into the right atrium of the heart. From the right atrium, the blood is pumped through the right ventricle into the lungs, where it is oxygenated. From the lungs, the oxygenated blood is conveyed into the left atrium. From the left atrium, the oxygenated blood is conveyed by the left ventricle back to the aorta.

As will be described in greater detail later, the femoral artery may be accessed and cannulated at the base of the femoral triangle just inferior to the midpoint of the inguinal ligament. A catheter may be inserted percutaneously into the femoral artery through this access site, passed through the iliac artery and aorta, and placed into either the left or right renal artery. This comprises an intravascular path that offers minimally invasive access to a respective renal artery and/or other renal blood vessels.

The wrist, upper arm, and shoulder region provide other locations for introduction of catheters into the arterial system. For example, catheterization of either the radial, brachial, or axillary artery may be utilized in select cases. Catheters introduced via these access points may be passed through the subclavian artery on the left side (or via the subclavian and brachiocephalic arteries on the right side), through the aortic arch, down the descending aorta and into the renal arteries using standard angiographic technique.

### 6. Properties and Characteristics of the Renal Vasculature

Since neuromodulation of a left and/or right renal plexus may be achieved in accordance with embodiments of the present technology through intravascular access, properties and characteristics of the renal vasculature may impose constraints upon and/or inform the design of apparatus, systems, and methods for achieving such renal neuromodulation. Some of these properties and characteristics may vary across the patient population and/or within a specific patient across time, as well as in response to disease states, such as hypertension, chronic kidney disease, vascular disease, end-stage renal disease, insulin resistance, diabetes, metabolic syndrome, etc. These properties and characteristics, as explained herein, may have bearing on the efficacy of the procedure and the specific design of the intravascular device. Properties of interest may include, for example, material/mechanical, spatial, fluid dynamic/hemodynamic, and/or thermodynamic properties.

As discussed previously, a catheter may be advanced percutaneously into either the left or right renal artery via a minimally invasive intravascular path. However, minimally invasive renal arterial access may be challenging, for example, because as compared to some other arteries that are routinely accessed using catheters, the renal arteries are often extremely tortuous, may be of relatively small diameter, and/or may be of relatively short length. Furthermore, renal arterial atherosclerosis is common in many patients, particularly those with cardiovascular disease. Renal arterial anatomy also may vary significantly from patient to patient, which further complicates minimally invasive access. Significant inter-patient variation may be seen, for example, in relative tortuosity, diameter, length, and/or atherosclerotic plaque burden, as well as in the take-off angle at which a renal artery branches from the aorta. Apparatus, systems, and methods for achieving renal neuromodulation via intravascular access can account for these and other aspects of renal arterial anatomy and its variations across the patient population when minimally invasively accessing a renal artery.

In addition to complicating renal arterial access, specifics of the renal anatomy also complicate establishment of stable contact between neuromodulatory apparatus and a luminal surface or wall of a renal artery. When the neuromodulatory apparatus includes a cryotherapeutic device, consistent positioning, appropriate contact force applied by the cryotherapeutic device to the vessel wall, and adhesion between the cryo-applicator and the vessel wall can be important for predictability. However, navigation can be impeded by the tight space within a renal artery, as well as tortuosity of the artery. Furthermore, establishing consistent contact can be complicated by patient movement, respiration, and/or the cardiac cycle because these factors may cause significant movement of the renal artery relative to the aorta, and the cardiac cycle may transiently distend the renal artery (i.e., cause the wall of the artery to pulse).

After accessing a renal artery and facilitating stable contact between a neuromodulatory apparatus and a luminal surface of the artery, nerves in and around the adventitia of the artery can be modulated via the neuromodulatory apparatus. Effectively applying thermal treatment from within a renal artery is non-trivial given the potential clinical complications associated with such treatment. For example, the intima and media of the renal artery are highly vulnerable to thermal injury. As discussed in greater detail below, the intima-media thickness separating the vessel lumen from its adventitia means that target renal nerves may be multiple millimeters distant from the luminal surface of the artery. Sufficient energy can be delivered to or heat removed from the target renal nerves to modulate the target renal nerves without excessively cooling or heating the vessel wall to the extent that the wall is frozen, desiccated, or otherwise potentially affected to an undesirable extent. A potential clinical complication associated with excessive heating is thrombus formation from coagulating blood flowing through the artery. Given that this thrombus may cause a kidney infarct, thereby causing irreversible damage to the kidney, thermal treatment from within the renal artery can be applied carefully. Accordingly, the complex fluid mechanics and thermodynamic conditions present in the renal artery during treatment, particularly those that may impact heat transfer dynamics at the treatment site, may be important in applying energy (e.g., heating thermal energy) and/or removing heat from the tissue (e.g., cooling thermal conditions) from within the renal artery.

The neuromodulatory apparatus can be configured to allow for adjustable positioning and repositioning of an energy delivery element within the renal artery since location of treatment may also impact clinical efficacy. For example, it may be tempting to apply a full circumferential treatment from within the renal artery given that the renal nerves may be spaced circumferentially around a renal artery. In some situations, full-circle lesions likely resulting from a continuous circumferential treatment may be potentially related to renal artery stenosis. Therefore, the formation of more complex lesions along a longitudinal dimension of the renal artery via the cryotherapeutic devices and/or repositioning of the neuromodulatory apparatus to multiple treatment locations may be desirable. It should be noted, however, that a benefit of creating a circumferential ablation may outweigh the potential of renal artery stenosis, or the risk may be mitigated with certain embodiments or in certain patients, and creating a circumferential ablation could be a goal. Additionally, variable positioning and repositioning of the neuromodulatory apparatus may prove to be useful in circumstances where the renal artery is particularly tortuous or where there are proximal branch vessels off the renal artery main vessel, making treatment in certain locations challenging. Manipulation of a device in a renal artery can also consider mechanical injury imposed by the device on the renal artery. Motion of a device in an artery, for example by inserting, manipulating, negotiating bends and so forth, may contribute to dissection, perforation, denuding intima, or disrupting the interior elastic lamina.

Blood flow through a renal artery may be temporarily occluded for a short time with minimal or no complications. However, occlusion for a significant amount of time can be avoided in some cases to prevent injury to the kidney such as ischemia. It can be beneficial to avoid occlusion altogether or, if occlusion is beneficial, to limit the duration of occlusion, for example, to 2-5 minutes.

Based on the above-described challenges of (1) renal artery intervention, (2) consistent and stable placement of the treatment element against the vessel wall, (3) effective application of treatment across the vessel wall, (4) positioning and potentially repositioning the treatment apparatus to allow for multiple treatment locations, and (5) avoiding or limiting duration of blood flow occlusion, various independent and dependent properties of the renal vasculature that may be of interest include, for example, (a) vessel diameter, vessel length, intima-media thickness, coefficient of friction, and tortuosity, (b) distensibility, stiffness, and modulus of elasticity of the vessel wall, (c) peak systolic, end-diastolic blood flow velocity, as well as the mean systolic-diastolic peak blood flow velocity, and mean/max volumetric blood flow rate, (d) specific heat capacity of blood and/or of the vessel wall, thermal conductivity of blood and/or of the vessel wall, and/or thermal convectivity of blood flow past a vessel wall treatment site and/or radiative heat transfer, (e) renal artery motion relative to the aorta induced by respiration, patient movement, and/or blood flow pulsatility, and (f) the takeoff angle of a renal artery relative to the aorta. These properties will be discussed in greater detail with respect to the renal arteries. However, dependent on the apparatus, systems, and methods utilized to achieve renal neuromodulation, such properties of the renal arteries also may guide and/or constrain design characteristics.

As noted above, an apparatus positioned within a renal artery can conform to the geometry of the artery. Renal artery vessel diameter, D_{RA}, typically is in a range of about 2-10 mm, with most of the patient population having a D_{RA} of about 4 mm to about 8 mm and an average of about 6 mm. Renal artery vessel length, L_{RA}, between its ostium at the aorta/renal artery juncture and its distal branchings, generally is in a range of about 5-70 mm, and a significant portion of the patient population is in a range of about 20-50 mm. Since the target renal plexus is embedded within the adventitia of the renal artery, the composite intima-media thickness (i.e., the radial outward distance from the artery's luminal surface to the adventitia containing target neural structures) also is notable and generally is in a range of about 0.5-2.5 mm, with an average of about 1.5 mm. Although a certain depth of treatment can be important to reach the target neural fibers, the treatment typically is not too deep (e.g., the treatment can be less than about 5 mm from inner wall of the renal artery) so as to avoid non-target tissue and anatomical structures such as the renal vein.

An additional property of the renal artery that may be of interest is the degree of renal motion relative to the aorta induced by respiration and/or blood flow pulsatility. A patient's kidney, which is located at the distal end of the renal artery, may move as much as four includes cranially with respiratory excursion. This may impart significant motion to the renal artery connecting the aorta and the kidney. Accordingly, the neuromodulatory apparatus can have a unique balance of stiffness and flexibility to maintain contact between a cryo-applicator or another thermal treatment element and the vessel wall during cycles of respiration. Furthermore, the takeoff angle between the renal artery and the aorta may vary significantly between patients, and also may vary dynamically within a patient (e.g., due to kidney motion). The takeoff angle generally may be in a range of about 30°-135°.

The foregoing embodiments of cryotherapeutic devices are configured to accurately position the cryo-applicators in and/or near the renal artery and/or renal ostium via a femoral approach, transradial approach, or another suitable vascular approach. In any of the foregoing embodiments described above with reference to FIGS. 1A-67, single balloons can be configured to be inflated to diameters of about 3 mm to about 8 mm, and multiple balloons can collectively be configured to be inflated to diameters of about 3 mm to about 8 mm, and in several embodiments 4 mm to 8 mm. Additionally, in any of the embodiments described herein with reference to FIGS. 1A-67, the balloons can individually and/or collectively have a length of about 3 mm to about 15 mm, and in several embodiments about 5 mm. For example, several specific embodiments of the devices shown in FIGS. 1A-67 can have a 5 mm long balloon that is configured to be inflated to a diameter of 4 mm to 8 mm. The shaft of the devices described above with reference to any of the embodiments shown in FIGS. 1A-67 can be sized to fit within a 6 Fr sheath, such as a 4 Fr shaft size.

### H. Examples

1. A cryotherapeutic system, comprising:
   a cartridge housing;
   a cartridge connector adjacent to the cartridge housing;
   a supply passage fluidly connected to the cartridge connector;
   a supply valve along the supply passage; and
   a control assembly including an actuator and a user interface, wherein the actuator is operably connected to the supply valve, and the control assembly is configured to signal the actuator to open the supply valve in response to a signal from the user interface.
2. The cryotherapeutic system of example 1, wherein the control assembly further includes a supply sensor operably connected to the supply passage and configured to detect a pressure within a portion of the supply passage between the cartridge connector and the supply valve, the user interface includes an initiation switch, and the control assembly is configured to override the initiation switch if the pressure is below a threshold value.
3. The cryotherapeutic system of example 1, further comprising a pressure-monitoring chamber fluidly separate from the supply passage, wherein the control assembly further includes a pressure-monitoring sensor operably connected to the pressure-monitoring chamber and configured to detect a pressure within the pressure-monitoring chamber, and the control assembly is configured to signal the actuator to close the supply valve if the pressure is greater than a threshold value.
4. The cryotherapeutic system of example 1, wherein the control assembly further includes a pressure-compensated flow regulator operably connected to the supply valve.
5. The cryotherapeutic system of example 1, wherein the user interface includes a remote control unit
6. The cryotherapeutic system of example 1, further comprising battery leads operably connected to the control assembly.
7. The cryotherapeutic system of example 1, wherein the cartridge connector includes a coupling member configured to pierce a membrane, open a check valve, or both.
8. The cryotherapeutic system of example 7, wherein the cartridge connector further includes a gasket around the coupling member, the gasket has a compressed state and an uncompressed state, the coupling member is recessed relative to the gasket in the uncompressed state, and the coupling member is protruding relative to the gasket in the compressed state.
9. The cryotherapeutic system of example 8, wherein the coupling member is a first coupling member, the cryotherapeutic system further comprises a cartridge having a second coupling member including a membrane, a check valve, or both, the cartridge further includes a contact portion around the second coupling member, and the contact portion is configured to press the gasket from the uncompressed state to the compressed state.
10. The cryotherapeutic system of example 9, wherein the cartridge has an internal volume between about 5 cc and about 500 cc.
11. The cryotherapeutic system of example 1, further comprising an exhaust passage and an exhaust portal, wherein the exhaust passage has an outlet end, and the exhaust portal is at the outlet end.
12. The cryotherapeutic system of example 11, wherein the exhaust portal is open to the atmosphere.
13. The cryotherapeutic system of example 11, wherein the exhaust passage is proximate the actuator such that transporting refrigerant exhaust through the exhaust passage during operation of the actuator reduces the operating temperature of the actuator.
14. The cryotherapeutic system of example 11, wherein the exhaust passage is proximate the cartridge housing such that transporting refrigerant exhaust through the exhaust passage when a cartridge is within the cartridge housing cools the cartridge.
15. The cryotherapeutic system of example 11, wherein the control assembly further includes a supply sensor operably connected to the supply passage and configured to detect a flow rate, a pressure, or both, of refrigerant within the supply passage, and an exhaust sensor operably connected to the exhaust passage and configured to detect a flow rate, a pressure, or both, of refrigerant within the exhaust passage.
16. The cryotherapeutic system of example 15, wherein the control assembly is configured to signal the actuator to close the supply valve if a difference between a first phase-independent flow rate or pressure within the supply passage and a second phase-independent flow rate or pressure within the exhaust passage, respectively, is greater than a threshold value.
17. The cryotherapeutic system of example 1, further comprising a filter fluidly connected to the supply passage.
18. The cryotherapeutic system of example 17, wherein the filter is a desiccating filter.
19. The cryotherapeutic system of example 17, wherein the filter includes a molecular sieve.
20. The cryotherapeutic system of example 17, wherein the filter is a first filter, the cryotherapeutic system further comprises a second filter fluidly connected to the supply passage between the cartridge connector and the first filter, the first filter is a desiccating filter, and the second filter is a particulate filter.
21. The cryotherapeutic system of example 20, further comprising a third filter fluidly connected to the supply passage distal from the first and second filters relative to the cartridge connector, wherein the third filter is a particulate filter.
22. The cryotherapeutic system of example 1, further comprising a sterilizer operably connected to the supply passage.
23. The cryotherapeutic system of example 22, wherein the sterilizer includes a radiation source.
24. The cryotherapeutic system of example 23, wherein the radiation source includes an ultraviolet light source.
25. The cryotherapeutic system of example 1, wherein the user interface includes an initiation switch, and the control assembly includes a timer triggered by the initiation switch.
26. The cryotherapeutic system of example 25, wherein the control assembly is configured to signal the actuator to open the supply valve in response to a signal from the initiation switch, and the control assembly is configured to signal the actuator to close the supply valve in response to a signal from the timer.
27. The cryotherapeutic system of example 25, wherein the timer is a mechanical timer.
28. The cryotherapeutic system of example 1, further comprising a pressure-relief passage, a pressure-relief portal, and a pressure-relief valve along the pressure-relief passage, wherein the pressure-relief passage has an outlet end, the pressure-relief portal is at the outlet end, and the pressure-relief passage or the pressure-relief valve is fluidly connected to the supply passage at a connection point between the cartridge connector and the supply valve.
29. The cryotherapeutic system of example 28, further comprising an isolation valve along the supply passage between the connection point and the supply valve, wherein the isolation valve defaults to a closed position, and the pressure-relief-valve defaults to an open position.
30. The cryotherapeutic system of example 1, further comprising a heater operably connected to the cartridge housing and configured to heat a cartridge within the cartridge housing.
31. The cryotherapeutic system of example 30, wherein the control assembly further includes a cartridge sensor configured to detect a temperature of a cartridge within the cartridge housing, and the control assembly is configured to activate the heater if the temperature is below a threshold value.
32. The cryotherapeutic system of example 31, wherein the user interface includes an initiation switch, and the control assembly is configured to override the initiation switch if the temperature is below the threshold value.
33. The cryotherapeutic system of example 1, wherein the cartridge housing includes a main portion and a lid removably connectable to the main portion.
34. The cryotherapeutic system of example 33, further comprising a console including the cartridge housing, wherein the cartridge housing has a first end portion and a second end portion opposite the first end portion, the lid is at the first end portion, the cartridge connector is at the second end portion, and the second end portion is lower than the first end portion when the console is upright on a flat surface.
35. The cryotherapeutic system of example 33, wherein the cartridge connector includes a first coupling member configured to pierce a membrane, open a check valve, or both, the cryotherapeutic system further comprises a cartridge having a second coupling member including a membrane, a check valve, or both, the cartridge is shaped to fit within the cartridge housing, and securing the lid to the main portion forces the second coupling member toward the first coupling member.
36. The cryotherapeutic system of example 35, further comprising a console including the cartridge housing, wherein the second coupling member is at a lowermost portion of the cartridge when the cartridge is within the cartridge housing, the lid is secured to the main portion, and the console is upright on a flat surface.
37. The cryotherapeutic system of example 35, wherein the cartridge has an internal volume between about 5 cc and about 500 cc.
38. The cryotherapeutic system of example 35, wherein the lid has a first coupling member, the main portion has a second coupling member, and engaging the first and second coupling members releasably locks the lid to the main portion.
39. The cryotherapeutic system of example 38, wherein the first and second coupling members include threading.
40. The cryotherapeutic system of example 38, wherein the first and second coupling members are portions of a latch clamp.
41. The cryotherapeutic system of example 1, further comprising a first outlet adapter, wherein the supply passage has a first end and a second end opposite the first end, the cartridge connector is at the first end, and the first outlet adapter is at the second end.
42. The cryotherapeutic system of example 41, further comprising an exhaust passage and a first inlet adapter, wherein the first outlet adapter is proximate the first inlet adapter.
43. The cryotherapeutic system of example 42, further comprising a pressure-monitoring chamber and pressure-monitoring adapter, wherein the pressure-monitoring chamber is fluidly separate from the refrigerant supply passage and the exhaust passage, and the pressure-monitoring adapter is proximate the first outlet adapter and the first inlet adapter.
44. The cryotherapeutic system of example 43, wherein the supply passage is a first supply passage, the cryotherapeutic system further comprises a second supply passage and a second outlet adapter, wherein the second supply passage has a first end and a second end opposite the first end, the first end of the second supply passage is fluidly connected to the first supply passage, the second outlet adapter is at the second end of the second supply passage, and the second outlet adapter is proximate the first outlet adapter, the first inlet adapter, and the pressure-monitoring adapter.
45. The cryotherapeutic system of example 44, further comprising a first umbilical connector, wherein the pressure-monitoring adapter is a first pressure-monitoring adapter, the first umbilical connector includes the first outlet adapter, the first inlet adapter, the first pressure-monitoring adapter, and the second outlet adapter, the cryotherapeutic system further comprises a second umbilical connector removably connectable to the first umbilical connector, and the second umbilical connector includes a second inlet adapter, a third outlet adapter, a second pressure-monitoring adapter, and a third inlet adapter removably connectable to the first outlet adapter, the first inlet adapter, the first pressure-monitoring adapter, and the second outlet adapter, respectively.
46. The cryotherapeutic system of example 45, wherein the second umbilical connector is disposable.
47. The cryotherapeutic system of example 1, further comprising a disposable bag configured to form a sterile barrier around at least a portion of the cryotherapeutic system.
48. The cryotherapeutic system of example 47, further comprising a cartridge within the bag, wherein the bag is sufficiently deformable to allow the cartridge to be grasped through the bag and moved into or out of the cartridge housing within the bag.
49. The cryotherapeutic system of example 48, wherein the cartridge has an internal volume between about 5 cc and about 500 cc.
50. The cryotherapeutic system of example 48, wherein the cartridge is a first cartridge and the cryotherapeutic system further comprises a second cartridge within the bag.
51. The cryotherapeutic system of example 47, wherein the cartridge housing includes a main portion and a lid removably connectable to the main portion, the bag includes a collar shaped to fit snugly around the cartridge housing, and the lid is disposable.
52. The cryotherapeutic system of example 47, wherein the cryotherapeutic system includes a power cord and the bag includes an elongated extension configured to form a sterile barrier around at least a portion of the power cord.
53. The cryotherapeutic system of example 47, further comprising an outlet adapter, wherein the supply passage has a first end and a second end opposite the first end, the cartridge connector is at the first end, the outlet adapter is at the second end, and the bag further includes a sterile-barrier portal configured to receive an inlet adapter removably connectable to the outlet adapter generally without disrupting the sterile barrier.
54. The cryotherapeutic system of example 1, further comprising a shell configured to form a sterile barrier around at least a portion of the cryotherapeutic system.
55. The cryotherapeutic system of example 54, wherein the shell includes a deformable membrane proximate to at least a portion of the user interface when the user interface is within the shell.
56. The cryotherapeutic system of example 54, wherein the shell includes a sterile-barrier portal proximate the cartridge housing when the cartridge housing is within the shell, and the sterile-barrier portal is configured to receive a cartridge generally without disrupting the sterile barrier.
57. The cryotherapeutic system of example 56, further comprising a sterile package including a disposable cartridge having an internal volume between about 5 cc and about 500 cc.
58. The cryotherapeutic system of example 54, wherein the shell includes a base and a cover removably connectable to the base.
59. The cryotherapeutic system of example 58, wherein the base includes recesses configured to fit over a patient's legs.
60. The cryotherapeutic system of example 1, further comprising a primary housing including the cartridge housing and configured to attach to an edge portion of a surgical table.
61. The cryotherapeutic system of example 60, wherein the primary housing has an upper portion including a user interface and a lower portion including the cartridge housing.
62. The cryotherapeutic system of example 60, further comprising a drape configured to form a sterile barrier over the primary housing.
63. A cryotherapeutic system, comprising:
   a cartridge connector having a first coupling member and a first locking member, wherein the first coupling member is configured to pierce a membrane, open a check valve, or both;
   a cartridge including a second coupling member and a second locking member, wherein the second coupling member includes a membrane, a check valve, or both, and the second locking member is configured to engage the first locking member to releasably lock the cartridge in a fixed position relative to the cartridge connector; and
   a supply passage fluidly connected to the cartridge connector.
64. The cryotherapeutic system of example 63, wherein the first and second locking members include threading.
65. The cryotherapeutic system of example 63, wherein the first and second locking members are portions of a spring-lock mechanism, and the cryotherapeutic system further includes a release switch operably connected to the spring-lock mechanism and configured to release the spring-lock mechanism so that the cartridge can be moved away from the cartridge connector.
66. The cryotherapeutic system of example 63, wherein the cartridge is disposable and has an internal volume between about 5 cc and about 500 cc.
67. The cryotherapeutic system of example 63, wherein the cartridge includes a lid.
68. A cryotherapeutic system, comprising:
   a cartridge housing;
   a cartridge connector adjacent to the cartridge housing; and
   a supply passage fluidly connected to the cartridge connector, wherein the cryotherapeutic system is handheld.
69. The cryotherapeutic system of example 68, wherein the cryotherapeutic system does not include a connection to an external power source.
70. The cryotherapeutic system of example 68, wherein the cryotherapeutic system is disposable.
71. A cryotherapeutic system, comprising:
   a cartridge connector;
   a supply passage fluidly connected to the cartridge connector; and
   a desiccating filter fluidly connected to the supply passage.
72. A cryotherapeutic system, comprising:
   a cryotherapeutic device including an elongated shaft having a distal portion, wherein the shaft is configured to locate the distal portion intravascularly at a treatment site in or otherwise proximate a renal artery or renal ostium; and
   a console operably connected to the cryotherapeutic device, the console including a cartridge connector and a supply passage fluidly connected to the cartridge connector.
73. The cryotherapeutic system of example 72, wherein the console further includes a first umbilical connector including a first outlet adapter, the system further includes a second umbilical connector operably connected to the elongated shaft and removably connectable to the first umbilical connector, the supply passage has a first end and a second end opposite the first end, the cartridge connector is at the first end, and the first outlet adapter is at the second end.
74. The cryotherapeutic system of example 72, further comprising a sterile package including a cartridge, wherein the cartridge is disposable and has an internal volume between about 5 cc and about 500 cc.
75. The cryotherapeutic system of example 72, further comprising a sterile package including the elongated shaft, wherein the elongated shaft is disposable.
76. The cryotherapeutic system of example 72, further comprising a sterile package including the console, wherein the console is disposable.
77. The cryotherapeutic system of example 72, wherein the cryotherapeutic system is generally self-contained for therapeutically effective cryogenic renal neuromodulation when the console includes a cartridge.

### I. Conclusion

The above detailed descriptions of embodiments of the present technology are for purposes of illustration only and are not intended to be exhaustive or to limit the present technology to the precise form(s) disclosed above. Various equivalent modifications are possible within the scope of the present technology, as those skilled in the relevant art will recognize. For example, while stages may be presented in a given order, alternative embodiments may perform stages in a different order. The various embodiments described herein and elements thereof may also be combined to provide further embodiments. In some cases, well-known structures and functions have not been shown or described in detail to avoid unnecessarily obscuring the description of embodiments of the present technology.

Where the context permits, singular or plural terms may also include the plural or singular terms, respectively. Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the terms "comprising" and the like are used throughout the disclosure to mean including at least the recited feature(s) such that any greater number of the same feature(s) and/or additional types of other features are not precluded. It will also be appreciated that various modifications may be made to the described embodiments without deviating from the present technology. Further, while advantages associated with certain embodiments of the present technology have been described in the context of those embodiments, other embodiments may also exhibit such advantages, and not all embodiments need necessarily exhibit such advantages to fall within the scope of the present technology. Accordingly, the disclosure and associated technology can encompass other embodiments not expressly shown or described herein.

## Claims

1. A cryotherapeutic system, comprising:
a cartridge housing;
a cartridge connector adjacent to the cartridge housing;
a supply passage fluidly connected to the cartridge connector;
a supply valve along the supply passage; and
a control assembly including an actuator and a user interface, wherein the actuator is operably connected to the supply valve, and the control assembly is configured to signal the actuator to open the supply valve in response to a signal from the user interface,
wherein the control assembly further includes a supply sensor operably connected to the supply passage and configured to detect a pressure within a portion of the supply passage between the cartridge connector and the supply valve, the user interface includes an initiation switch, and the control assembly is configured to override the initiation switch if the pressure is below a threshold value.

2. The cryotherapeutic system of claim 1, further comprising a pressure-monitoring chamber fluidly separate from the supply passage, wherein the control assembly further includes a pressure-monitoring sensor operably connected to the pressure-monitoring chamber and configured to detect a pressure within the pressure-monitoring chamber, and the control assembly is configured to signal the actuator to close the supply valve if the pressure is greater than a threshold value.

3. The cryotherapeutic system of claim 1, wherein the control assembly further includes a pressure-compensated flow regulator operably connected to the supply valve.

4. The cryotherapeutic system of claim 1, wherein the user interface includes a remote control unit.

5. The cryotherapeutic system of claim 1, further comprising battery leads operably connected to the control assembly.

6. The cryotherapeutic system of claim 1, wherein the cartridge connector includes a coupling member configured to pierce a membrane, open a check valve, or both.

7. The cryotherapeutic system of claim 1, further comprising an exhaust passage and an exhaust portal, wherein the exhaust passage has an outlet end, and the exhaust portal is at the outlet end.

8. The cryotherapeutic system of claim 1, further comprising a filter fluidly connected to the supply passage.

9. The cryotherapeutic system of claim 1, further comprising a sterilizer operably connected to the supply passage.

10. The cryotherapeutic system of claim 1, wherein the user interface includes an initiation switch, and the control assembly includes a timer triggered by the initiation switch.

11. The cryotherapeutic system of claim 1, further comprising a pressure-relief passage, a pressure-relief portal, and a pressure-relief valve along the pressure-relief passage, wherein the pressure-relief passage has an outlet end, the pressure-relief portal is at the outlet end, and the pressure-relief passage or the pressure-relief valve is fluidly connected to the supply passage at a connection point between the cartridge connector and the supply valve.

12. The cryotherapeutic system of claim 1, further comprising a heater operably connected to the cartridge housing and configured to heat a cartridge within the cartridge housing.

13. The cryotherapeutic system of claim 1, wherein the cartridge housing includes a main portion and a lid removably connectable to the main portion.

14. The cryotherapeutic system of claim 1, further comprising a first outlet adapter, wherein the supply passage has a first end and a second end opposite the first end, the cartridge connector is at the first end, and the first outlet adapter is at the second end; or further comprising a disposable bag configured to form a sterile barrier around at least a portion of the cryotherapeutic system; or further comprising a shell configured to form a sterile barrier around at least a portion of the cryotherapeutic system; or further comprising a primary housing including the cartridge housing and configured to attach to an edge portion of a surgical table.

## Patentansprüche

1. Kryotherapeutisches System, umfassend:
ein Kartuschengehäuse;
ein Kartuschenverbindungsstück benachbart zum Kartuschengehäuse;
eine Zuführleitung, die mit dem Kartuschenverbindungsstück fluidverbunden ist;
ein Zuführventil entlang der Zuführleitung; und
eine Steuerbaugruppe, einschließlich einer Betätigungsvorrichtung und einer Benutzerschnittstelle, wobei die Betätigungsvorrichtung mit dem Zuführventil wirkverbunden ist und die Steuerbaugruppe dazu konfiguriert ist, der Betätigungsvorrichtung als Reaktion auf ein Signal von der Benutzerschnittstelle zu signalisieren, das Zuführventil zu öffnen,
wobei die Steuerbaugruppe ferner einen Zuführsensor beinhaltet, der mit der Zuführleitung wirkverbunden ist und dazu konfiguriert ist, einen Druck innerhalb eines Abschnitts der Zuführleitung zwischen dem Kartuschenverbindungsstück und dem Zuführventil zu erfassen, die Benutzerschnittstelle einen Einleitungsschalter beinhaltet und die Steuerbaugruppe dazu konfiguriert ist, den Einleitungsschalter außer Kraft zu setzen, wenn der Druck unter einem Schwellenwert liegt.

2. Kryotherapeutisches System nach Anspruch 1, ferner umfassend eine Drucküberwachungskammer, die fluidisch von der Zuführleitung getrennt ist, wobei die Steuerbaugruppe ferner einen Drucküberwachungssensor beinhaltet, der mit der Drucküberwachungskammer wirkverbunden ist und dazu konfiguriert ist, einen Druck innerhalb der Drucküberwachungskammer zu erfassen, und die Steuerbaugruppe dazu konfiguriert ist, der Betätigungsvorrichtung zu signalisieren, das Zuführventil zu schließen, wenn der Druck über einem Schwellenwert liegt.

3. Kryotherapeutisches System nach Anspruch 1, wobei die Steuerbaugruppe ferner einen druckausgeglichenen Strömungsregler beinhaltet, der mit dem Zuführventil wirkverbunden ist.

4. Kryotherapeutisches System nach Anspruch 1, wobei die Benutzerschnittstelle eine Fernsteuereinheit beinhaltet.

5. Kryotherapeutisches System nach Anspruch 1, ferner umfassend Batterieleitungen, die mit der Steuerbaugruppe wirkverbunden sind.

6. Kryotherapeutisches System nach Anspruch 1, wobei das Kartuschenverbindungsstück ein Kupplungselement beinhaltet, das zum Durchdringen einer Membran, zum Öffnen eines Rückschlagventils oder zu beidem konfiguriert ist.

7. Kryotherapeutisches System nach Anspruch 1, ferner umfassend eine Abführleitung und eine Abführöffnung, wobei die Abführleitung ein Auslassende aufweist und sich die Abführöffnung am Auslassende befindet.

8. Kryotherapeutisches System nach Anspruch 1, ferner umfassend einen Filter, der mit der Zuführleitung fluidverbunden ist.

9. Kryotherapeutisches System nach Anspruch 1, ferner umfassend einen Sterilisator, der mit der Zuführleitung wirkverbunden ist.

10. Kryotherapeutisches System nach Anspruch 1, wobei die Benutzerschnittstelle einen Einleitungsschalter beinhaltet und die Steuerbaugruppe eine Zeitschaltuhr beinhaltet, die durch den Einleitungsschalter ausgelöst wird.

11. Kryotherapeutisches System nach Anspruch 1, ferner umfassend eine Druckablassleitung, eine Druckablassöffnung und ein Druckablassventil entlang der Druckablassleitung, wobei die Druckablassleitung ein Auslassende aufweist, sich die Druckablassöffnung am Auslassende befindet und die Druckablassleitung oder das Druckablassventil an einem Verbindungspunkt zwischen dem Kartuschenverbindungsstück und dem Zuführventil mit der Zuführleitung fluidverbunden ist.

12. Kryotherapeutisches System nach Anspruch 1, ferner umfassend eine Heizvorrichtung, die mit dem Kartuschengehäuse wirkverbunden ist und dazu konfiguriert ist, eine Kartusche in dem Kartuschengehäuse zu erwärmen.

13. Kryotherapeutisches System nach Anspruch 1, wobei das Kartuschengehäuse einen Hauptabschnitt und einen Deckel, der entfernbar mit dem Hauptabschnitt verbunden werden kann, beinhaltet.

14. Kryotherapeutisches System nach Anspruch 1, ferner umfassend einen ersten Auslassadapter, wobei die Zuführleitung ein erstes Ende und ein dem ersten Ende gegenüberliegendes zweites Ende aufweist, sich das Kartuschenverbindungsstück am ersten Ende befindet und sich der erste Auslassadapter am zweiten Ende befindet; oder ferner umfassend einen Einwegbeutel, der dazu konfiguriert ist, eine sterile Barriere um zumindest einen Abschnitt des kryotherapeutischen Systems zu bilden; oder ferner umfassend eine Hülle, die dazu konfiguriert ist, eine sterile Barriere um zumindest einen Abschnitt des kryotherapeutischen Systems zu bilden; oder ferner umfassend ein Primärgehäuse, welches das Kartuschengehäuse beinhaltet und dazu konfiguriert ist, an einem Randabschnitt eines Operationstisches befestigt zu werden.

## Revendications

1. Système de cryothérapie, comprenant :
un boîtier de cartouche ;
un connecteur de cartouche adjacent au boîtier de cartouche ;
un passage d'alimentation relié de manière fluidique au connecteur de cartouche ;
une vanne d'alimentation le long du passage d'alimentation ; et
un ensemble de commande comprenant un actionneur et une interface utilisateur, l'actionneur étant relié de manière fonctionnelle à la vanne d'alimentation, et l'ensemble de commande étant configuré pour signaler à l'actionneur d'ouvrir la vanne d'alimentation en réponse à un signal provenant de l'interface utilisateur,
l'ensemble de commande comprenant en outre un capteur d'alimentation relié de manière fonctionnelle au passage d'alimentation et conçu pour détecter une pression dans une partie du passage d'alimentation entre le connecteur de cartouche et la vanne d'alimentation, l'interface d'utilisateur comprenant un commutateur d'amorçage, et l'ensemble de commande étant conçu pour neutraliser le commutateur d'amorçage si la pression est inférieure à une valeur seuil.

2. Système de cryothérapie selon la revendication 1, comprenant en outre une chambre de surveillance de pression séparée de manière fluidique du passage d'alimentation, l'ensemble de commande comprenant en outre un capteur de surveillance de pression relié de manière fonctionnelle à la chambre de surveillance de pression et conçu pour détecter une pression dans la chambre de surveillance de pression, et l'ensemble de commande étant conçu pour signaler à l'actionneur de fermer la vanne d'alimentation si la pression est supérieure à une valeur seuil.

3. Système de cryothérapie selon la revendication 1, l'ensemble de commande comprenant en outre un régulateur de débit à pression compensée relié de manière fonctionnelle à la vanne d'alimentation.

4. Système de cryothérapie selon la revendication 1, l'interface utilisateur comprenant une unité de commande à distance.

5. Système de cryothérapie selon la revendication 1, comprenant en outre des fils de batterie reliés de manière fonctionnelle à l'ensemble de commande.

6. Système de cryothérapie selon la revendication 1, le connecteur de cartouche comprenant un élément de couplage conçu pour percer une membrane, ouvrir un clapet anti-retour, ou les deux à la fois.

7. Système de cryothérapie selon la revendication 1, comprenant en outre un passage d'échappement et un portail d'échappement, le passage d'échappement possédant une extrémité de sortie, et le portail d'échappement étant situé à l'extrémité de sortie.

8. Système de cryothérapie selon la revendication 1, comprenant en outre un filtre relié de manière fluidique au passage d'alimentation.

9. Système de cryothérapie selon la revendication 1, comprenant en outre un stérilisateur relié de manière fonctionnelle au passage d'alimentation.

10. Système de cryothérapie selon la revendication 1, l'interface utilisateur comprenant un commutateur d'amorçage, et l'ensemble de commande comprenant un minuteur déclenché par le commutateur d'amorçage.

11. Système de cryothérapie selon la revendication 1, comprenant en outre un passage de décharge, un portail de décharge et une vanne de décharge le long du passage de décharge, le passage de décharge possédant une extrémité de sortie, le portail de décharge étant situé à l'extrémité de sortie, et le passage de décharge ou la vanne de décharge étant relié de manière fluidique au passage d'alimentation au niveau d'un point de connexion situé entre le connecteur de cartouche et la vanne d'alimentation.

12. Système de cryothérapie selon la revendication 1, comprenant en outre un radiateur relié de manière fonctionnelle au boîtier de cartouche et conçu pour chauffer une cartouche dans le boîtier de cartouche.

13. Système de cryothérapie selon la revendication 1, le boîtier de cartouche comprenant une partie principale et un couvercle pouvant être relié de manière amovible à la partie principale.

14. Système de cryothérapie selon la revendication 1, comprenant en outre un premier adaptateur de sortie, le passage d'alimentation possédant une première extrémité et une seconde extrémité opposée à la première extrémité, le connecteur de cartouche étant situé à la première extrémité et le premier adaptateur de sortie étant situé à la seconde extrémité ; ou comprenant en outre un sac jetable conçu pour former une barrière stérile autour d'au moins une partie du système de cryothérapie ; ou comprenant en outre une enveloppe conçue pour former une barrière stérile autour d'au moins une partie du système de cryothérapie ; ou comprenant en outre un logement principal comprenant le boîtier de cartouche et conçu pour être fixé à une partie de bord d'une table chirurgicale.
